# EUROPEAN PATENT SPECIFICATION

(11) **EP 1 732 534 B1**
(45) Date of publication and mention of the grant of the patent: **23.07.2008**
(21) Application number: 05716814.8
(22) Date of filing: 25.02.2005
(51) Int. Cl.: A61K 31/197, A61P 9/04, A61P 9/10

(54) **USE OF METHYLENE AMIDE DERIVATIVES IN CARDIOVASCULAR DISORDERS**
VERWENDUNG VON METHYLENAMID-DERIVATEN BEI KARDIOVASKULÄREN ERKRANKUNGEN
UTILISATION DE DERIVES DE METHYLENE AMIDE DANS LE TRAITEMENT DES MALADIES CARDIOVASCULAIRES

(30) Priority: 27.02.2004 EP 04100778
(43) Date of publication of application: 20.12.2006
(73) Proprietor: Laboratoires Serono SA, 1267 Coinsins, Vaud (CH)
(72) Inventor: HOOFT VAN HUIJSDUIJNEN, Rob, CH-1233 Bernex (CH); RICHARD, Vincent, F-76230 Bois Guillaume (FR)
(74) Representative: Merck Serono International S.A. Intellectual Property
(86) International application number: PCT/EP2005/050823
(87) International publication number: WO 2005/082347

(56) References cited:
- WO-A-03/064376
- DE-A- 10 117 823

## Description

### Field of the invention

The present invention is related to the use of methylene amide derivatives of Formula (I) for the prevention and/or the treatment of cardiovascular disorders such as coronary obstruction and heart failure, in particular for the prevention and/or treatment of endothelial dysfunction in heart failure. The compounds of this invention are particularly useful in the treatment of increased peripheral vasoconstriction in chronic heart failure.

### Background of the invention

Despite major advances in the fields of cardiology and cardiac surgery in recent decades, heart failure still carries a high morbidity and mortality.

Nearly 5 millon Americans suffer from heart failure, with an incidence of about 10 per 1,000 population among the population older than 65 years of age *(*Jessup et al., 2003, N Engl. J. Med, 348 (20), 2007-2018*).*

Over the past decade, the rate of hospitalization for heart failure has increased by 159 percent *(Jessup et al., 2003, above).* Some reasons for such a progressive incidence are population aging, better treatment of cardiac diseases such as acute myocardial infarction and non-cardiac diseases such as cancer.

Heart failure is a constellation of signs and symptoms caused by inadequate performance of the heart. Its evolutive and progressive nature has led specialists to define four stages of heart failure (1 to 4) from high risk of development of heart failure to progressive structural abnormalities of the heart to end-stage symptoms *(*Hunt et al., 2001, J. Am. Coll. Cardiol., 38, 2101-13*).*

Mainly, heart failure is defined by the inability of the heart to eject blood and to provide adequate perfusion of the peripheral organs. Heart failure does not only affect the myocardium, but also has many consequences on the peripheral circulation. Especially, heart failure is associated with an increased peripheral vascular resistance, secondary to peripheral vasoconstriction. This vasoconstriction is heterogeneous, and mostly affects the 'non essential' territories such as the skin, the intestine and the skeletal muscle, in order to maintain perfusion in the 'essential' territories such as the brain or the heart in a context of decreased cardiac output. However, this initially adaptive mechanism may on the long term increase cardiac afterload (resistance to ventricular contraction) and cardiac work, and thus aggravate contractile dysfunction and contribute to the transition from compensated to decompensated heart failure. Long term impairment or loss of heart muscle activity leads to the development of Chronic Heart Failure (CHF).

The endothelium is constituted of a monolayer of cells located at the interface between blood and the vascular wall. The endothelium plays an important role in the control of human vascular tone and in the regulation of platelet and leukocyte functions by releasing endothelium-derived nitric oxide (NO).

An endothelial dysfunction, commonly assessed by a decreased endothelial production of NO in response to blood flow, induces an increased peripheral vasoconstriction and therefore an increased peripheral resistance. Endothelial dysfunction has been shown to occur both experimentally and clinically in early cardiovascular diseases, including long term during heart failure.

NO is a local factor which plays a major role in the control of vascular tone, regional blood flow and blood pressure. NO is continuously released by endothelial cells through the activation of endothelial NO synthase (eNOS).

Blood-flow is the major physiological stimulus for the permanent release of NO which leads to flow-dependent vasodilatation or flow-mediated vasodilatation (FMD). Flow-mediated vasodilatation is achieved through the flow-mediated activation of eNOS.

Constitutive NO production opposes vasoconstrictor influences such as that of the sympathetic system, or of the vasoconstrictor peptides angiotensin II or endothelin, and thus is now universally considered to be responsible for the existence of a strong permanent vasodilatory tone in the circulation.

The physiological roles of NO are not limited to vasodilatation. Indeed, endothelium-derived NO is also a potent inhibitor of platelet aggregation and adhesion, through increased platelet cGMP (Radomski et al., 1987, Br. J. Pharmacol., 92, 639-646). When produced at physiological concentrations, NO also exerts potent anti-inflammatory effect, and especially inhibits the adhesion of leukocytes to endothelial cells.

A decreased endothelial production of NO induces an increased in peripheral vasoconstriction and platelet aggregation, leading to subsequent increased risks of vasospasm and thrombosis, respectively. Moreover, given the inhibitory effects of NO on leukocyte activation and adhesion, endothelial dysfunction is considered to be one of the triggering factors for local vascular inflammatory responses which lead to the development of atherosclerosis (Ross, 1993, Nature, 362:801-809).

Several recent clinical data stress the role of endothelial dysfunction, NO production impairment or eNOS gene mutation or deficiency in cardiovascular diseases.

The hypothesis of a link between endothelial dysfunction and the progression of atherosclerosis is supported by several epidemiological studies showing that early endothelial dysfunction is a predictor of the development of atherosclerosis and of coronary artery disease (Suwaidi et al., 2000, Circulation, 101, 948-954*;* Schachinger et al., 2000, Circulation, 101, 1899-1906*;* Perticone et al., 2001, Circulation, 104, 191-196).

A recent clinical study reported that a mutation on the eNOS gene that decreases the NOS half-life in patient is associated a poorer event-free survival in patients with heart failure (McNamara et al., 2003, Circulation, 107, 1598-1602), in agreement with data in eNOS deficient mice (Scherrer-Crosbie et al., 2001, Circulation, 104, 1286-1291).

Substituted methylene amide derivatives of Formula (I) have been developed as Protein Tyrosine Phosphatase (PTP) inhibitors, particularly Protein Tyrosine Phosphatase 1B (PTP1B) inhibitors for the treatment of metabolic disorders mediated by insulin resistance or hyperglycemia (WO 03/064376).

Protein-tyrosine phosphatases (PTPs) play an important role in the regulation of phosphorylation of proteins and represent the counterparts of kinases. PTPs modulates the interaction of insulin with its receptor and the post-receptor signalling pathway by catalysing the dephosphorylation of cellular substrates of the insulin receptor kinase. Inhibitors of PTP-1B are known in the treatment of diabetes *(*Moller et al., 2000, Current Opinion in Drug Discovery & Development 3(5), 527-540).

Several treatments for heart failure have been developed such as β-blockers, diuretics, angiotensin receptor blockers, Angiotensin Converting Enzyme (ACE) inhibitors have been developed *(*Chin et al., 2001, Current Opinion in Investigational drugs, 2(7), 923-928*).*

However, the multifactorial and progressive nature of heart failure provides multiple opportunities for therapeutic intervention and the need for new treatments for heart failure, especially chronic heart failure.

The clinical evidences listed above suggest that increased peripheral resistance is partly due to decreased vasodilator influences, and particularly of the permanent, flow-induced release of nitric oxide (NO).

Therefore, impaired peripheral production of NO might contribute to aggravate heart failure, and thus pharmacological interventions that restore NO production in heart failure might exert beneficial effects in this disease.

### Summary of the invention

The present invention relates to the use of substituted methylene amide derivatives of Formula (I) 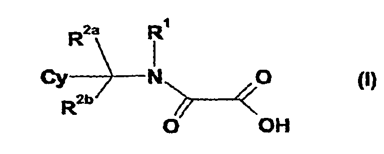 for the treatment and/or prevention of cardiovascular disorders such as coronary obstruction and heart failure, in particular for the treatment and/or prevention of endothelial dysfunction in chronic heart failure. The compounds of this invention are particularly useful in the treatment of increased peripheral vasoconstriction in chronic heart failure.

### Detailed description of the invention

The following paragraphs provide definitions of the various chemical moieties that make up the compounds according to the invention and are intended to apply uniformly throughout the specification and claims unless an otherwise expressly set out definition provides a broader definition.

"PTPs" are protein tyrosine phosphatases and include for instance PTP1B, TC-PTP, PTP-β, DEP-1, LAR, SHP-1, SHP-2, GLEPP-1, PTP-κ, PTP-µ, VHR, hVH5, LMW-PTP, PTEN.

"C₁-C₁₂-alkyl" or "C₁-C₁₅-alkyl" refers to straight or branched monovalent alkyl groups having 1 to 12 or 1 to 15 carbon atoms. This term is exemplified by groups such as methyl, ethyl, n-propyl, isopropyl, n-butyl, isobutyl, tert-butyl, n-hexyl, n-octyl, n-nonyl, n-dodecyl, tridecyl, pentadecyl, n-pentyl and the like in straight or branched forms thereof

"Aryl" refers to an unsaturated, aromatic carbocyclic group of from 6 to 14 carbon atoms having a single ring (e.g. phenyl) or multiple condensed rings (e.g. naphthyl). Preferred aryl include phenyl, naphthyl, phenantrenyl and the like.

"C₁-C₁₂-alkyl aryl" refers to C₁-C₁₂-alkyl groups having an aryl substituent, including benzyl, phenethyl and the like.

"Heteroaryl" refers to a monocyclic heteromatic, or a bicyclic or a tricyclic fused-ring heteroaromatic group. Particular examples of heteroaromatic groups include optionally substituted pyridyl, pyrrolyl, furyl, thienyl, imidazolyl, oxazolyl, isoxazolyl, thiazolyl, isothiazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl; 1,3,4-triazinyl, 1,2,3-triazinyl, benzofuryl, [2,3-dihydro]benzofuryl, isobenzofuryl, benzothienyl, benzotriazolyl, isobenzothienyl, indolyl, isoindolyl, 3H-indolyl, benzimidazolyl, imidazo[1,2-a]pyridyl, benzothiazolyl, benzoxazolyl, quinolizinyl, quinazolinyl, pthalazinyl, quinoxalinyl, cinnolinyl, napthyridinyl, pyrido[3,4-b]pyridyl, pyrido[3,2-b]pyridyl, pyrido[4,3-b]pyridyl, quinolyl, isoquinolyl, tetrazolyl, 5,6,7,8-tetrahydroquinolyl, 5,6,7,8-tetrahydroisoquinolyl, purinyl, pteridinyl, carbazolyl, xanthenyl or benzoquinolyl.

"C₁-C₁₂-alkyl heteroaryl" refers to C₁-C₁₂-alkyl groups having a heteroaryl substituent, including 2-furylmethyl, 2-thienylmethyl, 2-(1H-indol-3-yl)ethyl and the like.

"Alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂) and the like.

"Alkynyl" refers to alkynyl groups having from 2 to 18 carbon atoms and having at least 1-2 sites of alkynyl unsaturation, e.g. ethynyl (-C≡CH), propargyl (-CH₂C≡CH), or -C≡CH-(C₂-C₁₆)alkyl.

"Acyl" refers to the group -C(O)R where R includes "C₁-C₁₂-alkyl", "aryl", "heteroaryl", "C₁-C₁₂-alkyl aryl" or "C₁-C₁₂-alkyl heteroaryl".

"Acyloxy" refers to the group -OC(O)R where R includes "C₁-C₁₂-alkyl", "aryl", "heteroaryl", "C₁-C₁₂-alkyl aryl" or "C₁-C₁₂-alkyl heteroaryl".

"Alkoxy" refers to the group -O-R where R includes "C₁-C₁₂-alkyl" or "aryl" or "heteroaryl" or "C₁-C₁₂-alkyl aryl" or "C₁-C₁₂-alkyl heteroaryl". Preferred alkoxy groups include by way of example, methoxy, ethoxy, phenoxy and the like.

"Alkoxycarbonyl" refers to the group -C(O)OR where R includes "C₁-C₁₂-alkyl" or "aryl" or "heteroaryl" or "C₁-C₁₂-alkyl aryl" or "C₁-C₁₂-alkyl heteroaryl".

"Aminocarbonyl" refers to the group -C(O)NRR' where each R, R' includes independently hydrogen or C₁-C₁₂-alkyl or aryl or heteroaryl or "C₁-C₁₂-alkyl aryl" or "C₁-C₁₂-alkyl heteroaryl".

"Acylamino" refers to the group -NR(CO)R' where each R, R' is independently hydrogen or "C₁-C₁₂-alkyl" or "aryl" or "heteroaryl" or "C₁-C₁₂-alkyl aryl" or "C₁-C₁₂-alkyl heteroaryl".

"Halogen" refers to fluoro, chloro, bromo and iodo atoms.

"Substituted or unsubstituted": Unless otherwise constrained by the definition of the individual substituent, the above set out groups, like "alkyl", "alkenyl", "alkynyl", "aryl" and "heteroaryl" etc. groups can optionally be substituted with from 1 to 5 substituents selected from the group consisting of "C₁-C₆-alkyl", "C₂-C₆-alkenyl", "C₂-C₆-alkynyl", "cycloalkyl", "heterocycloalkyl", "C₁-C₆-alkyl aryl", "C₁-C₆-alkyl heteroaryl", "C₁-C₆-alkyl cycloalkyl", "C₁-C₆-alkyl heterocycloalkyl", "amino", "ammonium", "acyl", "acyloxy", "acylamino", "aminocarbonyl", "alkoxycarbonyl", "ureido", "aryl", "carbamate", "heteroaryl", "sulfinyl", "sulfonyl", "alkoxy", "sulfunyl", "halogen", "carboxy", trihalomethyl, cyano, hydroxy, mercapto, nitro, and the like. Alternatively said substitution could also comprise situations where neighbouring substituents have undergone ring closure, notably when vicinal functional substituents are involved, thus forming, *e*.*g*., lactams, lactons, cyclic anhydrides, but also acetals, thioacetals, aminals formed by ring closure for instance in an effort to obtain a protective group.

"Sulfonyl" refers to group "-SO₂-R" wherein R is selected from H, "aryl", "heteroaryl", "C₁-C₁₂-alkyl", "C₁-C₁₂-alkyl" substituted with halogens e.g. an -SO₂-CF₃ group, "C₁-C₁₂-alkyl aryl" or "C₁-C₁₂-alkyl heteroaryl".

"Sulfoxy" refers to a group "-S(O)-R" wherein R is selected from H, "C₁-C₁₂-alkyl", "C₁-C₁₂-alkyl" substituted with halogens e.g. an -SO-CF₃ group, "aryl", "heteroaryl" , "C₁-C₁₂-alkyl aryl" or "C₁-C₁₂-alkyl heteroaryl".

"Thioalkoxy" refers to groups -S-R where R includes "C₁-C₁₂-alkyl" or "aryl" or "heteroaryl" or "C₁-C₁₂-alkyl aryl" or "C₁-C₁₂-alkyl heteroaryl". Preferred thioalkoxy groups include thiomethoxy, thioethoxy, and the like.

"Resistance artery" refers to an artery with a diameter that is sufficiently small (<300µm), for the vessel to significantly influence local resistance to blood flow and its regulation. Resistance arteries provide the main peripheral resistance to blood flow, and as thus are important in the control and regulation of blood pressure, and blood flow in the major organ systems.

The term "heart failure" includes various stages of evolution and progression such as described in *Jessup et al*., *2003,* above. It includes for example structural abnormalities of the heart, diastolic heart failure and systolic heart failure.

"Pharmaceutically acceptable salts or complexes" refers to salts or complexes of the below-specified compounds of Formula (I). Examples of such salts include, but are not restricted, to base addition salts formed by reaction of compounds of Formula (I) with organic or inorganic bases such as hydroxide, carbonate or bicarbonate of a metal cation such as those selected in the group consisting of alkali metals (sodium, potassium or lithium), alkaline earth metals (e.g. calcium or magnesium), or with an organic primary, secondary or tertiary alkyl amine. Amine salts derived from methylamine, dimethylamine, trimethylamine, ethylamine, diethylamine, triethylamine, morpholine, N-Me-D-glucamine, N,N'-bis(phenyhnethyl)-1,2-ethanediamine, tromethamine, ethanolamine, diethanolamine, ethylenediamine, N-methylmorpholine, procaine, piperidine, piperazine and the like are contemplated being within the scope of the instant invention.

Also comprised are salts which are formed from to acid addition salts formed with inorganic acids (e.g. hydrochloric acid, hydrobromic acid, sulfuric acid, phosphoric acid, nitric acid, and the like), as well as salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and poly-galacturonic acid.

"Pharmaceutically active derivative" refers to any compound that upon administration to the recipient, is capable of providing directly or indirectly, the activity disclosed herein. The term "indirectly" also encompasses prodrugs which may be converted to the active form of the drug via endogenous enzymes or metabolism. Said prodrug is comprised of the active drug compound itself and a chemical masking group.

"Enantiomeric excess" (ee) refers to the products that are obtained by an asymmetric synthesis, i.e. a synthesis involving non-racemic starting materials and/or reagents or a synthesis comprising at least one enantioselective step, whereby a surplus of one enantiomer in the order of at least about 52% ee is yielded. In the absence of an asymmetric synthesis, e.g. the corresponding esters of the substituted methylene amides of formula I, racemic products are usually obtained that do however also have a PTP inhibiting activity.

Said formula also comprises its tautomers, its geometrical isomers, its optically active forms as enantiomers, diastereoisomers and its racemate forms, as well as pharmaceutically acceptable salts thereof. Preferred pharmaceutically acceptable salts of the Formula (I), are base addition salts formed by reaction of compounds of Formula (I) with pharmaceutically acceptable bases like N-methyl-D-glucamine, tromethamine, sodium, potassium or calcium salts of carbonates, bicarbonates or hydroxides.

The methylene amide derivatives according to the present invention are those of Formula (I):

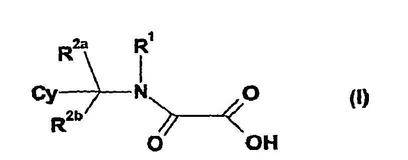

Formula (I) comprises also the geometrical isomers, the optically active forms, including enantiomers, diastereomers and its racemate forms, as well as pharmaceutically acceptable salts and pharmaceutically active derivatives thereof.

The substituents R¹, R^{2a}, R^{2b} and Cy within Formula (I) are defined as follows :
R¹ is selected from the group consisting of substituted or unsubstituted (C₁-C₁₅)-alkyl, substituted or unsubstituted (C₁-C₁₂)-alkyl, substituted or unsubstituted (C₁-C₆)-alkyl, substituted or unsubstituted (C₂-C₁₂)-alkenyl, substituted or unsubstituted (C₂-C₁₂)-alkynyl, substituted or unsubstituted aryl, substituted or unsubstituted heteroaryl, substituted or unsubstituted (3-8-membered) cycloalkyl or heterocycloalkyl, substituted or unsubstituted (C₁-C₁₂)-alkyl-aryl or substituted or unsubstituted (C₁-C₁₂)-alkyl-heteroaryl, substituted or unsubstituted (C₂-C₁₂)-alkenyl-aryl or -heteroaryl, substituted or unsubstituted (C₂-C₁₂)-alkynyl-aryl or -heteroaryl;
R^{2a} and R^{2b} are each independently from each other selected from the group comprising or consisting of H or substituted or unsubstituted (C₁-C₁₂)alkyl, preferably R^{2a} and R^{2b} are each H;
Cy is selected from D and E;
D is selected from substituted thienyl and substituted phenyl wherein substituents are selected from phenyl, oxadiazole and 1 or 2 moieties selected from the group consisting of -NH-CO-R³, -SO₂-NR³R^{3'}, and -CO-NR³R^{3'} in which R³, R³' are independently selected from H, substituted or unsubstituted (C₁-C₁₅)alkyl, substituted or unsubstituted (C₂-C₁₂)alkenyl, substituted or unsubstituted (C₂-C₁₂)alkynyl, substituted or unsubstituted aryl, substituted or unsubstituted heteroaryl, substituted or unsubstituted (3-8-membered) cycloalkyl or substituted or unsubstituted heterocycloalkyl, substituted or unsubstituted (C₁-C₁₂)alkyl aryl or heteroaryl, substituted or unsubstituted (C₂-C₁₂)alkenyl-aryl or - heteroaryl, substituted or unsubstituted (C₂-C₁₂)alkynyl-aryl or -heteroaryl;
E is selected from aryl, heteroaryl, (3-8-membered)-cycloalkyl and heterocycloalkyl wherein aryl, heteroaryl, (3-8-membered)-cycloalkyl and heterocycloalkyl are substituted by optionally substituted (C₂-C₁₈)alkynyl;

Such aryl or heteroaryl include phenyl, naphthyl, phenantrenyl, pyrrolyl, furyl, thienyl, imidazolyl, pyridyl, oxazolyl, isoxazolyl, thiazolyl, isothiazolyl, pyrazolyl, 1,2,3-triazolyl, 1,2,4-triazolyl, 1,2,3-oxadiazolyl, benzo(1,2,5)oxadiazolyl, 1,2,4-oxadiazolyl, 1,2,5-oxadiazolyl, 1,3,4-oxadiazolyl, tetrazolyl, 1,3,4-triazinyl, 1,2,3-triazinyl, benzopyrimidinyl, benzofuryl, [2,3-dihydro]benzofuryl, isobenzofuryl, benzothienyl, benzotriazolyl, isobenzothienyl, indolyl, isoindolyl, 3*H*-indolyl, benzimidazolyl, benzothiazolyl, benzoxazolyl, pyridazinyl, pyrimidyl, quinolizinyl, quinazolinyl, pthalazinyl, quinoxalinyl, cinnolinyl, napthyridinyl, quinolyl, isoquinolyl, tetrazolyl, 5,6,7,8-tetrahydroquinolyl, 5,6,7,8-tetrahydroisoquinolyl, purinyl, pteridinyl, xanthenyl, benzoquinolyl, oxolanyl, pyrolidinyl, pyrazolidinyl, 2H-benzo[d]1,3-dioxolenyl, indanyl, imidazolidinyl, 1,2,4-oxadiazolidinyl, 1,2,5-oxadiazolidinyl, 1,3,4-oxadiazolidinyl or isoxazolidinyl.

In a preferred embodiment, R^{2a} and R^{2b} are H.

In another preferred embodiment of the present invention, R¹ is A wherein A is selected fom substituted or unsubstituted aryl, substituted or unsubstituted heteroaryl, substituted or unsubstituted (3-8 membered)heterocycloalkyl and substituted or unsubstituted (3-8 membered)cycloalkyl, in particular a substituted or unsubstituted phenyl.

In another preferred embodiment, R¹ is a moiety of the formula -CH₂-A or -CH₂-CH₂-A, with A being selected from substituted or unsubstituted aryl, substituted or unsubstituted heteroaryl, substituted or unsubstituted (3-8-membered)heterocycloalkyl and substituted or unsubstituted (3-8-membered)cycloalkyl.

In particular, A may be a phenyl, pyridinyl, benzo-1,3-dioxolenyl, biphenyl, naphthyl, quinoxalinyl, thiazolyl, thienyl, furanyl or a piperidinyl group, being optionally substituted by 1 or 2 moieties selected from the group consisting of cyano, halogen, NO₂, (Ci-C₆)alkoxy, aryloxy or heteroaryloxy, (C₁-C₆)thioalkoxy, (C₁-C₁₂)alkyl, optionally halogenated (C₁-C₁₂)alkyl, (C₂-C₁₂)alkenyl, (C₂-C₁₂)alkynyl, aryl, heteroaryl, (3-8-membered)cycloalkyl or heterocycloalkyl, (C₁-C₁₂)alkyl aryl or heteroaryl, (C₂-C₁₂)alkenyl aryl or heteroaryl, (C₂-C₁₂)alkynyl aryl or heteroaryl, -COR³, -COOR³, -CO-NR³R^{3'}, - NHCOR³ wherein R³ is (C₁-C₁₂)alkyl or (C₂-C₁₂)alkenyl, -SOR³, -SO₂R³, -SO₂NR³R^{3'} with R³, R^{3'} being independently from each other selected from the group consisting of H, straight or branched (C₁-C₁₂)alkyl, (C₂-C₁₂)alkenyl, (C₂- C₁₂)alkynyl, aryl, heteroaryl, (3-8-membered)cycloalkyl or heterocycloalkyl.

According to one embodiment, Cy is D.

According to another embodiment R^{3'} is H and R³ is selected from the group consisting of diphenyl-ethyl, dodecyl, octyl, 4-pentyl-benzyl, 4-phenoxy-phenethyl, ethyl-thiophen-2-yl, pentadecyl, tridecyl, hexyloxy-phenyl, (2-ethyl)-hexyl.

According to another embodiment Cy is E.

According to a further embodiment Cy is E wherein E is selected from phenyl, pyridinyl, naphthyl and benzofuranyl group; phenyl, pyridinyl, naphthyl and benzofuranyl group being substituted by B-R⁴ wherein B is ethynyl group and R⁴ is substituted or unsubstituted (C₆-C₁₆)alkyl, substituted or unsubstituted (3-8 membered) cycloalkyl, substituted or unsubstituted (C₁-C₁₂)alkyl-(3-8 membered) cycloalkyl, substituted or unsubstituted phenyl or substituted or unsubstituted (C₁-C₁₂)alkyl phenyl.

More particularly, E is phenyl being substituted by B-R⁴ wherein B is ethynyl group and R⁴ is substituted or unsubstituted (C₆-C₁₆)alkyl.

According to a further embodiment R^{2a} and R^{2b} are each H, R¹ is -CH₂-A, or -CH₂-CH₂-A with A being phenyl or thienyl, optionally substituted by cyano, halogen, methoxy, hydroxy, phenoxy, -NO₂, trifluoromethyl while Cy is D, wherein D is selected from thienyl, phenyl and biphenyl; wherein thienyl, phenyl and biphenyl being substituted by - SO₂R³, -CO-NR³R^{3'} in which R^{3'} is H and R³ is (C₇-C₁₂)alkyl, particularly (C₈-C₁₂)alkyl and more particularly a docecyl group.

According to another further embodiment R^{2a} and R^{2b} are each H, R¹ is -CH₂-A, or -CH₂-CR₂-A with A being phenyl or thienyl, optionally substituted by cyano, halogen, methoxy, hydroxy, phenoxy, -NO₂, trifluoromethyl while Cy is D, wherein D is selected from thienyl, phenyl and biphenyl; wherein thienyl, phenyl and biphenyl being substituted by - SO₂R³, -CO-NR³R^{3'} in which R^{3'} is H and R³ is (C₇-C₁₅)alkyl, particularly (C₈-C₁₅)alkyl and most preferred a dodecyl group.

According to another further embodiment R^{2a} and R^{2b} are each H; R¹ is selected from phenyl, benzyl, phenethyl, 1-methylbenzyl which may be substituted by (C₁-C₆)alkyl group or a cycloalkyl group; Cy is D, wherein D is selected from phenyl and biphenyl group; wherein phenyl and biphenyl being substituted with a moiety selected from -NH-CO-R³, - CO-NH-R³, and a oxadiazole group substituted with R³, wherein R³ is (C₇-C₁₅)alkyl, particularly (C₈-C₁₅)alkyl and more particularly a dodecyl group.

More preferred compounds are those of Formula (I') 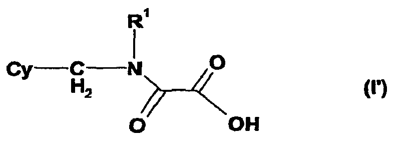 wherein
R¹ is selected from the group consisting of phenyl, benzyl, phenethyl, 1-methylbenzyl which may be substituted by (C₁-C₆)alkyl group or a cycloalkyl group;
Cy is D, wherein D is selected from phenyl and biphenyl; phenyl and biphenyl being substituted with a group selected from NH-CO-R³, -CO-NH-R³ and an oxadiazole group substituted with R³ in which R³ is (C₇-C₁₅)alkyl, particularly (C₈-C₁₅)alkyl and more particularly a dodecyl group.

Compounds of the present invention include in particular those of the group consisting of:
(benzyl{4-[(dodecylamino)carbonyl] benzyl}amino)(oxo)acetic acid;
oxo{{4-[(pentadecylamino)carbonyl]benzyl}[4-(trifluoromethyl)benzyl]aminol}acetic acid;
(benzyl{4-[(pentadecylamino)carbonyl]benzyl}amino)(oxo)acetic acid;
(benzyl{4-[(tridecylamino)carbonyl]benzyl}amino)(oxo)acetic acid;
[benzyl(4-{[dodecyl(methyl)amino]carbonyl}benzyl)amino](oxo)acetic acid;
{(4-{[dodecyl(methyl)amino]carbonyl}benzyl)[4-(trifluoromethyl)benzyl]amino}-(oxo) acetic acid;
([1-(tert-butoxycarbonyl)-4-piperidinyl]{4-[(dodecylamino)carbonyl]benzyl}-amino)-(oxo) acetic acid;
{{4-[(dodecylamino)carbonyl]benzyl}[4-(trifluoromethyl)benzyl]amino}(oxo)acetic acid;
{{4-[(dodecylamino)carbonyl]benzyl}[3-(trifluoromethyl)benzyl]amino}(oxo)acetic acid;
({[1-(tert-butoxycarbonyl)-4-piperidinyl]methyl}{4-[(dodecylamino)carbonyl]-benzyl} amino)(oxo)acetic acid;
oxo{[4-(tridecanoylamino)benzyl][4-(trifluoromethyl)beuzyl]amino}acetic acid;
[benzyl(4-{[4-(hexyloxy)benzoyl]amino}benzyl)amino](oxo)acetic acid;
oxo {[4-(trifluoromethyl)benzyl] [4-(10-undecenoylamino)benzyl]amino} acetic acid;
oxo {4-[(9E)-9-tetradecenoylamino]benzyl}[4-(trifluoromethyl)benzyl]amino} acetic acid;
{benzyl[4-(tridecanoylamino)benzyl]amino}(oxo)acetic acid;
{{4-[(2-hydroxydodecyl)amino]benzyl}[4-(trifluoromethyl)benzyl]amino}-(oxo)-acetic acid;
oxo{[4-(trifluoromethyl)benzyl][4-(3-undecyl-1,2,4-oxadiazol-5-yl)benzyl]-amino}-acetic acid;
{({5-[(dodecylamino)sulfonyl]-2-thienyl}methyl)[4-(trifluoromethyl)benzyl]amino}-(oxo) acetic acid;
[{4-[(dodecylamino)carbonyl]benzyl}({1-[(4-methoxyphenyl)sulfonyl]-4-piperi-dinyl} methyl)amino](oxo)acetic acid;
[{4-[(dodecylamino)carbonyl]benzyl}(2-carboxy-1-phenylethyl)amino](oxo)acetic acid;
[{4-[(dodecylamino)carbonyl]benzyl}(Z-methoxy-I-methylethyl)amino](oxo)acetic acid;
(4-bromo{4-[(dodecylamino)carbonyl]benzyl}anilino)(oxo)acetic acid;
({4-[(dodecylamino)carbonyl]benzyl}anilino)(oxo)acetic acid;
([2-(3-chlorophenyl)ethyl]{4-[(dodecylamino)carbonyl]benzyl}amino)(oxo)acetic acid;
{{4-[(dodecylamino)carbonyl]benzyl}[2-(3-methoxyphenyl)ethyl]amino}(oxo)acetic acid;
{{4-[(dodecylamino)carbonyl]benzyl}[(d,1)-trans-2-phenylcyclopropyl]amino}-(oxo)acetic acid;
([(d,1)-trans-2-(benzyloxy)cyclopentyl]{4-[(dodecylamino)carbonyl]benzyl}-amino)-(oxo) acetic acid;
({4-[(dodecylamino)carbonyl]benzyl}-4-phenoxyanilino)(oxo)acetic acid;
[{4-[(dodecylamino)carbonyl]benzyl}(1,2,3,4-tetrahydro-1-naphthalenyl)amino]-(oxo) acetic acid;
((1-benzyl-4-piperidinyl){4-[(dodecylamino)carbonyl]benzyl}amino)(oxo)acetic acid;
{{4-[(dodecylamino)carbonyl]benzyl}[2-(4-phenoxyphenyl)ethyl]amino}(oxo)acetic acid;
{{4-[(dodecylamino)carbonyl]benzyl}[2-(2-phenoxyphenyl)ethyl]amino}(oxo)acetic acid;
((2-[1,1'-biphenyl]-4-ylethyl){4-[(dodecylamino)carbonyl]benzyl}amino)(oxo)acetic acid;
(([1,1'-biphenyl]-3-ylmethyl){4-[(dodecylamino)carbonyl]benzyl}amino)(oxo)acetic acid;
(3-(benzyloxy){4-[(dodecylamino)carbonyl]benzyl}anilino)(oxo)acetic acid;
([4-(benzoylamino)benzyl]{4-[(dodecylamino)carbonyl]benzyl}amino)(oxo)acetic acid;
N-(carboxycarbonyl)-N-{4-[(dodecylamino)carbonyl]benzyl}-3-phenyl-beta-alanine;
{{4-[(dodecylamino)carbonyl]benzyl}[4-(1,2,3-thiadiazol-4-yl)benzyl]amino}-(oxo)acetic acid;
[{4-[(dodecylamino)carbonyl]benzyl}(4-pentylbenzyl)amino](oxo)acetic acid;
[{4-[(dodecylamino)carbonyl]benzyl}(1-phenylethyl)amino](oxo)acetic acid;
{{4-[(dodecylanimo)carbonyl]benzyl}[1-(1-naphthyl)ethyl]amino}(oxo)acetic acid;
(benzyl{3-[(dodecylamino)carbonyl]benzyl}amino)(oxo)acetic acid;
{{3-[(dodecylamino)carbonyl]benzyl}[4-(methylsulfonyl)benzyl]amino}(oxo)acetic acid;
((3-cyanobenzyl){3-[(dodecylamino)carbonyl]benzyl}amino)(oxo)acetic acid;
{{3-[(dodecylamino)carbonyl]benzyl}[4-(trifluoromethyl)benzyl]amino}(oxo)acetic acid;
[(4-chlorobenzyl)(3-{[(4-pentylbenzyl)amino]carbonyl}benzyl)amino](oxo)acetic acid;
oxo{[4-({[2-(2-thienyl)ethyl]amino}carbonyl)benzyl][4-(trifluoromethyl)-benzyl] amino} acetic acid;
{benzyl[(3'-{[(2,2-diphenylethyl)amino]carbonyl}[1,1'-biphenyl]-4-yl)methyl]-amino} (oxo)acetic acid;
{(3-cyanobenzyl)[(3'-{[(2,2-diphenylethyl)amino]carbonyl}[1,1'-biphenyl]-4-yl)methyl] amino}(oxo)acetic acid;
{(4-chlorobenzyl)[(3'-{[(2,2-diphenylethyl)amino]carbonyl}[1,1'-biphenyl]-4-yl)methyl] amino}(oxo)acetic acid;
{[(3'-{[(2,2-diphenylethyl)amino]carbonyl}[1,1'-biphenyl]-4-yl)methyl][4-(trifluoromethyl)benzyl]amino}(oxo)acetic acid;
((3-cyanobenzyl){[3'-({[2-(4-phenoxyphenyl)ethyl]amino}carbonyl)[1,1'-biphenyl]-4-yl] methyl}amino)(oxo)acetic acid;
oxo{{[3'-({[2-(4-phenoxyphenyl)ethyl]amino}carbonyl)[1,1'-biphenyl]-4-yl]methyl}-[4-(trifluoromethyl)benzyl]amino}acetic acid;
[(3-cyanobenzyl)({3'-[(octylamino)carbonyl][1,1'-biphenyl]-4-yl}methyl)amino]-(oxo) acetic acid;
[(4-chlorobenzyl)({3'-[(octylamino)carbonyl][1,1'-biphenyl]-4-yl}methyl)amino]-(oxo) acetic acid;
{({3'-[(octylamino)carbonyl] [1,1'-biphenyl]-4-yl}methyl)[4-(trifluoromethyl)-benzyl] amino}(oxo)acetic acid;
{(3-cyanobenzyl)[(3'-{[(3-phenylpropyl)amino]carbonyl}[1,1'-biphenyl]-4-yl)methyl] amino}(oxo)acetic acid;
[(3-cyanobenzyl)({3'-[(dodecylamino)carbonyl][1,1'-biphenyl]-4-yl}methyl)-amino]-(oxo) acetic acid;
[(4-chlorobenzyl)({3'-[(dodecylamino)carbonyl][1,1'-biphenyl]-4-yl}methyl)-amino]-(oxo) acetic acid;
{({3'-[(dodecylamino)carbonyl][1,1'-biphenyl]-4-yl}methyl)[4-(trifluoromethyl)-benzyl] amino}(oxo)acetic acid;
{benzyl[(3'-{[(4-pentylbenzyl)amino]carbonyl}[1,1'-biphenyl]-4-yl)methyl]amino}-(oxo) acetic acid;
{(3-cyanobenzyl)[(3'-{[(4-pentylbenzyl)amino]carbonyl}[1,1'-biphenyl]-4-yl)-methyl] amino}(oxo)acetic acid;
{(4-chlorobenzyl)[(3'-{[(4-pentylbenzyl)amino]carbonyl}[1,1'-biphenyl]-4-yl)-methyl] amino}(oxo)acetic acid;
oxo{[(3'-{[(4pentylbenzyl)amino]carbonyl}[1,1'-biphenyl]-4-yl)methyl][4-(trifluoromethyl)benzyl]amino}acetic acid;
oxo{[(3'-{[(4-phenylbutyl)amino]carbonyl}[1,1'-biphenyl]-4-yl)methyl][4-(trifluoroniethyl)benzyl]ammo}acetic acid;
{(3-cyanobenzyl)[(3'-{[(2-mesitylethyl)amino]carbonyl}[1,1'-biphenyl]-4-yl)-methyl] amino}(oxo)acetic acid;
{(4-chlorobenzyl)[(3'-{[(2-mesitylethyl)amino]carbonyl}[1,1'-biphenyl]-4-yl)-methyl] amino}(oxo)acetic acid;
{[(3'-{[(2-mesitylethyl)amino]carbonyl}[1,1'-biphenyl]-4-yl)methyl][4-(trifluoro-methyl) benzyl]amino}(oxo)acetic acid;
((4-chlorobenzyl){[3'-({[2-(4-methoxyphenyl)ethyl]amino}carbonyl)[1,1'-biphenyl]-4-yl] methyl}amino)(oxo)acetic acid;
[{4-[(dodecylamino)carbonyl]benzyl}(4-methoxybenzyl)amino](oxo)acetic acid;
{{4-[(dodecylamino)carbonyl]benzyl}[4-(methylsulfonyl)benzyl]amino}(oxo)acetic acid;
[{3-[(dodecylamino)carbonyl]benzyl}(4-methoxybenzyl)amino](oxo)acetic acid;
{{3-[(dodecylamino)carbonyl]benzyl}[3-(trifluoromethyl)benzyl]ainmo}(oxo)acetic acid;
({4-[(dodecylamino)carbonyl]benzyl} {[6-(trifluoromethyl)-3-pyridinyl]methyl}-amino) (oxo)acetic acid;
4-[((carboxycarbonyl){3-[(dodecylamino)carbonyl]benzyl}amino)methyl]benzoic acid;
({3-[(dodecylamino)carbonyl]benzyl}{4-[hydroxy(oxido)amino]benzyl}-amino) (oxo) acetic acid;
[{3-[(dodecylamino)carbonyl]benzyl}(2-fluorobenzyl)amino](oxo)acetic acid;
[{3-[(dodecylamino)carbonyl]benzyl}(2-pyridinylmethyl)amino](oxo)aceric acid;
[{3-[(dodecylamino)carbonyl]benzyl}(3-thienylmethyl)amino](oxo)acetic acid;
[{3-[(dodecylamino)carbonyl]benzyl}(4-hydroxybenzyl)amino](oxo)acetic acid;
[{3-[(dodecylamino)carbonyl]benryl}(4-phenoxybenzyl)amino](oxo)acetic acid;
({3-[(dodecylamino)carbonyl]benzyl}{[6-(trifluoromethyl)-3-pyridinyl]methyl}-amino) (oxo)acetic acid;
3-[((carboxycarbonyl){3-[(dodecylamino)carbonyl]benzyl}amino)methyl]benzoic acid;
5-[((carboxycarbonyl){3-[(dodecylamino)carbonyl]benzyl}amino)methyl]-2-thiophenecarboxylic acid;
({4-[(dodecylamino)carbonyl]benzyl}{4-[hydroxy(oxido)amino]-benzyl}-amino)-(oxo) acetic acid;
((1,3-benzodioxol-5-ylmethyl){4-[(dodecylamino)carbonyl]-benzyl}amino)-(oxo)-acetic acid;
[{4-[(dodecylamino)carbonyl]benzyl}(2-fluorobenzyl)amino](oxo)acetic acid;
[{4-[(dodecylamino)carbonyl]benzyl}(4-phenoxybenzyl)amino](oxo)acetic acid;
4-[((carboxycarbonyl){4-[(dodecylamino)carbonyl]benzyl}amino)methyl]benzoic acid;
5-[((carboxycarbonyl){4-[(dodecylamino)carbonyl]benzyl}amino)methyl]-2-thiophene carboxylic acid;
[{3-[(dodecylamino)carbonyl]benzyl}(2-thienylmethyl)amino](oxo)acetic acid;
[{4-[(dodecylamino)carbonyl]benzyl}(isopropyl)amino](oxo)acetic acid;
((3,5-dichlorobenzyl){4-[(dodecylamino)carbonyl]benzyl}amino)(oxo)acetic acid;
[(3,5-dichlorobenzyl)(4-{[(3,3-diphenylpropyl)amino]carbonyl}-benzyl)amino]-(oxo)acetic acid;
[(4-{[(2-[1,1'-biphenyl]-4-ylethyl)amino]carbonyl}benzyl)(3,5-dichlorobenzyl)-amino] (oxo)acetic acid;
[(1,3-benzodioxol-5-ylmethyl)(4-{[(2-[1,1'-biphenyl]-4-ylethyl)amino]carbonyl}-benzyl) amino](oxo)acetic acid;
(2,3-dihydro-1H-inden-1-yl{4-[(dodecylamino)carbonyl]benzyl}amino)(oxo)acetic acid;
{2,3-dihydro-1H-inden-1-yl[4-({[2-(4-phenoxyphenyl)ethyl]amino}-carbonyl)-benzyl] amino}(oxo)acetic acid;
[{4-[(dodecylamino)carbonyl]benzyl}(4-pyridinylmethyl)amino](oxo)acetic acid;
([4-(dimethylamino)benzyl]{4-[(dodecylamino)carbonyl]benzyl}amino)(oxo)acetic acid;
[{4-[(dodecylamino)carbonyl]benzyl}(3-pyridinylmethyl)amino](oxo)acetic acid;
((4-cyanobenzyl){4-[(dodecylamino)carbonyl]benzyl}amino)(oxo)acetic acid;
[{4-[(dodecylamino)carbonyl]benzyl}(1,3-thiazol-2-ylmethyl)amino](oxo)aceric acid;
({4-[(dodecylamino)carbonyl]benzyl}{[2-(4-morpholinyl)-1,3-thiazol-5-yl]methyl}-amino) (oxo)acetic acid;
[{3-[(dodecylamino)carbonyl]benzyl}(4-pyridinylmethyl)amino](oxo)acetic acid;
[{3-[(dodecylamino)carbonyl]benzyl}(3-pyridinylinethyl)amino](oxo)acetic acid;
[{3-[(dodecylamino)carbonyl]benzyl}(3-hydroxybenzyl)amino](oxo)acetic acid;
((4-cyanobenzyl){3-[(dodecylamino)carbonyl]benzyl}amino)(oxo)acetic acid;
[{3-[(dodecylamino)carbonyl]benzyl}(1,3-thiazol-2-ylmethyl)amino](oxo)acetic acid;
({3-[(dodecylamino)carbonyl]benzyl}{[2-(4-morpholinyl)-1,3-thiazol-5-yl]methyl}-amino) (oxo)acetic acid;
((1,3-benzodioxol-5-ylmethyl){3-[(dodecylamino)carbonyl]-benzyl}amino)-(oxo) acetic acid;
[{4-[(dodecylamino)carbonyl]benzyl}(2-thienylmethyl)amino](oxo)acetic acid;
[{4-[(dodecylamino)carbonyl]benzyl}(2-pyridinyhnethyl)amino](oxo)acetic acid;
[{4-[(dodecylamino)carbonyl]benzyl}(3-thienylmethyl)amino](oxo)acetic acid;
[{4-[(dodecylanvno)carbonyl]benzyl}(4-hydroxybenzyl)amino](oxo)acetic acid;
3-[((carboxycarbonyl){4-[(dodecylamino)carbonyl]benzyl}amino)methyl]benzoic acid;
[cyclopentyl({5-[(dodecylamino)sulfonyl]-2-thienyl}methyl)amino](oxo)acetic acid;
[benzyl({5-[(dodecylamino)sulfonyl]-2-thienyl}methyl)amino](oxo)acetic acid;
(({5-[(dodecylamino)sulfonyl]-2-thienyl}methyl){3-[hydroxy(oxido)amino]-benzyl}-amino)(oxo)acetic acid;
[({5-[(dodecylamino)sulfonyl]-2-thienyl}methyl)(4-methoxybenzyl)amino]-(oxo)-acetic acid;
[({5-[(dodecylamino)sulfonyl]-2-thienyl}methyl)(2-fluorobenzyl)amino](oxo)acetic acid;
{({5-[(dodecylamino)sulfonyl]-2-thienyl}methyl) [4-(methylsulfonyl)-benzyl]-amino}(oxo)acetic acid;
[({5-[(dodecylamino)sulfonyl]-2-thienyl}methyl)(4-phenoxybenzyl)amino]-(oxo)-acetic acid;
4-{[(carboxycarbonyl)({5-[(dodecylamino)sulfonyl]-2-thienyl}methyl)-amino]-methyl} benzoic acid;
(({5-[(dodecylamino)sulfonyl]-2-thienyl}methyl){[6-(trifluoromethyl)-3-pyridinyl]-methyl}amino)(oxo)acetic acid;
{({5-[(dodecylamino)sulfonyl]-2-thienyl}methyl)[3-(trifluoromethyl)benzyl]amino}-(oxo) acetic acid;
[(3-chlorobenzyl)({5-[(dodecylamino)sulfonyl]-2-thienyl}methyl)anvno](oxo)acetic acid;
{[(5-{[(3,3-diphenylpropyl)amino]sulfonyl}-2-thienyl)methyl][3-(trifluoromethyl)-benzyl] amino}(oxo)acetic acid;
{(3-chlorobenzyl)[(5-{[(3,3-diphenylpropyl)amino]sulfonyl}-2-thienyl)methyl]-amino} (oxo)acetic acid;
oxo {{[5-({2-(4-phenoxyphenyl)ethyl]amino}sulfonyl)-2-thienyl]methyl}[3-(trifluoromethyl)benzyl]amino}acetic acid;
((3-chlorobenzyl){[5-({[2-(4-phenoxyphenyl)ethyl]amino}sulfonyl)-2-thienyl]-methyl} amino)(oxo)acetic acid;
{[(5-{[(2-[1,1'-biphenyl]-4-ylethyl)amino]sulfonyl}-2-thienyl)methyl][3-(trifluoro-methyl) benzyl]amino}(oxo)acetic acid;
(({1-[(cyclohexylamino)carbonyl]-4-piperidinyl}methyl){4-[(dodecylamino)-carbonyl] benzyl}amino)(oxo)acetic acid;
([(1-{[4-(dimethylamino)anilino]carbonyl}-4-piperidinyl)methyl]{4-[(dodecyl-amino) carbonyl]benzyl}amino)(oxo)acetic acid;
{{4-[(dodecylamino)carbonyl]benzyl}[(1-hexanoyl-4-piperidinyl)methyl]-amino}-(oxo) acetic acid;
({4-[(dodecylamino)carbonyl]benzyl}{[1-(3-iodobenzoyl)-4-piperidinyl]methyl}-amino) (oxo)acetic acid;
{{4-[(dodecylamino)carbonyl]benzyl}[(1-1(2E)-3-[3-(trifluoromethyl)phenyl]-2-propenoyl}-4-piperidinyl)methyl]amino}(oxo)acetic acid;
({4-[(dodecylamino)carbonyl]benzyl}{[1-(2-quinoxalinylcarbonyl)-4-piperidinyl]-methyl} amino)(oxo)acetic acid;
[({1-[(4-methoxyphenyl)sulfonyl]-4-piperidinyl}methyl)(4-{[(4-phenoxybenzyl) amino] carbonyl}benzyl)amino](oxo)acetic acid;
[{[1-(3-iodobenzoyl)-4-piperidinyl]methyl}(4-{[(4-phenoxybenzyl)amino]-carbonyl} benzyl) amino](oxo)acetic acid;
oxo{(4-{[(4-phenoxybenzyl)amino]carbonyl}benzyl)[(1-{(2E)-3-[3-(lrifluoromethyl) phenyl]-2-propenoyl}-4-piperidinyl)methyl]amino}acetic acid;
{{4-[(dodecylamino)carbonyl]phenyl}[2-(methoxycarbonyl)benzyl]-amino}(oxo) acetic acid;
[[4-({[2-(1,1'-biphenyl-4-yl)ethyl]amino}carbonyl)-2-bromobenzyl](4-iodobenzyl)-amino] (oxo)acetic acid;
[(2-bromo-4-{[(4-pentylbenzyl)amino]carbonyl}benzyl)(4-iodobenzyl)amino]-(oxo)acetic acid;
[{2-bromo-4-[(dodecylamino)carbonyl]benzyl}(4-iodobenzyl)amino](oxo)acetic acid;
[(2,6-dibromo-4-{[(4-pentylbenzyl)amino]carbonyl}benzyl)(4-iodobenzyl)amino]-(oxo) acetic acid;
((4-iodobenzyl){[4'-({[2-(4-phenoxyphenyl)ethyl]amino}carbonyl)-1,1'-biphenyl-4-yl] methyl}amino)(oxo)acetic acid;
{[2-bromo-4-({[2-(4-phenoxyphenyl)ethyl]amino}carbonyl)benzyl][(4'-fluoro-1,1'-biphenyl-3-yl)melhyl]amino}(oxo)acetic acid;
{[4-({[2-(1,1'-biphenyl-4-yl)ethyl]amino}carbonyl)-2-bromobenzyl][(4'-fluoro-1,1'-biphenyl-3-yl)methyl]amino}(oxo)acetic acid;
{(2-bromo-4-{[(4-pentylbenzyl)amino]carbonyl}benzyl)[(4'-fluoro-1,1'-biphenyl-3-yl) methyl]amino}(oxo)acetic acid;
{[2,6-dibromo-4-({[2-(4-phenoxyphenyl)ethyl]amino}carbonyl)benzyl][(4'-fluoro-1,1'-biphenyl-3-yl)methyl]amino}(oxo)acetic acid;
{[4-({[2-(1,1'-biphenyl-4-yl)ethyl]amino}carbonyl)-2,6-dibromobenzyl][(4'-fluoro-1,1'-biphenyl-3-yl)methyl]amino}(oxo)acetic acid;
{(2,6-dibromo-4-{[(4-pentylbenzyl)amino]carbonyl}benzyl)[(4'-fluoro-1,1'-biphenyl-3-yl) methyl]amino}(oxo)acetic acid;
{{2,6-dibromo-4-[(dodecylamino)carbonyl]benzyl}[(4'-fluoro-1,1'-biphenyl-3-yl)methyl] amino}(oxo)acetic acid;
([(4'-fluoro-1,1'-biphenyl-3-yl)methyl]{[4'-({[2-(4-phenoxyphenyl)ethyl]amino}-carbonyl)-1,1'-biphenyl-4-yl]methyl}amino)(oxo)acetic acid;
{({4'-[(dodecylamino)carbonyl]-1,1'-biphenyl-4-yl}methyl)[(4'-fluoro-1,1'-biphenyl-3-yl) methyl]amino}(oxo)acetic acid;
{(2-bromo-4-{[(4-pentylbenzyl)amino]carbonyl}benzyl)[2-(trifluoromethoxy)-benzyl] amino}(oxo)acetic acid;
{(2,6-dibromo-4-{[(4-pentylbenzyl)amino]carbonyl}benzyl)[2-(trifluoromethoxy)-benzyl] amino}(oxo)acetic acid;
oxo {{[4'-({[2-(4-phenoxyphenyl)ethyl]amino}carbonyl)-1,1'-biphenyl-4-yl]methyl}-[2-(trifluoromethoxy)benzyl]amino}acetic acid;
{({4'-[(dodecylamino)carbonyl]-1,1'-biphenyl-4-yl}methyl)[2-(trifluoromethoxy)-benzyl] amino}(oxo)acetic acid;
[[2-bromo-4-({[2-(4-phenoxyphenyl)ethyl]amino}carbonyl)benzyl](3-phenoxy-benzyl) amino](oxo)acetic acid;
[[4-({[2-(1,1'-biphenyl-4-yl)ethyl]amino}carbonyl)-2-bromobenzyl](3-phenoxybenzyl) amino](oxo)acetic acid;
[(2-bromo-4-{[(4-pentylbenzyl)amino]carbonyl}benzyl)(3-phenoxybenzyl)-amino] (oxo) acetic acid;
[[2,6-dibromo-4-({[2-(4-phenoxyphenyl)ethyl]amino}carbonyl)benzyl](3-phenoxy benzyl)amino](oxo)acetic acid;
[[4-({[2-(1,1'-biphenyl-4-yl)ethyl]amino}carbonyl)-2,6-dibromobenzyl](3-phenoxy-benzyl) amino](oxo)acetic acid;
[(2,6-dibromo-4-{[(4-pentylbenzyl)amino]carbonyl}benzyl)(3phenoxybenzyl)-amino] (oxo)acetic acid;
[{2,6-dibromo-4-[(dodecylamino)carbonyl]benzyl}(3-phenoxybenzyl)amino](oxo)-acetic acid;
oxo((3-phenoxybenzyl){[4'-({[2-(4-phenoxyphenyl)ethyl]amino}carbonyl)-1,1'-biphenyl-4-yl]methyl}amino)acetic acid;
oxo[[(4'-{[(4-pentylbenzyl)amino]carbonyl}-1,1'-biphenyl-4-yl)methyl](3-phenoxybenzyl) amino]acetic acid;
[({4'-[(dodecylamino)carbonyl]-1,1'-biphenyl-4-yl}methyl)(3-phenoxybenzyl)-amino](oxo) acetic acid;
[[2-bromo-4-({[2-(4-phenoxyphenyl)ethyl]amino}carbonyl)benzyl](2-iodobenzyl)-amino] (oxo)acetic acid;
[[4-({[2-(1,1'-biphenyl-4-yl)ethyl]amino}carbonyl)-2-bromobenzyl](2-iodobenzyl)-amino] (oxo)acetic acid;
[(2-bromo-4-{[(4-pentylbenzyl)amino]carbonyl}benzyl)(2-iodobenzyl)amino]-(oxo)acetic acid;
[{2-bromo-4-[(dodecylamino)carbonyl]benzyl}(2-iodobenzyl)amino](oxo)acetic acid
([2-bromo-4-({[2-(4-phenoxyphenyl)ethyl]amino}carbonyl)benzyl]{[2'-(trifluoro-methyl)-1,1'-biphenyl-4-yl]methyl}amino)(oxo)acetic acid;
([4-({[2-(1,1'-biphenyl-4-yl)ethyl]amino}carbonyl)-2-bromobenzyl]{[2'-(trifluoro-methyl)-1,1'-biphenyl-4-yl]methyl}amino)(oxo)acetic acid;
((2-bromo-4-{[(4 pentylbenzyl)amino]carbonyl}benzyl){[2'-(trifluoromethyl)-1,1'-biphenyl -4-yl]methyl}amino)(oxo)acetic acid;
((2-bromo-4-{[(4-pentylbenzyl)amino]carbonyl}benzyl){[2'-(trifluoromethyl)-1,1'-biphenyl -4-yl]methyl}amino)(oxo)acetic acid;
({2-bromo-4-[(dodecylamino)carbonyl]benzyl}{[2'-(trifluoromethyl)-1,1'-biphenyl-4-yl] methyl}amino)(oxo)acetic acid;
([4-({[2-(1,1'-biphenyl-4-yl)ethyl]amino}carbonyl)-2,6-dibromobenzyl]{[2'-(tri-fluoro methyl)-1,1'-biphenyl-4-yl]methyl}amino)(oxo)acetic acid;
((2,6-dibromo-4-{[(4-pentylbenzyl)amino]carbonyl}benzyl){[2'-(trifluoromethyl)-1,1'-bipbenyl-4-yl]methyl}amino)(oxo)acetic acid;
({2,6-dibromo-4-[(dodecylamino)carbonyl]benzyl}{[2'-(trifluoromethyl)-1,1'-biphenyl-4-yl]methyl}amino)(oxo)acetic acid;
(({4'-[(dodecylamino)carbonyl]-1,1'-biphenyl-4-yl}methyl){[2'-(trifluoromethyl)-1,1'-biphenyl-4-yl]methyl}amino)(oxo)acetic acid;
[[4-({[2-(1,1'-biphenyl-4-yl)ethyl]amino}carbonyl)-2-bromobenzyl](1,1'-biphenyl-2-ylmethyl)amino](oxo)acetic acid;
[(1,1'-biphenyl-2-ylmethyl)(2-bromo-4-{((4-pentylbenzyl)amino]carbonyl}benzyl)-amino] (oxo)acetic acid;
((1,1'-biphenyl-2-ylmethyl){2-bromo-4-[(dodecylamino)carbonyl]benzyl}-amino)-(oxo) acetic acid;
{(1,1'-biphenyl-2-ylmethyl)[2,6-dibromo-4-({[2-(4-phenoxyphenyl)ethyl]amino}-carbonyl) benzyl]amino}(oxo)acetic acid;
[[4-({[2-(1,1'-biphenyl-4-yl)ethyl]amino}carbonyl)-2,6-dibromobenzyl](1,1'-biphenyl-2-ylmethyl)amino](oxo)acetic acid;
[(1,1'-biphenyl-2-ylmethyl)(2,6-dibromo-4-{[(4-pentylbenryl)amino]carbonyl}-benzyl) amino](oxo)acetic acid;
((1,1'-biphenyl-2-ylmethyl){2,6-dibromo-4-[(dodecylamino)carbonyl]benzyl}-amino)(oxo) acetic acid;
{(2-bromo-4-{[(4-pentylbenzyl)amino]carbonyl}benzyl)[4-(trifluoromethoxy)-benzyl] amino}(oxo)acetic acid;
{{2-bromo-4-[(dodecylamino)carbonyl]benzyl}[4-(trifluoromethoxy)benzyl]amino}-(oxo) acetic acid;
{(2,6-dibromo-4-{[(4-pentylbenzyl)ammo]carbonyl}benzyl)[4-(trifluoromethoxy)-benzyl] amino}(oxo)acetic acid;
{(2-bromo-4-{[(4-pentylbenzyl)amino]carbonyl}benzyl)[3-(trifluoromethoxy)-benzyl] amino}(oxo)acetic acid;
{{2-bromo-4-[(dodecylamino)carbonyl]benzyl}[3-(trifluoromethoxy)benzyl]amino}-(oxo) acetic acid;
{(2,6-dibromo-4-{[(4-pentylbenzyl)amino]carbonyl}benzyl)[3-(trifluoromethoxy)-benzyl] amino}(oxo)acetic acid;
{{2,6-dibromo-4-[(dodecylamino)carbonyl]benzyl}[3-(trifluoromethoxy)benzyl]-amino} (oxo)acetic acid;
{({4'-[(dodecylamino)carbonyl]-1,1'-biphenyl-4-yl}methyl)[3-(trifluoromethoxy)-benzyl] amino}(oxo)acetic acid;
[[2-bromo-4-({[2-(4-phenoxyphenyl)ethyl]amino}carbonyl)benzyl](4-phenoxy-benzyl) amino](oxo)acetic acid;
[[4-({[2-(1,1'-biphenyl-4-yl)ethyl]amino}carbonyl)-2-bromobenzyl](4-phenoxy-benzyl) amino](oxo)acetic acid;
[(2-bromo-4-{[(4-pentylbenzyl)amino]carbonyl}benzyl)(4-phenoxybenzyl)-amino](oxo) acetic acid;
[{2-bromo-4-[(dodecylamino)carbonyl]benzyl}(4-phenoxybenzyl)amino](oxo)acetic acid;
[[4-({[2-(1,1'-biphenyl-4-yl)ethyl]amino}carbonyl)-2,6-dibromobenzyl](4-phenoxy-benzyl) amino](oxo)acetic acid;
[(2,6-dibromo-4-{[(4-pentylbenzyl)amino]carbonyl}benzyl)(4-phenoxybenzyl)-amino] (oxo)acetic acid;
{[4-({[2-(1,1'-biphenyl-4-yl)ethyl]amino}carbonyl)-2-bromobenzyl][4-(trifluoro-methyl) benzyl]amino}(oxo)acetic acid;
{(2-bromo-4-{[(4-pentylbenzyl)amino]carbonyl}benzyl)[4-(trifluoromethyl)-benzyl]-amino}(oxo)acetic acid;
{{2-bromo-4-[(dodecylamino)carbonyl]benzyl}[4-(trifluoromethyl)benzyl]amino}-(oxo) acetic acid;
{(2,6-dibromo-4-{[(4-pentylbenzyl)amino]carbonyl}benzyl)[4-(trifluoromethyl)-benzyl] amino}(oxo)acetic acid;
{{2,6-dibromo-4-[(dodecylamino)carbonyl]benzyl}[4-(trifluoromethyl)benzyl]-amino} (oxo)acetic acid;
oxo{[(4'-{[(4-pentylbenzyl)amino]carbonyl}-1,1'-biphenyl-4-yl)methyl][4-(trifluoromethyl) benzyl]amino}acetic acid;
{{2-bromo-4-[(dodecylamino)carbonyl]benzyl}[3-(trifluoromethyl)benzyl]-amino} (oxo)acetic acid;
{{2,6-dibromo-4-[(dodecylamino)carbonyl]benzyl}[3-(trifluoromethyl)benzyl]-amino} (oxo)acetic acid;
oxo{[(4'-{[(4-pentylbenzyl)amino]carbonyl}-1,1'-biphenyl-4-yl)methyl][3-(trifluoro methyl)benzyl]amino}acetic acid;
{(4-dibenzo[b,d]furan-4-ylbenzyl)[4-(trifluoromethyl)benzyl]amino}(oxo)acetic acid;
{(4-dibenzo[b,d]furan-4-ylbenzyl)[4-(trifluoromethyl)benzyl]amino}(oxo)acetic acid, N-methyl-D-glucamine (i.e. 1-deoxy-1-(methylamino)glucitol) salt;
({4-[(dodecylamino)carbonyl]benzyl}{1-[4-(trifluoromethyl)phenyl]ethyl}amino)-(oxo) acetic acid;
({4-[(dodecylamino)carbonyl]benzyl}{1-[4-(trifluoromethyl)phenyl]ethyl}amino)-(oxo) acetic acid, N-methyl-D-glucamine (i.e. 1-deoxy-1-(methylamino)glucitol) salt;
{({4'-[(octylamino)carbonyl]-1,1'-biphenyl-4-yl}methyl)[4-(trifluoromethyl)benzyl]-amino}(oxo)acetic acid;
oxo{(4-tetradec-1-ynylbenzyl)[4-(trifluoromethyl)benzyl]amino}acetic acid;
{(4-dodec-1-ynylbenzyl)[4-(trifluoromethyl)benzyl]amino}(oxo)acetic acid;
{{4-[(dodecylamino)carbonyl]benzyl}[4-(trifluoromethyl)phenyl]amino}(oxo)acetic acid;
[{4-[(dodecylamino)carbonyl]benzyl}(2-methoxyphenyl)amino](oxo)acetic acid;
((1,2-diphenylethyl){4-[(dodecylamino)carbonyl]benzyl}amino)(oxo)acetic acid;
N-(carboxycarbonyl)-N-{4-[(dodecylamino)carbonyl]benzyl}-L-phenylalanine;
[{4-[(dodecylamino)carbonyl]benzyl}(3-phenoxyphenyl)amino](oxo)acetic acid;
[{4-[(dodecylamino)carbonyl]benzyl}(2-isopropoxyphenyl)amino](oxo)acetic acid;
[{4-[(dodecylamino)carbonyl]benzyl}(4-iodophenyl)amino](oxo)acetic acid;
{{4-[(dodecylamino)carbonyl]benzyl}[3-fluoro-4-(trifluoromethyl)benzyl]-amino} (oxo)
acetic acid;
((3-chloro-2-methylphenyl){4-[(dodecylamino)carbonyl]benzyl}amino)(oxo)acetic acid;
4'-((carboxycarbonyl){4-[(dodecylamino)carbonyl]benzyl}amino)-1,1'-biphenyl-2-carboxylic acid;
((2,4-dichlorobenzyl){4-[(dodecylamino)carbonyl]benzyl}amino)(oxo)acetic acid;
[{4-[(dodecylamino)carbonyl]benzyl}(1-phenylpropyl)amino](oxo)acetic acid;
([2-(4-chlorophenyl)propyl]{4-[(dodecylamino)carbonyl]benzyl}amino)(oxo)acetic acid;
[{4-[(dodecylamino)carbonyl]benzyl}(4-isopropoxyphenyl)amino](oxo)acetic acid;
([4-(benzyloxy)phenyl]{4-[(dodecylamino)carbonyl]benzyl}amino)(oxo)acetic acid;
{{4-[(dodecylamino)carbonyl]benzyl}[2-(trifluoromethyl)benzyl]amino}(oxo)acetic acid;
[{4-[(dodecylamino)carbonyl]benzyl}(2-methoxybenzyl)amino](oxo)acetic acid;
([(1R)-1-(4-chlorophenyl)ethyl]{4-[(dodecylamino)carbonyl]benzyl}amino)-(oxo) acetic acid;
((3,4-dichlorobenzyl){4-[(dodecylamino)carbonyl]benzyl}amino)(oxo)acetic acid
((1-benzothien-3-ylmethyl){4-[(dodecylanuno)carbonyl]benzyl}amino)(oxo)acetic acid;
([2-(2,6-dichlorophenyl)ethyl]{4-[(dodecylamino)carbonyl]benzyl}amino)(oxo)acetic acid;
({4-[(dodecylamino)carbonyl]benzyl}{2-[3-(trifluoromethyl)phenyl]ethyl}-amino)-(oxo) acetic acid;
{{4-[(dodecylamino)carbonyl]benzyl}[2-(3-fluorophenyl)ethyl]amino}(oxo)acetic acid;
([(1S)-1-(4-chlorophenyl)ethyl]{4-[(dodecylamino)carbonyl]benzyl}amino)(oxo)-acetic acid;
{{4-[(dodecylamino)carbonyl]benzyl}[(1S)-1-phenylethyl]amino}(oxo)acetic acid;
{{4-[(dodecylamino)carbonyl]benzyl}[(1R)-1-phenylethyl]amino}(oxo)acetic acid;
([3-(benzyloxy)phenyl]{4-[(dodecylamino)carbonyl]benzyl}amino)(oxo)acetic acid;
N-(carboxycarbonyl)-N-{4-[(dodecylamino)carbonyl]benzyl}-D-phenylalanine;
{{4-[(dodecylamino)carbonyl]phenyl}[4-(trifluoromethyl)benzyl]amino}(oxo)acetic acid;
{{4-[(dodecylamino)carbonyl]phenyl}[4-(trifluoromethyl)benzyl]amino}(oxo)acetic acid,
N-methyl-D-glucamine (i.e. 1-deoxy-1-(methylamino)glucitol) salt;
oxo{{1-[4-(trifluoromethyl)phenyl]ethyl}[4-(3-undecyl-1,2,4-oxadiazol-5-yl)benzyl] amino}acetic acid;
oxo{{1-[4-(trifluoromethyl)phenyl]ethyl}[4-(3-undecyl-1,2,4-oxadiazol-5-yl)benzyl]
amino}acetic acid, N-methyl-D-glucamine (i.e. 1-deoxy-1-(methylamino)glucitol) salt;
([(2-butyl-1-benzofuran-3-yl)methyl]{4-[(dodecylamino)carbonyl]benzyl}-amino)-(oxo) acetic acid;
{(1-{4-[(dodecylamino)carbonyl]phenyl}ethyl)[4-(trifluoromethyl)benzyl]amino}-(oxo) acetic acid;
{(1-{4-[(dodecylamino)carbonyl]phenyl}ethyl)[4-(trifluoromethyl)benzyl]amino}-(oxo)acetic acid, N-methyl-D-glucamine (i.e. 1-deoxy-1-(methylamino)glucitol) salt;
{(4-{[(4-octylphenyl)amino]carbonyl}benzyl)[4-(trifluoromethyl)benzyl]-amino}(oxo) acetic acid;
{(3-chlorobenzyl)[4-(3-undecyl-1,2,4-oxadiazol-5-yl)benzyl]amino}(oxo)acetic acid;
{(3-chlorobenzyl)[4-(3-undecyl-1,2,4-oxadiazol-5-yl)benzyl]amino}(oxo)acetic acid, N-methyl-D-glucamine (i.e. 1-deoxy-1-(methylamino)glucitol) salt;
{{cyclopentyl[4-(trifluoromethyl)phenyl]methyl}[4-(tridecanoylamino)benzyl]-amino} (oxo)acetic acid;
oxo([4-(trifluoromethyl)benzyl]{[4-(3-undecyl-1,2,4-oxadiazol-5-yl)-1-naphthyl]-methyl} amino)acetic acid;
oxo([4-(trifluoromethyl)benzyl] {[4-(3-undecyl-1,2,4-oxadiazol-5-yl)-1-naphthyl]-methyl}amino)acetic acid, N-methyl-D-glucamine (i.e. 1-deoxy-1-(methylamino)-glucitol) salt;
{{cyclopentyl[4-(tifluoromethyl)phenyl]methyl}[4-(3-undecyl-1,2,4-oxadiazol-5-yl) benzyl]amino}(oxo)acetic acid;
{{cyclopentyl[4-(trifluoromethyl)phenyl]methyl}[4-(3-undecyl-1,2,4-oxadiazol-5-yl)
benzyl]amino}(oxo)acetic acid, N-methyl-D-glucamine (i.e. 1-deoxy-1-(methyl-amino)glucitol) salt;
{(4-dibenzo[b,d]furan-4-ylphenyl)[4-(trifluoromethyl)benzyl]amino}(oxo)acetic acid;
{(4-dibenzo[b,d]furan-4-ylphenyl)[4-(trifluoromethyl)benzyl]amino}(oxo)acetic acid, N-methyl-D-glucamine (i.e. 1-deoxy-1-(methylamino)glucitol) salt;
{[4-(octyloxy)benzyl][4-(trifluoromethyl)benzyl]amino}(oxo)acetic acid;
{[4-(octyloxy)benzyl][4-(trifluoromethyl)benzyl]amino}(oxo)acetic acid, N-methyl-D-gliicamine (i.e. 1-deoxy-1-(methylamino)glucitol) salt;
[[2-(3-chlorophenyl)ethyl](4-dec-1-ynylbenzyl)amino](oxo)acetic acid;
([2-(3-chlorophenyl)ethyl]{4-[(1Z)-dec-1-enyl]benzyl}amino)(oxo)acetic acid;
{[2-(3-chlorophenyl)ethyl][4-(3-undecyl-1,2,4-oxadiazol-5-yl)benzyl]amino}(oxo)-acetic acid;
{[2-(3-chlorophenyl)ethyl][4-(3-undecyl-1,2,4-oxadiazol-5-yl)benzyl]amino}(oxo)-acetic acid, N-methyl-D-glucamine (i.e. 1-deoxy-1-(methylamino)glucitol) salt;
oxo{{(1R)-1-[4-(trifluoromethyl)phenyl]ethyl}[4-(3-undecyl-1,2,4-oxadiazol-5-yl)benzyl] amino}acetic acid;
oxo{{(1R)-1-[4-(trifluoromethyl)phenyl]ethyl}[4-(3-undecyl-1,2,4-oxadiazol-5-yl)benzyl] amino}acetic acid, N-methyl-D-glucamine (i.e. 1-deoxy-1-(methylamino)-glucitol) salt;
oxo{[4-(trifluoromethyl)phenyl][4-(3-undecyl-1,2,4-oxadiazol-5-yl)benzyl]amino}-acetic acid;
oxo{[4-(trifluoromethyl)phenyl][4-(3-undecyl-1,2,4-oxadiazol-5-yl)benzyl]amino}-acetic acid, N-methyl-D-glucamine (i.e. 1-deoxy-1-(methylamino)glucitol) salt;
oxo {{(1S)-1-[4-(trifluoromethyl)phenyl]ethyl}[4-(3-undecyl-1,2,4-oxadiazol-5-yl)benzyl] amino}acetic acid;
oxo {{(1S)-1-[4-(trifluoromethyl)phenyl]ethyl}[4-(3-undecyl-1,2,4-oxadiazol-5-yl)benzyl]amino}acetic acid, N-methyl-D-glucamine (i.e. 1-deoxy-1-(methylamino)-glucitol) salt;
[(3-chlorobenzyl)(4-dec-1-ynylbenzyl)amino](oxo)acetic acid;
[(3-chlorobenzyl)(4-dec-1-ynylbenzyl)amino](oxo)acetic acid, N-methyl-D-glucamine (i.e. 1-deoxy-1-(methylamino)glucitol) salt;
[[2-(3-chlorophenyl)ethyl](4-oct-1-ynylbenzyl)amino](oxo)acetic acid;
[[2-(3-chlorophenyl)ethyl](4-oct-1-ynylbenzyl)amino](oxo)acetic acid, N-methyl-D-glucamine (i.e. 1-deoxy-1-(methylamino)glucitol) salt;
{(4-dec-1-ynylbenzyl)[4-(trifluoromethyl)phenyl]amino}(oxo)acetic acid;
((4-dec-1-ynylbenzyl){1-[4-(trifluoromethyl)phenyl]ethyl}amino)(oxo)acetic acid;
((4-dec-1-ynylbenzyl){1-[4-(trifluoromethyl)phenyl]ethyl}amino)(oxo)acetic acid, N-methyl-D-glucamine (i.e. 1-deoxy-1-(methylamino)glucitol) salt;
{{1-methyl-1-[4-(trifluoromethyl)phenyl]ethyl}[4-(3-undecyl-1,2,4-oxadiazol-5-yl)benzyl] amino}(oxo)acetic acid;
{{1-methyl-1-[4-(trifluoromethyl)phenyl]ethyl}[4-(3-undecyl-1,2,4-oxadiazol-5-yl)benzyl]amino}(oxo)acetic acid, N-methyl-D-glucamine (i.e. 1-deoxy-1-(methylamino) glucitol) salt;
{[2-(3-chlorophenyl)ethyl][4-(3-octyl-1,2,4-oxadiazol-5-yl)beozyl]amino}(oxo)acetic acid;
{[2-(3-chlorophenyl)ethyl][4-(3-octyl-1,2,4-oxadiazol-5-yl)benzyl]amino}(oxo)acetic acid,
N-methyl-D-glucamine (i.e. 1-deoxy-1-(methylamino)glucitol) salt;
{[4-(3-octyl-1,2,4-oxadiazol-5-yl)benzyl][4-(trifluoromethyl)benzyl]amino}-(oxo) acetic acid;
{[4-(3-octyl-1,2,4-oxadiazol-5-yl)benzyl][4-(trifluoromethyl)benzyl]amino}-(oxo)-acetic acid, N-methyl-D-glucamine (i.e. 1-deoxy-1-(methylamino)glucitol) salt;
{{[4-(dodecyloxy)-1-naphthyl]methyl}[4-(trifluoromethyl)benzyl]amino}(oxo)acetic acid;
{{[4-(dodecyloxy)-1-naphthyl]methyl}[4-(trifluoromethyl)benzyl]amino}(oxo)acetic acid,
N-methyl-D-glucamine (i.e. 1-deoxy-1-(methylamino)glucitol) salt
[(4-bromobenzyl)(4-oct-1-ynylbenzyl)amino](oxo)acetic acid;
[{4-[(dodecylamino)carbonyl]benzyl}(2-hydroxy-1-phenylethyl)amino](oxo)acetic acid;
((4-dec-1-ynylbenzyl){1-methyl-1-[4-(trifluoromethyl)phenyl]ethyl}amino)(oxo)-acetic acid;
((4-dec-1-ynylbenzyl){1-methyl-1-[4-(trifluoromethyl)phenyl]ethyl}amino)(oxo)-acetic acid, N-methyl-D-glucamine (i.e. 1-deoxy-1-(methylamino)glucitol) salt;
oxo{{4-[(9Z)-tetradec-9-enoylamino]benzyl}[4-(trifluoromethyl)benzyl]amino}-acetic acid;
{(4-dec-1-ynylbenzyl)[4-(trifluoromethyl)benzyl]amino}(oxo)acetic acid;
oxo {[4-(trifluoromethyl)benzyl][3-(3-undecyl-1,2,4-oxadiazol-5-yl)benzyl]-amino}acetic acid;
oxo{[4-(trifluoromethyl)benzyl][3-(3-undecyl-1,2,4-oxadiazol-5-yl)benzyl]amino}-acetic acid, N-methyl-D-glucamine (i.e. 1-deoxy-1-(methylamino)glucitol) salt;
{(4-dodecylbenzyl)[4-(trifluoromethyl)benzyl]amino}(oxo)acetic acid;
{(4-dodecylbenzyl)[4-(trifluoromethyl)benzyl]amino}(oxo)acetic acid, N-methyl-D-glucamine (i.e. 1-deoxy-1-(methylamino)glucitol) salt;
{[4-({[(2-butyl-1-benzofuran-3-yl)methyl]amino}carbonyl)benzyl][4-(trifluoro-methyl) benzyl]amino}(oxo)acetic acid;
{(4-{[4-(benzyloxy)benzoyl]amino}benzyl)[4-(trifluoromethyl)benzyl]amino}-(oxo) acetic acid;
{(3,5-dichlorobenzyl)(4-(tridecanoylamino)benzyl]amino}(oxo)acetic acid;
{(3,5-dichlorobenzyl)[4-(tridecanoylamino)benzyl]amino}(oxo)acetic acid, N-methyl -D-glucamine (i.e. 1-deoxy-1-(methylamino)glucitol) salt;
{{4-[(4-octylphenyl)ethynyl]benzyl}[4-(trifluoromethyl)benzyl]amino}(oxo)acetic acid;
oxo {[4-(trifluoromethyl)benzyl][4-(5-undecyl-1,2,4-oxadiazol-3-yl)benzyl]amino}-acetic acid;
oxo {[4-(trifluoromethyl)benzyl][4-(5-undecyl-1,2,4-oxadiazol-3-yl)benzyl]amino}-acetic
acid, N-methyl-D-glucamine (i.e. 1-deoxy-1-(methylamino)glucitol) salt;
{{4-[2-(4-octylphenyl)ethyl]benzyl}[4-(trifluoromethyl)benzyl]amino}(oxo)acetic acid;
{(4-{[4-(heptyloxy)phenyl]ethynyl}benzyl)[4-(trifluoromethyl)benzyl]amino}-(oxo)acetic acid;
{{4-[(4-butylphenyl)ethynyl]benzyl}[4-(trifluoromethyl)benzyl]amino}(oxo)acetic acid;
{{4-[(4-hexylphenyl)ethynyl]benzyl}[4-(trifluoromethyl)benzyl]amino}(oxo)acetic acid;
{{4-[(4-hexylphenyl)ethynyl]benzyl}[4-(trifluoromethyl)benzyl]amino}(oxo)acetic acid,
N-methyl-D-glucamine (i.e. 1-deoxy-1-(methylamino)glucitol) salt;
oxo{(4-{[4-(pentyloxy)phenyl]ethynyl}benzyl)[4-(trifluoromethyl)benzyl]-amino}-acetic acid;
oxo{{4-[(4-propylphenyl)ethynyl]benzyl}[4-(trifluoromethyl)benzyl]amino}acetic acid;
[[2-(3-chlorophenyl)ethyl](4-dodec-1-ynylbenzyl)amino](oxo)acetic acid;
[[2-(3-chlorophenyl)ethyl](4-dodec-1-ynylbenzyl)amino](oxo)acetic acid, N-methyl-D-glucamine (i.e. 1-deoxy-1-(methylamino)glucitol) salt;
{(4-oct-1-ynylbenzyl)[4-(trifluoromethyl)benzyl]amino}(oxo)acetic acid;
{[4-(11-hydroxyundec-1-ynyl)benzyl][4-(trifluoromethyl)benzyl]amino}(oxo)acetic acid;
{[4-(11-methoxy-11-oxoundec-1-ynyl)benzyl][4-(trifluoromethyl)benzyl]amino}-(oxo) acetic acid;
11-[4-({(carboxycarbonyl)[4-(trifluoromethyl)benzyl]amino}methyl)phenyl]undec-10-ynoic acid;
{(4-{[4-(benzyloxy)phenyl]ethynyl}benzyl)[4-(trifluoromethyl)benzyl]amino}-(oxo)acetic acid;
{(4-{2-[4-(heptyloxy)phenyl]ethyl}benzyl)[4-(trifluoromethyl)benzyl]amino}(oxo)-acetic acid;
{{4-[2-(4-butylphenyl)ethyl]benzyl}[4-(trifluoromethyl)benzyl]amino}(oxo)acetic acid;
{{4-[2-(4-hexylphenyl)ethyl]benzyl}[4-(trifluoromethyl)benzyl]amino}(oxo)acetic acid;
{{4-[2-(4-hexylphenyl)ethyl]benzyl}[4-(trifluoromethyl)benzyl]amino}(oxo)acetic acid, N-methyl-D-glucamine (i.e. 1-deoxy-1-(methylamino)glucitol) salt;
oxo {(4-{2-[4-(pentyloxy)phenyl]ethyl}benzyl)[4-(trifluoromethyl)benzyl]-amino}acetic acid;
oxo{{4-[2-(4-propylphenyl)ethyl]benzyl}[4-(trifluoromethyl)benzyl]amino}acetic acid;
11-[4-({(carboxycarbonyl)[4-(trifluoromethyl)benzyl]amino}methyl)phenyl]-undecanoic acid;
{[4-(11-hydroxyundecyl)benzyl][4-(trifluoromethyl)benzyl]aimno}(oxo)acetic acid;
{(4-dodec-1-ynylbenzyl)[4-(trifluoromethyl)phenyl]amino}(oxo)acetic acid;
{(4-dodec-1-ynylbenzyl)[4-(trifluoromethyl)phenyl]amino}(oxo)acetic acid, N-methyl-D-glucamine (i.e. 1-deoxy-1-(methylamino)glucitol) salt;
oxo([4-(trifluoromethyl)benzyl]{4-[2-(3-undecyl-1,2,4-oxadiazol-5-yl)ethyl]benzyl}-amino)acetic acid;
oxo([4-(trifluoromethyl)benzyl]{4-[2-(3-undecyl-1,2,4-oxadiazol-5-yl)ethyl]benzyl}-amino)acetic acid, N-methyl-D-glucamine (i.e. 1-deoxy-1-(methylamino)glucitol) salt;
{{4-[2-(3-octyl-1,2,4-oxadiazol-5-yl)ethyl]benzyl}[4-(trifluoromethyl)benzyl]-amino} (oxo)acetic acid;
{{4-[2-(3-octyl-1,2,4-oxadiazol-5-yl)ethyl]benzyl}[4-(trifluoromethyl)benzyl]-amino} (oxo)acetic acid, N-methyl-D-glucamine (i.e. 1-deoxy-1-(methylamino)glucitol) salt;
{{4-[(4-octylbenzoyl)amino]benzyl}[4-(trifluoromethyl)benzyl]amino}(oxo)acetic acid;
{{4-[(4-octylbenzoyl)amino]benzyl}[4-(trifluoromethyl)benzyl]amino}(oxo)acetic acid, N-methyl-D-glucamine (i.e. 1-deoxy-1-(methylamino)glucitol) salt;
oxo{[(1-tridecanoylpiperidin-4-yl)methyl][4-(trifluoromethyl)benzyl]amino}acetic acid;
{{[1-(4-octylbenzoyl)piperidin-4-yl]methyl}[4-(trifluoromethyl)benzyl]-amino}-(oxo) acetic acid;
{{[1-(4-octylbenzoyl)piperidin-4-yl]methyl}[4-(trifluoromethyl)benzyl]amino}-(oxo)acetic acid, N-methyl-D-glucamine (i.e. 1-deoxy-1-(methylamino)glucitol) salt;
{[(3-dec-1-ynyl-1-benzofuran-5-yl)methyl][4-(trifluoromethyl)benzyl]amino}-(oxo)acetic acid;
{[(3-dodec-1-ynyl-1-benzofuran-5-yl)methyl][4-(trifluoromethyl)benzyl]amino}-(oxo) acetic acid;
oxo{({3-[(4-propylphenyl)ethynyl]-1-benzofuran-5-yl}methyl)[4-(trifluoromethyl)-benzyl] amino}acetic acid;
[(4-dodee-1-ynylbenzyl)(4-fluorobenzyl)amino](oxo)acetic acid;
[bis(4-oct-1-ynylbenryl)aanino](oxo)acetic acid;
{[(6-dodec-1-ynylpyridin-3-yl)methyl][4-(trifluoromethyl)benzyl]amino}(oxo)acetic acid;
{(3-dodec-1-ynylbenzyl)[4-(trifluoromethyl)benzyl]amino}(oxo)acetic acid;
{[2-(2-fluorophenyl)ethyl][4-(3-undecyl-1,2,4-oxadiazol-5-yl)benzyl]amino}-(oxo)acetic acid;
{[2-(2-fluorophenyl)ethyl][3-(3-undecyl-1,2,4-oxadiazol-5-yl)benzyl]amino}-(oxo)acetic acid;
{[2-(2-fluorophenyl)ethyl][4-(3-octyl-1,2,4-oxadiazol-5-yl)benzyl]amino}(oxo)acetic acid;
{[2-(3,4-dichlorophenyl)ethyl][4-(3-undecyl-1,2,4-oxadiazol-5-yl)benzyl]amino}-(oxo) acetic acid;
{[2-(3,4-dichlorophenyl)ethyl][3-(3-undecyl-1,2,4-oxadiazol-5-yl)benzyl]amino}-(oxo) acetic acid;
{[2-(3,4-dichlorophenyl)ethyl][4-(3-octyl-1,2,4-oxadiazol-5-yl)benzyl] amino}(oxo)acetic acid;
{[2-(1,1'-biphenyl-4-yl)ethyl][4-(3-undecyl-1,2,4-oxadiazol-5-yl)benzyl]amino}-(oxo)acetic acid;
{[2-(1,1'-biphenyl-4-yl)ethyl][3-(3-undecyl-1,2,4-oxadiazol-5-yl)benzyl]amino}-(oxo) acetic acid;
{[2-(1,1'-biphenyl-4-yl)ethyl][4-(3-octyl-1,2,4-oxadiazol-5-yl)benzyl]amino}-(oxo) acetic acid;
oxo{5,6,7,8-tetrahydronaphthalen-1-yl[4-(3-undecyl-1,2,4-oxadiazol-5-yl)benzyl]-amino} acetic acid;
oxo{5,6,7,8-tetrahydronaphthalen-1-yl[3-(3-undecyl-1,2,4-oxadiazol-5-yl)benzyl]-amino} acetic acid;
[[4-(3-octyl-1,2,4-oxadiazol-5-yl)benzyl](5,6,7,8-tetrahydronaphthalen-1-yl)amino]-(oxo) acetic acid;
{(1,1'-biphenyl-3-ylmethyl)[4-(3-undecyl-1,2,4-oxadiazol-5-yl)benzyl]amino}-(oxo)acetic acid;
{(1,1'-biphenyl-3-ylmethyl)[3-(3-undecyl-1,2,4-oxadiazol-5-yl)benzyl]amino}-(oxo) acetic acid;
{(1,1'-biphenyl-3-ylmethyl)[4-(3-octyl-1,2,4-oxadiazol-5-yl)benzyl]amino}-(oxo)-acetic acid;
{(1-benzothien-3-ylmethyl)[4-(3-undecyl-1,2,4-oxadiazol-5-yl)benzyl]amino}-(oxo)-acetic acid;
{(1-benzothien-3-ylmethyl)[3-(3-undecyl-1,2,4-oxadiazol-5-yl)benzyl]amino}(oxo)-acetic acid;
{(1-benzothien-3-ylmethyl)[4-(3-octyl-1,2,4-oxadiazol-5-yl)benzyl]amino}(oxo)-acetic acid;
oxo{[2-(trifluoromethyl)benzyl][4-(3-undecyl-1,2,4-oxadiazol-5-yl)benzyl]amino}-acetic acid;
oxo{[2-(trifluoromethyl)benzyl][3-(3-undecyl-1,2,4-oxadiazol-5-yl)benzyl]amino} acetic acid;
{[4-(3-octyl-1,2,4-oxadiazol-5-yl)benzyl][2-(trifluoromethyl)benzyl]amino}(oxo)-acetic acid;
oxo{[3-(trifluoromethyl)benzyl][4-(3-undecyl-1,2,4-oxadiazol-5-yl)benzyl]-amino}-acetic acid;
oxo {[3-(trifluoromethyl)benzyl][3-(3-undecyl-1,2,4-oxadiazol-5-yl)benzyl]-amino}-acetic acid;
{[4-(3-octyl-1,2,4-oxadiazol-5-yl)benzyl][3-(trifluoromethyl)benzyl]amino}-(oxo)-acetic acid;
{(2-methoxybenzyl)[4-(3-undecyl-1,2,4-oxadiazol-5-yl)benzyl]amino}(oxo)acetic acid {(2-methoxybenzyl)[3-(3-undecyl-1,2,4-oxadiazol-5-yl)benzyl]amino}(oxo)-acetic acid;
{(2-methoxybenzyl)[4-(3-octyl-1,2,4-oxadiazol-5-yl)benzyl]amino}(oxo)acetic acid;
oxo{{4-[(trifluoromethyl)sulfonyl]benzyl}[4-(3-undecyl-1,2,4-oxadiazol-5-yl)-benzyl] amino}acetic acid;
oxo{{4-[(trifluoromethyl)sulfonyl]benzyl}[3-(3-undecyl-1,2,4-oxadiazol-5-yl)-benzyl] amino}acetic acid;
([4-(3-octyl-1,2,4-oxadiazol-5-yl)benzyl]{4-[(trifluoromethyl)-sulfonyl]benzyl}-amino) (oxo)acetic acid;
{1,3-benzodioxol-5-yl[4-(3-undecyl-1,2,4-oxadiazol-5-yl)benzyl]amino}(oxo)acetic acid;
{1,3-benzodioxol-5-yl[3-(3-undecyl-1,2,4-oxadiazol-5-yl)benzyl]amino}(oxo)acetic acid;
{1,3-benzodioxol-5-yl[4-(3-octyl-1,2,4-oxadiazol-5-yl)benzyl]amino}(oxo)acetic acid;
{[(4-dodec-1-ynyl-1-naphthyl)methyl][4-(trifluoromethyl)benzyl]amino}(oxo)acetic acid;
{[(4-dec-1-ynyl-1-naphthyl)methyl][4-(trifluoromethyl)benzyl]amino}(oxo)acetic acid;
{[(4-dec-1-ynyl-1-naphthyl)methyl][4-(trifluoromethyl)benzyl]amino}(oxo)acetic acid;
oxo {[4-(trifluoromethyl)benzyl][4-(4-undecyl-1,3-thiazol-2-yl)benzyl]amino}acetic acid;
{(4-dec-1-ynylbenzyl)[2-(2-fluorophenyl)ethyl]amino}(oxo)acetic acid;
{(4-dodec-1-ynylbenzyl)[2-(2-fluorophenyl)ethyl]amino}(oxo)acetic acid;
{{[4-(dodecyloxy)-1-naphthyl]methyl}[2-(2-fluorophenyl)ethyl]amino}(oxo)acetic acid;
{[2-(2-fluorophenyl)ethyl][4-(octyloxy)benzyl]amino}(oxo)acetic acid;
{(4-dec-1-ynylbenzyl)[2-(trifluoromethyl)benzyl]amino}(oxo)acetic acid;
{(4-dodec-1-ynylbenzyl)[2-(trifluoromethyl)benzyl]amino}(oxo)acetic acid;
{{[4-(dodecyloxy)-1-naphthyl]methyl}[2-(trifluoromethyl)benzyl]amino}(oxo)acetic acid;
{[4-(octyloxy)benzyl][2-(trifluoromethyl)benzyl]amino}(oxo)acetic acid;
{(4-dec-1-ynylbenzyl)[2-(3,4-dichlorophenyl)ethyl]amino}(oxo)acetic acid;
[[2-(3,4-dichlorophenyl)ethyl](4-dodec-1-ynylbenzyl)amino](oxo)acetic acid;
([2-(3,4-dichlorophenyl)ethyl]{[4-(dodecyloxy)-1-naphthyl]methyl}amino)(oxo) acetic acid;
{[2-(3,4-dichlorophenyl)ethyl][4-(octyloxy)benzyl]amino}(oxo)acetic acid;
({4-[(4-hexylphenyl)ethynyl]benzyl}{1-methyl-1-[4-(trifluoromethyl)phenyl] ethyl} amino)(oxo)acetic acid;
{[4-(5-cyclohexylpent-1-ynyl)benzyl][4-(trifluoromethyl)benzyl]amino}(oxo)acetic acid;
{{3-[(4-hexylphenyl)ethynyl]benzyl}[4-(trifluoromethyl)benzyl]amino}(oxo)acetic acid;
{[4-(4-ethyl-3-hydroxyoct-1-ynyl)beazyl][4-(trifluoromethyl)benzyl]amino}-(oxo)-acetic acid;
{(2-dec-1-ynylbenzyl)[4-(trifluoromethyl)benzyl]amino}(oxo)acetic acid;
{(4-dec-1-ynylbenzyl)[4-(trifluoromethyl)benzyl]amino}(oxo)acetic acid, L-lysine salt;
{(4-dec-1-ynylbenzyl)[4-(trifluoromethyl)benzyl]amino}(oxo)acetic acid, tromethamine (i.e. (2-amino-2-hydroxymethyl)-1,3-propanediol) salt;
{(4-dec-1-ynylbenzyl)[4-(trifluoromethyl)benzyl]amino}(oxo)acetic acid, L-Arginine salt;
Sodium {(4-dec-1-ynylbenzyl)[4-(trifluoromethyl)benzyl]amino}(oxo)acetate.

The compounds of Formula (I) are useful in the prevention and/or treatment of cardiovascular disorders such as coronary obstruction and heart failure.

The compounds of Formula (I) are particularly useful in the prevention and/or treatment of chronic heart failure. In particular, compounds of the invention are particularly useful in the prevention and/or treatment of a cardiovascular disorder selected from endothelial dysfunction, endothelial NO production impairment and increased peripheral vasoconstriction in heart failure.

According to another embodiment, it is provided a use of a compound of Formula (I) for the preparation of a medicament for the prevention and/or treatment of cardiovascular disorders.

According to a preferred embodiment, it is provided a use of a compound of Formula (I) for the preparation of a medicament for the prevention and/or treatment of heart failure, particularly chronic heart failure.

According to a further embodiment, it is provided a use of a compound of Formula (I) for the preparation of a medicament for the prevention and/or treatment of endothelial dysfunction, endothelial NO production impairment and increased peripheral vasoconstriction in heart failure, particularly chronic heart failure.

According to another embodiment, it is provided a method for the prevention and/or treatment of cardiovascular disorders such as coronary obstruction, heart failure, including chronic heart failure, comprising the administration of a compound of Formula (I) in a patient in need thereof.

The methylene amide derivatives of the present invention may be prepared from readily available starting materials using general methods and procedures such as described in WO 03/064376.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

Methylene amide derivatives of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of these inventions can be administered by a variety of routes including oral, rectal, transdermal, subcutaneous, intravenous, intramuscular, and intranasal. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules or the like in the case of solid compositions. In such compositions, the substituted methylene amide derivative according to the invention is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate buffered saline or other injectable carriers known in the art. As above mentioned, substituted methylene amide derivatives of Formula (I) in such compositions is typically a minor component, frequently ranging between 0.05 to 10% by weight with the remainder being the injectable carrier and the like.

The above described components for orally administered or injectable compositions are merely representative. Further materials as well as processing techniques and the like are set out in Part 8 of Remington's Pharmaceutical Sciences, 17th Edition, 1985, Marck Publishing Company, Easton, Pennsylvania.

The compounds of this invention can also be administered in sustained release forms or from sustained release drug delivery systems. A description of representative sustained release materials can also be found in the incorporated materials in *Remington's Pharmaceutical Sciences.*

Preparation of pharmaceutical formulations of methylene amide derivatives of the present invention are described in WO 03/064376.

In the following the present invention shall be illustrated by means of some examples which are not construed to be viewed as limiting the scope of the invention.

### Brief description of the drawings:

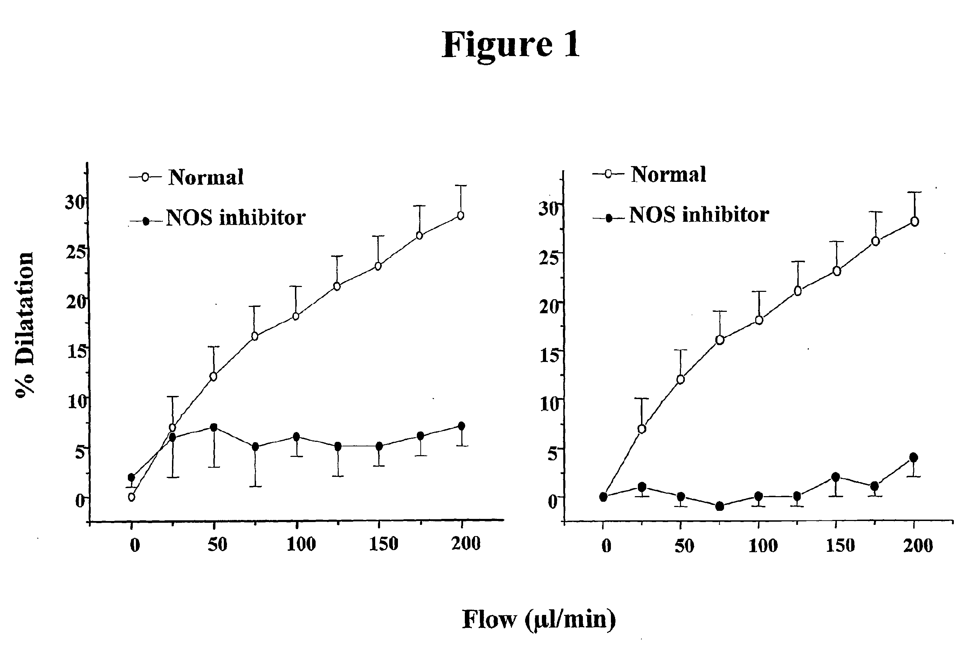

**Figure 1** shows the flow-mediated dilatation response of mesenteric arteries (% dilatation) versus intra-vascular flow (µl/min) in mice after pre-constriction by phenylephrine.
   The flow-mediated dilatation response in a normal mouse (open circles) is compared to the flow-mediated dilatation response of mesenteric arteries in either normal mouse subjected to an inhibitor of NO synthesis (left, black circles) or in a mouse wherein chronic heart failure (CHF) has been induced as described in Example 1 (right, black circles).

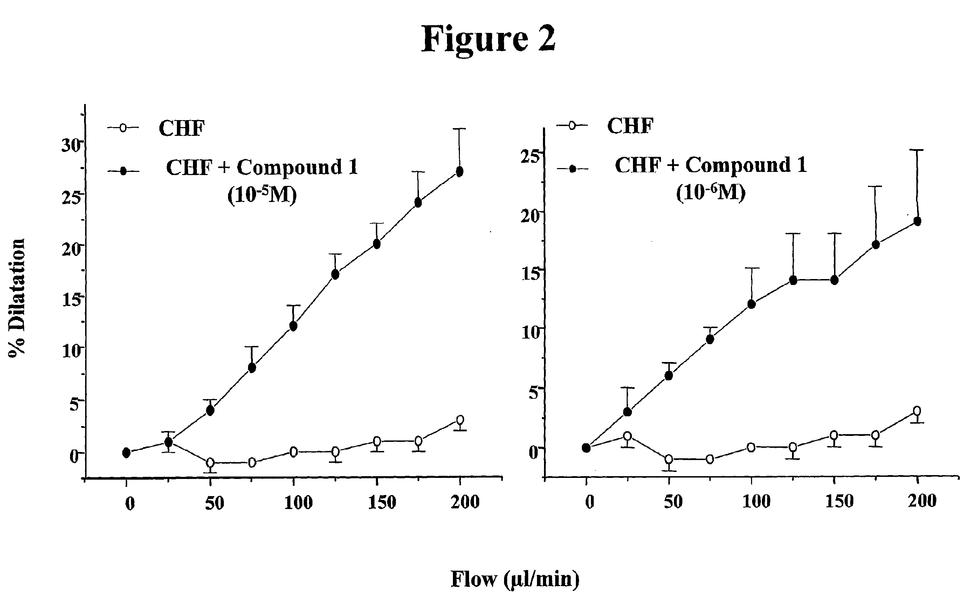

**Figure 2** shows the flow-mediated dilatation response of mesenteric arteries (% dilatation) versus intra-vascular flow (µl/min) after pre-constriction by phenylephrine, in mice wherein chronic heart failure (CHF) has been induced.
   The flow-mediated dilatation response in an untreated mouse presenting CHF (left and right open circles) is compared to the flow-mediated dilatation response of mesenteric arteries in a mouse presenting CHF after treatment with 10⁻⁵ M of compound (1) of the invention (left black circles) and with 10⁻⁶ M of compound (1) of the invention (right black circles) as described in Example 1.

The following abbreviations are hereinafter used in the accompanying examples:
**min** (minute), **µm** (micrometers), µl (microliters), **M** (molar), **ACE** (Angiotensin Converting Enzyme), **CHF** (Chronic Heart Failure), **FMD** (Flow-mediated Dilatation), **NO** (Nitric Oxide), **PTPs** (Protein -Tyrosine Phosphatases).

### Examples: Biological assays

In order to show the activity of compounds of Formula (I) in the restoration of flow-mediated vasodilatation after chronic heart failure, compounds of Formula (I) may be subjected to the following assay.

Experimentally, flow-induced release of NO in small peripheral arteries (i.e. arteries that contribute to the increased resistance in heart failure) may be assessed in vitro in isolated perfused, pressurized arteries such as small (<300µm diameter) mesenteric or hindquarter arteries.

### Example 1: Coronary artery occlusion model

Chronic heart failure is induced in C57BL6 mice (n=12) by coronary ligation according to the model developed in rats by Varin et al.,1999, Circulation, 99, 2951-2957. This model shows that chronic heart failure abolishes flow-induced, NO-mediated vasodilatation of small peripheral arteries, in a context of preserved NO-mediated response to acetylcholine.

Mice are anesthetized, ventilated and a thoracotomy (incision of the pleural cavity) is performed. The left main coronary artery is ligated close to its origin. This procedure leads to the development of an infarct involving around 40% of the left ventricle. Post infarction mortality in this mouse model is around 40-50%.

The development of heart failure is assessed by echocardiography in the closed chest state 7 days and 2 months after coronary ligation. Sham-operated (normal) mice are subjected to the same protocol except that the artery is not be ligated.

Six mice are present in each group and mice that survived myocardial infarction are further used in the study.

After two months, the mice is anesthetized and subjected to laparotomy (incision into the abdominal cavity).

A portion of the mesenteric artery (diameter 250-350 µm) is carefully isolated under a dissection microscope, and a 1.5-to 2.0-mm length segment is cannulated on both ends, transferred to an arteriograph (Living Systems Instrumentation) and pressurized to 60 mm Hg.

Flow-mediated vasodilatation (FMD), i.e. changes in vessel diameter in response to stepwise increases in intra-luminal flow (0-200 µl/min) is evaluated after preconstriction by phenylephrine (incubated 2min at 10⁻⁵ M) after a contraction plateau is reached, at baseline and after incubation with a compound of the invention:

### 1) Endothelial dysfunction in mouse heart failure

In this model of chronic heart failure, the dilatation response to an increase of blood-flow (flow-mediated dilatation) is roughly abolished (dilatation at flow 200 ml/min: sham: 24±2% and CHF: 5±1 %).

The inhibition of the dilatation response is similar as in the case of the administration of an inhibitor of NO synthesis (NG-nitro-L-arginine at 10⁻⁵M) in normal arteries as shown on Figure 1.

### 2) Flow-mediated dilatation restoration

In these arteries where chronic heart failure has been induced, flow-mediated dilatation can be restored to the normal level by the *in-vitro* incubation (30 min) of the arteries with {{4-[(4-hexylphenyl)ethynyl]benzyl}[4-(trifluoromethyl)benzyl]amino}(oxo)acetic acid **(1)** of the invention diluted in perfusion buffer (Krebs buffer) from a solution at 10⁻³ M to a final concentration of 10⁻⁵ M and 10⁻⁶ M, respectively as shown on Figure 2 left and right.

This example illustrates that compounds of the invention are able to acutely restore endothelial function in mice with chronic heart failure.

## Claims

1. Use of a methylene amide derivative of Formula (I) : 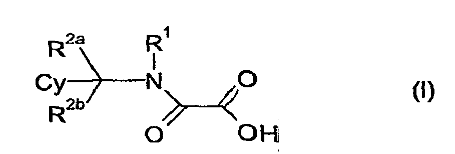 as well as its geometrical isomers, its optically active forms as enantiomers, diastereomers and its racemate forms, as well as pharmaceutically acceptable salts and pharmaceutically active derivatives thereof, wherein
R¹ is selected from (C₁-C₁₅)alkyl, (C₂-C₁₂)alkenyl, (C₂-C₁₂)alkynyl, aryl, heteroaryl, (3-8-membered)-cycloalkyl or heterocycloalkyl, (C₁-C₁₂)alkyl-aryl or (C₁-C₁₂)alkyl-heteroaryl, (C₂-C₁₂)alkenyl-aryl or -heteroaryl, (C₂-C₁₂)alkynyl-aryl and -heteroaryl;
R^{2a} and R^{2b} are each independently from each other selected from H and (C₁-C₁₂)alkyl;
Cy is selected from D and E;
D is selected from substituted thienyl and substituted phenyl wherein substituents are selected from phenyl, oxadiazole and 1 or 2 moieties selected from the group consisting of -NH-CO-R³, -SO₂-NR³R^{3'}, and -CO-NR³R^{3'};
E is selected from aryl, heteroaryl, (3-8-membered)-cycloalkyl and heterocycloalkyl wherein aryl, heteroaryl, (3-8-membered)-cycloalkyl and heterocycloalkyl are substituted by (C₂-C₁₈)alkynyl;
R³, R^{3'} are independently selected from H, (C₁-C₁₅)alkyl, (C₂-C₁₂)alkenyl, (C₂-C₁₂)alkynyl, aryl, heteroaryl, (3-8-membered)cycloalkyl or heterocycloalkyl, (C₁-C₁₂)alkyl aryl or heteroaryl, (C₂-C₁₂)alkenyl-aryl or -heteroaryl, (C₂-C₁₂)alkynyl-aryl or -heteroaryl;
for the preparation of a medicament for the treatment and/or prevention of cardiovascular disorders.

2. Use according to claim 1 wherein R^{2a} and R^{2b} are each H.

3. Use according to claims 1 or 2 wherein Cy is D.

4. Use according to any of the preceding claims wherein R^{3'} is H and R³ is selected from the group consisting of diphenyl-ethyl, dodecyl, octyl, 4-pentyl-benzyl, 4-phenoxy-phenethyl, ethyl-thiophen-2-yl, pentadecyl, tridecyl, hexyloxy-phenyl or (2-ethyl)-hexyl.

5. Use according to claim 1 wherein Cy is E.

6. Use according to claim 5 wherein E is phenyl, pyridinyl, naphthyl or benzofuranyl group, being substituted by B-R⁴ wherein B is ethynyl group and R⁴ is (C₆-C₁₆)alkyl, (3-8 membered) cycloalkyl, (C₁-C₁₂)alkyl-(3-8 membered) cycloalkyl, phenyl or (C₁-C₁₂)alkyl phenyl.

7. Use according to claim 6 wherein E is phenyl being substituted by B-R⁴ wherein B is ethynyl group and R⁴ is (C₆-C₁₆)alkyl.

8. Use according to any of the preceding claims wherein R¹ is a moiety -CH₂-A, or -CH₂-CH₂-A with A being an aryl, heteroaryl, (3-8-membered)heterocycloalkyl or (3-8-membered)cycloalkyl.

9. Use according to any of claims 1 to 7 wherein R¹ is A, with A being aryl, heteroaryl, (3-8-membered)heterocycloalkyl or (3-8-membered)cycloalkyl.

10. Use according to claims 8 or 9, wherein A is selected from the group consisting of phenyl, pyridinyl, benzo-1,3-dioxolenyl, biphenyl, naphthyl, quinoxalinyl, thiazolyl, thienyl, furanyl or a piperidinyl group, being optionally substituted by 1 or 2 cyano, halogen, NO₂, (C₁-C₆)alkoxy, aryloxy or heteroaryloxy, (C₁-C₆)thioalkoxy, (C₁-C₁₂)alkyl, (C₁-C₁₂)alkyl-X wherein X is halogen, (C₂-C₁₂)alkenyl, (C₂-C₁₂)alkynyl, aryl, heteroaryl, (3-8 membered) cycloalkyl or heterocycloalkyl, (C₁-C₁₂)alkyl aryl or heteroaryl, (C₂-C₁₂)alkenyl aryl or heteroaryl, (C₂-C₁₂)alkynyl aryl or heteroaryl, -COR³, -COOR³, -CO-NR³R^{3'}, -NHCOR³ wherein R³ is a (C₁-C₁₂)alkyl or (C₁-C₁₂)alkenyl, - SOR³, -SO₂R³, -SO₂NR³R^{3'} with R³, R^{3'} being independently from each other selected from the group consisting of H, straight or branched (C₁-C₁₂)alkyl, (C₂-C₁₂)alkenyl, (C₂-C₁₂)alkynyl, aryl, heteroaryl, (3-8-membered)-cycloalkyl or heterocycloalkyl.

11. Use according to any claims 1 to 4 or 8 to 10 wherein:
R^{2a} and R^{2b} are each H; R¹ is -CH₂-A, with A being phenyl or thienyl, optionally substituted by cyano, halogen, methoxy, hydroxy, phenoxy, -NO₂, trifluoromethyl; Cy is a thienyl, phenyl or biphenyl being substituted by -SO₂R³, -CO-NR³R^{3'} in which R^{3'} is H and R³ is (C₇-C₁₂)alkyl.

12. Use according to any claims 1 to 4 or 8 to 10 wherein:
R^{2a} and R^{2b} are each H; R¹ is-CH₂-A, with A being phenyl or thienyl, optionally substituted by cyano, halogen, methoxy, hydroxy, phenoxy, -NO₂, trifluoromethyl; Cy is a thienyl, phenyl or biphenyl being substituted by -SO₂R³, -CO-NR³R^{3'} in which R^{3'} is H and R³ is (C₇-C₁₅)alkyl.

13. Use according to any claims 1 to 4 or 8 to 10 wherein the methylene amide derivative is of Formula (I'): 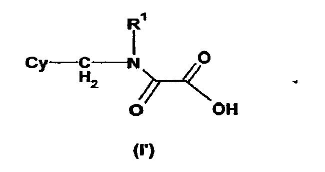 Wherein
R¹ is selected from the group consisting of phenyl, benzyl, phenethyl, 1-methylbenzyl which may be substituted by (C₁-C₆)alkyl group or a cycloalkyl group; Cy is a phenyl or a biphenyl group substituted with a moiety selected from the group consisting of - NH-CO-R³, -CO-NH-R³, or an oxadiazole group substituted with R³, wherein R³ is (C₇-C₁₅)alkyl.

14. Use according to claim 13 wherein R³ is (C₈-C₁₅)alkyl.

15. Use according to claims 13 and 14 wherein R³ is dodecyl.

16. Use according to any of the preceding claims wherein the methylene amide derivative is selected from the following group:
(benzyl{4-[(dodecylamino)carbonyl] benzyl}amino)(oxo)acetic acid;
oxo{{4-[(pentadecylamino)carbonyl]benzyl}[4-(trifluoromethyl)benzyl]amino}acetic acid;
(benzyl{4-[(pentadecylamino)carbonyl]benzyl}amino)(oxo)acetic acid;
(benzyl{4-[(tridecylamino)carbonyl]benzyl}amino)(oxo)acetic acid;
[benzyl(4-{[dodecyl(methyl)amino]carbonyl}benzyl)amino](oxo)acetic acid;
{(4-{[dodecyl(methyl)amino]carbonyl}benzyl)[4-(trifluoromethyl)benzyl]amino}-(oxo)acetic acid;
([1-(tert-butoxycarbonyl)-4-piperidinyl]{4-[(dodecylamino)carbonyl]benzyl}-amino)-(oxo)acetic acid;
{{4-[(dodecylamino)carbonyl]benzyl}[4-(trifluoromethyl)benzyl]amino}(oxo)acetic acid;
{{4-[(dodecylamino)carbonyl]benzyl}[3-(trifluoromethyl)benzyl]amino}(oxo)acetic acid;
({[1-(tert-butoxycarbonyl)-4-piperidinyl]methyl}{4-[(dodecylamino)carbonyl]-benzyl} amino)(oxo)acetic acid;
oxo{[4-(tridecanoylamino)benzyl][4-(trifluoromethyl)benzyl]arnino}acetic acid;
[benzyl(4-{[4-(hexyloxy)benzoyl]amino}benzyl)amino](oxo)acetic acid;
oxo{[4-(trifluoromethyl)benzyl][4-(10-undecenoylamino)benzyl]amino}acetic acid;
oxo{{4-[(9E)-9-tetradecenoylamino]benzyl}[4-(trifluoromethyl)benzyl]amino}acetic acid;
{benzyl[4-(tridecanoylamino)benzyl]axnino}(oxo)acetic acid;
{{4-[(2-hydroxydodecyl)amino]benzyl}[4-(trifluoromethyl)benzyl]amino}-(oxo)-acetic acid;
oxo{[4-(trifluoromethyl)benzyl][4-(3-undecyl-1,2,4-oxadiazol-5-yl)benzyl]-amino}-acetic acid;
{({5-[(dodecylamino)sulfonyl]-2-thienyl}methyl)(4-(trifluoromethyl)benzyl]amino}-(oxo)acetic acid;
[{4-[(dodecylamino)carbonyl]benzyl}({1-[(4-methoxyphenyl)sulfonyl]-4-piperi-dinyl} methyl)amino](oxo)acetic acid;
[{4-[(dodecylamino)carbonyl]benzyl}(2-carboxy-1-phenylethyl)amino](oxo)acetic acid;
[{4-[(dodecylamino)carbonyl]benzyl}(2-methoxy-1-methylethyl)amino](oxo)acetic acid;
(4-bromo{4-[(dodecylamino)carbonyl]benzyl}anilino)(oxo)acetic acid;
({4-[(dodecylamino)carbonyl]benzyl}anilino)(oxo)acetic acid;
([2-(3-chlorophenyl)ethyl] {4-[(dodecylamino)carbonyl]benzyl}amino)(oxo)acetic acid;
{{4-[(dodecylamino)carbonyl]benzyl}[2-(3-methoxyphenyl)ethyl]amino}(oxo)acetic acid;
{{4-[(dodecylamino)carbonyl]benzyl}[(d,1)-trans-2-phenylcyclopropyl]amino}-(oxo) acetic acid;
([(d,1)-trans-2-(benzyloxy)cyclopentyl]{4-[(dodecylamino)carbonyl]benzyl}-amino)-(oxo)acetic acid;
({4-[(dodecylamino)carbonyl]benzyl}-4-phenoxyanilino)(oxo)acetic acid;
[{4-[(dodecylamino)carbonyl]benzyl}(1,2,3,4-tetrahydro-1-naphtbalenyl)amino]-(oxo) acetic acid;
((1-benzyl-4-piperidinyl){4-[(dodecylamino)carbonyl]benzyl}amino)(oxo)acetic acid;
{{4-[(dodecylamino)carbonyl]benzyl}[2-(4-phenoxyphenyl)ethyl]amino}(oxo)acetic acid;
{{4-[(dodecylamino)carbonyl]benzyl}[2-(2-phenoxyphenyl)ethyl]amino}(oxo)acetic acid;
((2-[1,1'-biphenyl]-4-ylethyl){4-[(dodecylamino)carbonyl]benzyl}amino)(oxo)acetic acid;
(([1,1'-biphenyl]-3-ylmethyl){4-[(dodecylamino)carbonyl]benzyl}amino)(oxo)acetic acid;
(3-(benzyloxy){4-[(dodecylamino)carbonyl]benzyl}anilino)(oxo)acetic acid;
([4-(benzoylamino)benzyl]{4-[(dodecylamino)carbonyl]benzyl}amino)(oxo)acetic acid;
N-(carboxycarbonyl)-N-{4-[(dodecylamino)carbonyl]benzyl}-3-phenyl-beta-alanine;
{{4-[(dodecylamino)carbonyl]benzyl}[4-(1,2,3-thiadiazol-4-yl)benzyl]amino}-(oxo) acetic acid;
[{4-[(dodecylamino)carbonyl]benzyl}(4-pentylbenzyl)amino](oxo)acetic acid;
[{4-[(dodecylamino)carbonyl]benzyl}(1-phenylethyl)amino](oxo)acetic acid;
{{4-[(dodecylamino)carbonyl]benzyl}[1-(1-naphthyl)ethyl]amino}(oxo)acetic acid;
(benzyl{3-[(dodecylamino)carbonyl]benzyl}amino)(oxo)acetic acid;
{{3-[(dodecylamino)carbonyl]benzyl}[4-(methylsulfonyl)benzyl]amino}(oxo)acetic acid;
((3-cyanobenzyl){3-[(dodecylamino)carbonyl]benzyl)amino)(oxo)acetic acid;
{{3-[(dodecylamino)carbonyl]benzyl}[4-(trifluoromethyl)benzyl]amino}(oxo)acetic acid;
[(4-chlorobenzyl)(3-{[(4-pentylbenzyl)amino]carbonyl)benzyl)amino](oxo)acetic acid;
oxo {4-({[2-(2-thienyl)ethyl]amino}carbonyl)benzyl][4-(trifluoromethyl)-benzyl] amino}acetic acid;
{benzyl[(3'-{[(2,2-diphenylethyl)amino]carbonyl}[1,1'-biphenyl]-4-yl)methyl]-amino} (oxo)acetic acid;
{(3-cyanobenzyl)[(3'-{[(2,2-diphenylethyl)amino]carbonyl}[1,1'-biphenyl]-4-yl) methyl]amino}(oxo)acetic acid;
{(4-chlorobenzyl)[(3'-{[(2,2-diphenylethyl)amino]carbonyl}[1,1'-biphenyl]-4-yl) methyl]amino}(oxo)acetic acid;
{[(3'-{[(2,2-diphenylethyl)amino]carbonyl}[1,1'-biphenyl]-4-yl)methyl][4-(trifluoromethyl)benzyl]amino}(oxo)acetic acid;
((3-cyanobenzyl){[3'-({[2-(4-phenoxyphenyl)ethyl]amino}carbonyl)[1,1'-biphenyl]-4-yl]methyl}amino)(oxo)acetic acid;
oxo{{[3'-({[2-(4-phenoxyphenyl)ethyl]amino}carbonyl)[1,1'-biphenyl]-4-yl]methyl}-[4-(trifluoromethyl)benzyl]amino}acetic acid;
[(3-cyanobenzyl)({3'-[(octylamino)carbonyl][1,1'-biphenyl]-4-yl}methyl)amino]-(oxo) acetic acid;
[(4-chlorobenzyl)({3'-[(octylamino)carbonyl][1,1'-biphenyl]-4-yl}methyl)amino]-(oxo) acetic acid;
{({3'-[(octylamino)carbonyl][1,1'-biphenyl]-4-yl}methyl)[4-(trifluoromethyl)-benzyl] amino}(oxo)acetic acid;
{(3-cyanobenzyl)[(3'-{[(3-phenylpropyl)amino]carbonyl}[1,1'-biphenyl]-4-yl)methyl] amino}(oxo)acetic acid;
[(3-cyanobenzyl)({3'-[(dodecylamino)carbonyl][1,1'-biphenyl]-4-yl}methyl)-amino]-(oxo)acetic acid;
[(4-chlorobenzyl)({3'-[(dodecylamino)carbonyl][1,1'-biphenyl]-4-yl}methyl)-amino]-(oxo)acetic acid;
{({3'-[(dodecylamino)carbonyl][1,1'-biphenyl]-4-yl}methyl)[4-(trifluoromethyl)-benzyl]amino}(oxo)acetic acid;
{benzyl[(3'-{[(4-pentylbenzyl)amino]carbonyl}[1,1'-biphenyl]-4-yl)methyl]amino}-(oxo)acetic acid;
{(3-cyanobenzyl)[(3'-{[(4-pentylbenzyl)amino]carbonyl}[1,1'-biphenyl]-4-yl)-methyl] amino}(oxo)acetic acid;
{(4-chlorobenzyl)[(3'-{[(4-pentylbenzyl)amino]carbonyl}[1,1'-biphenyl]-4-yl)-methyl] amino}(oxo)acetic acid;
oxo{[(3'-{[(4-pentylbenzyl)amino]carbonyl}[1,1'-biphenyl]-4-yl)methyl][4-(trifluoromethyl)benzyl]amino}acetic acid;
oxo{[(3'-{[(4-phenylbutyl)amino]carbonyl}[1,1'-biphenyl]-4-yl)methyl][4-(trifluoromethyl)benzyl]amino}acetic acid;
{(3-cyanobenzyl)[(3'-{[(2-mesitylethyl)amino]carbonyl}[1,1'-biphenyl]-4-yl)-methyl] amino}(oxo)acetic acid;
{(4-chlorobenzyl)[(3'-{[(2-mesitylethyl)amino]carbonyl}[1,1'-biphenyl]-4-yl)-methyl] amino}(oxo)acetic acid;
{[(3'-{[(2-mesitylethyl)amino]carbonyl}[1,1'-biphenyl]-4-yl)methyl][4-(trifluoromethyl)benzyl]amino}(oxo)acetic acid;
((4-chlorobenzyl){[3'-({[2-(4-metlnoxyphenyl)ethyl]amino}carbonyl)[1,1'-biphenyl]-4-yl]methyl}amino)(oxo)acetic acid;
[{4-[(dodecylamino)carbonyl]benzyl}(4-methoxybenzyl)amino](oxo)acetic acid;
{{4-[(dodecylamino)carbonyl]benzyl}[4-(methylsulfonyl)benzyl]amino}(oxo)acetic acid;
[{3-[(dodecylamino)carbonyl]benzyl}(4-methoxybenzyl)amino](oxo)acetic acid;
{{3-[(dodecylamino)carbonyl]benzyl}[3-(trifluoromethyl)benzyl]amino}(oxo)acetic acid;
({4-[(dodecylamino)carbonyl]benzyl}{[6-(trifluoromethyl)-3-pyridinyl]methyl}-amino)(oxo)acetic acid;
4-[((carboxycarbonyl){3-[(dodecylamino)carbonyl]benzyl}amino)methyl]benzoic acid;
({3-[(dodecylamino)carbonyl]benzyl}{4-[hydroxy(oxido)amino]benzyl}-amino) (oxo) acetic acid;
[{3-[(dodecylamino)carbonyl]benzyl}(2-fluorobenzyl)amino](oxo)acetic acid;
[{3-[(dodecylamino)carbonyl]benzyl}(2-pyridinylmethyl)amino](oxo)acetic acid;
[{3-[(dodecylamino)carbonyl]benzyl}(3-thienylmethyl)amino](oxo)acetic acid;
[{3-[(dodecylamino)carbonyl]benzyl}(4-hydroxybenzyl)amino](oxo)acetic acid;
[{3-[(dodecylamino)carbonyl]benzyl}(4-phenoxybenzyl)amino](oxo)acetic acid;
({3-[(dodecylamino)carbonyl]benzyl}{[6-(trifluoromethyl)-3-pyridinyl]methyl}-amino)(oxo)acetic acid;
3-[((carboxycarbonyl){3-[(dodecylamino)carbonyl]benzyl}amino)methyl]benzoic acid;
5-[((carboxycarbonyl){3-[(dodecylamino)carbonyl]benzyl}amino)methyl]-2-thiophenecarboxylic acid;
({4-[(dodecylamino)carbonyl]benzyl}{4-[hydroxy(oxido)amino]-benzyl}-amino)-(oxo)acetic acid;
((1,3-benzodioxol-5-ylmethyl){4-[(dodecylamino)carbonyl]-benzyl}amino)-(oxo)-acetic acid;
[{4-[(dodecylamino)carbonyl]benzyl}(2-fluorobenzyl)amino](oxo)acetic acid;
[{4-[(dodecylamino)carbonyl]benzyl}(4-phenoxybenzyl)amino](oxo)acetic acid;
4-[((carboxycarbonyl){4-[(dodecylamino)carbonyl]benzyl}amino)methyl]benzoic acid;
5-[((carboxycarbonyl){4-[(dodecylamino)carbonyl]benzyl}amino)methyl]-2-thiophene carboxylic acid;
[{3-[(dodecylamino)carbonyl]benzyl}(2-thienylmethyl)amino](oxo)acetic acid;
[{4-[(dodecylamino)carbonyl]benzyl}(isopropyl)amino](oxo)acetic acid;
((3,5-dichlorobenzyl){4-[(dodecylamino)carbonyl]benzyl}amino)(oxo)acetic acid;
[(3,5-dichlorobenzyl)(4-{[(3,3-diphenylpropyl)amino]carbonyl}-benzyl)amino]-(oxo) acetic acid;
[(4-{[(2-[1,1'-biphenyl]-4-ylethyl)amino]carbonyl}benzyl)(3,5-dichlorobenzyl)-amino] (oxo)acetic acid;
[(1,3-benzodioxol-5-ylmethyl)(4-{[(2-[1,1'-biphenyl]-4-ylethyl)amino]carbonyl}-benzyl)amino](oxo)acetic acid;
(2,3-dihydro-1H-inden-1-yl{4-[(dodecylamino)carbonyl]benzyl}amino)(oxo)acetic acid;
{2,3-dihydro-1H-inden-1-yl[4-({[2-(4-phenoxyphenyl)ethyl]amino}-carbonyl)-benzyl] amino}(oxo)acetic acid;
[{4-[(dodecylamino)carbonyl]benzyl}(4-pyridinylmethyl)amino](oxo)acetic acid;
([4-(dimethylamino)benzyl]{4-[(dodecylamino)carbonyl]benzyl}amino)(oxo)acetic acid;
[{4-[(dodecylamino)carbonyl]benzyl}(3-pyridinylmethyl)amino](oxo)acetic acid;
((4-cyanobenzyl){4-[(dodecylamino)carbonyl]benzyl}amino)(oxo)acetic acid;
[{4-[(dodecylamino)carbonyl]benzyl}(1,3-thiazol-2-ylmethyl)amino](oxo)acetic acid;
({4-[(dodecylamino)carbonyl]benzyl}{[2-(4-morpholinyl)-1,3-thiazol-5-yl]methyl}-amino)(oxo)acetic acid;
[{3-[(dodecylamino)carbonyl]benzyl}(4-pyridinylmethyl)amino](oxo)acetic acid;
[{3-[(dodecylamino)carbonyl]benzyl}(3-pyridinylmethyl)amino](oxo)acetic acid;
[{3-[(dodecylamino)carbonyl]benzyl}(3-hydroxybenzyl)amino](oxo)acetic acid;
((4-cyanobenzyl){3-[(dodecylamino)carbonyl]benzyl]amino)(oxo)acetic acid;
[{3-[(dodecylamino)carbonyl]benzyl}(1,3-thiazol-2-ylmethyl)amino](oxo)acetic acid;
({3-[(dodecylamino)carbonyl]benzyl}{[2-(4-morpholinyl)-1,3-thiazol-5-yl]methyl]-amino)(oxo)acetic acid;
((1,3-benzodioxol-5-ylmethyl){3-[(dodecylamino)carbonyl]-benzyl}amino)-(oxo) acetic acid;
[{4-[(dodecylamino)carbonyl]benzyl}(2-thienylmethyl)amino](oxo)acetic acid;
[{4-[(dodecylamino)carbonyl]benzyl}(2-pyridinylmethyl)amino](oxo)acetic acid;
[{4-[(dodecylamino)carbonyl]benzyl}(3-thienyhnethyl)amino](oxo)acetic acid;
[{4-[(dodecylamino)carbonyl]benzyl}(4-hydroxybenzyl)amino](oxo)acetic acid;
3-[((carboxycarbonyl){4-[(dodecylamino)carbonyl]benzyl}amino)methyl]benzoic acid;
[cyclopentyl({5-[(dodecylamino)sulfonyl]-2-thienyl}methyl)amino](oxo)acetic acid;
[benzyl({5-[(dodecylamino)sulfonyl]-2-thienyl}methyl)amino](oxo)acetic acid;
(({5-[(dodecylamino)sulfonyl]-2-thienyl]methyl){3-[hydroxy(oxido)amino]-benzyl}-amino)(oxo)acetic acid;
[({5-[(dodecylamino)sulfonyl]-2-thienyl}methyl)(4-methoxybenzyl)amino]-(oxo)-acetic acid;
[({5-[(dodecylamino)sulfonyl]-2-thienyl}methyl)(2-fluorobenzyl)amino](oxo)acetic acid;
{({5-[(dodecylamino)sulfonyl]-2-thienyl}methyl)[4-(methylsulfonyl)-benzyl]-amino}(oxo)acetic acid;
[({5-[(dodecylamino)sulfonyl]-2-thienyl}methyl)(4-phenoxybenzyl)amino]-(oxo)-acetic acid;
4-{[(carboxycarbonyl)({5-[(dodecylamino)sulfonyl]-2-thienyl}methyl)-amino]-methyl}benzoic acid;
(({5-[(dodecylamino)sulfonyl]-2-thienyl}methyl){[6-(trifluoromethyl)-3-pyridinyl]-methyl}amino)(oxo)acetic acid;
{({5-[(dodecylamino)sulfonyl]-2-thienyl}methyl)[3-(trifluoromethyl)benzyl]amino}-(oxo)acetic acid;
[(3-chlorobenzyl)({5-[(dodecylamino)sulfonyl]-2-thienyl}methyl)amino](oxo)acetic acid;
{[(5-{[(3,3-diphenylpropyl)amino]sulfonyl}-2-thienyl)methyl][3-(trifluoromethyl)-benzyl]amino}(oxo)acetic acid;
{(3-chlorobenzyl)[(5-{[(3,3-diphenylpropyl)amino]sulfonyl}-2-thienyl)methyl]-amino}(oxo)acetic acid;
oxo{{[5-({[2-(4-phenoxyphenyl)ethyl]amino}sulfonyl)-2-thienyl]methyl] [3-(trifluoromethyl)benzyl]amino} acetic acid;
((3-chlorobenzyl){[5-({[2-(4-phenoxyphenyl)ethyl]amino}sulfonyl)-2-thienyl]-methyl}amino)(oxo)acetic acid;
{[(5-{[(2-[1,1'-biphenyl]-4-ylethyl)amino]sulfonyl]-2-thienyl)methyl][3-(trifluoromethyl)benzyl]amino}(oxo)acetic acid;
(({1-[(cyclohexylamino)carbonyl]-4-piperidinyl}methyl){4-[(dodecylamino)-carbonyl] benzyl}amino)(oxo)acetic acid;
([(1-{[4-(dimethylamino)anilino]carbonyl}-4-piperidinyl)methyl]{4-[(dodecyl-amino) carbonyl]benzyl}amino)(oxo)acetic acid;
{{4-[(dodecylamino)carbonyl]benzyl}[(1-hexanoyl-4-piperidinyl)methyl]-amino}-(oxo)acetic acid;
({4-[(dodecylamino)carbonyl]benzyl}{[1-(3-iodobenzoyl)-4-piperidinyl]methyl}-amino)(oxo)acetic acid;
{{4-[(dodecylamino)carbonyl]benzyl}[(1-{(2E)-3-[3-(trifluoromethyl)phenyl]-2-propenoyl}-4-piperidinyl)methyl]amino}(oxo)acetic acid;
({4-[(dodecylamino)carbonyl]benzyl}{[1-(2-quinoxalinylcarbonyl)-4-piperidinyl]-methyl}amino)(oxo)acetic acid;
[({1-[(4-methoxyphenyl)sulfonyl]-4-piperidinyl}methyl)(4-{[(4-phenoxybenzyl) amino]carbonyl}benzyl)amino](oxo)acetic acid;
[{[1-(3-iodobenzoyl)-4-piperidinyl]methyl}(4-{[(4-phenoxybenzyl)amino]-carbonyl} benzyl)amino](oxo)acetic acid;
oxo{(4-{[(4-phenoxybenzyl)amino]carbonyl}benzyl)[(1-{(2E)-3-[3-(trifluoromethyl) phenyl]-2-propenoyl}-4-piperidinyl)methyl]amino} acetic acid;
{{4-[(dodecylamino)carbonyl]phenyl}[2-(methoxycarbonyl)benzyl]-amino}(oxo) acetic acid;
[[4-({[2-(1,1'-biphenyl-4-yl)ethyl]amino}carbonyl)-2-bromobenzyl](4-iodobenzyl)-amino](oxo)acetic acid;
[(2-bromo-4-{[(4-pentylbenzyl)amino]carbonyl}benzyl)(4-iodobenzyl)amino]-(oxo) acetic acid;
[{2-bromo-4-[(dodecylamino)carbonyl]benzyl}(4-iodobenzyl)amino](oxo)acetic acid;
[(2,6-dibromo-4-{[(4-pentylbenzyl)amino]carbonyl}benzyl)(4-iodobenzyl)amino]-(oxo)acetic acid;
((4-iodobenzyl){[4'-({[2-(4-phenoxyphenyl)ethyl]amino}carbonyl)-1,1'-biphenyl-4-yl] methyl}amino)(oxo)acetic acid;
{[2-bromo-4-({[2-(4-phenoxyphenyl)ethyl]amino}carbonyl)benzyl][(4'-fluoro-1,1'-biphenyl-3-yl)methyl]amino}(oxo)acetic acid;
{[4-({[2-(1,1'-biphenyl-4-yl)ethyl]amino}carbonyl)-2-bromobenzyl][(4'-fluoro-1,1'-biphenyl-3-yl)methyl]amino}(oxo)acetic acid;
{(2-bromo-4-{[(4-pentylbenzyl)amino]carbonyl}benzyl)[(4'-fluoro-1,1'-biphenyl-3-yl) methyl]amino}(oxo)acetic acid;
{[2,6-dibromo-4-({[2-(4-phenoxyphenyl)ethyl]amino}carbonyl)benzyl][(4'-fluoro-1,1'-biphenyl-3-yl)methyl]amino}(oxo)acetic acid;
{[4-({[2-(1,1'-biphenyl-4-yl)ethyl]amino}carbonyl)-2,6-dibromobenzyl][(4'-fluoro-1,1'-biphenyl-3-yl)methyl]amino}(oxo)aceric acid;
{(2,6-dibromo-4-{[(4-pentylbenzyl)amino]carbonyl}benzyl)[(4'-fluoro-1,1'-biphenyl-3-yl)methyl]amino}(oxo)acetic acid;
{{2,6-dibromo-4-[(dodecylamino)carbonyl]benzyl}[(4'-fluoro-1,1'-biphenyl-3-yl) methyl]amino}(oxo)acetic acid;
([(4'-fluoro-1,1'-biphenyl-3-yl)methyl]{[4'-({[2-(4-phenoxyphenyl)ethyl]amino}-carbonyl)-1,1'-biphenyl-4-yl]methyl}amino)(oxo)acetic acid;
{({4'-[(dodecylamino)carbonyl]-1,1'-biphenyl-4-yl}methyl)[(4'-fluoro-1,1'-biphenyl-3-yl)methyl]amino}(oxo)acetic acid;
{(2-bromo-4-{[(4-pentylbenzyl)amino]carbonyl}benzyl)[2-(trifluoromethoxy)-benzyl] amino}(oxo)acetic acid;
{(2,6-chbromo-4-{[(4-pentylbenzyl)amino]carbonyl}benzyl)[2-(trifluoromethoxy)-benzyl]amino}(oxo)acetic acid;
oxo{{[4'-({[2-(4-phenoxyphenyl)ethyl]amino}carbonyl)-1,1'-biphenyl-4-yl]methyl}-[2-(trifluoromethoxy)benzyl]amino}acetic acid;
{({4'-[(dodecylamino)carbonyl]-1,1'-biphenyl-4-yl}methyl)[2-(trifluoromethoxy)-benzyl]amino}(oxo)acetic acid;
[[2-bromo-4-({[2-(4-phenoxyphenyl)ethyl]amino}carbonyl)benzyl](3-phenoxy-benzyl) amino](oxo)acetic acid;
[[4-({[2-(1,1'-biphenyl-4-yl)ethyl]amino}carbonyl)-2-bromobenzyl](3-phenoxybenzyl) amino](oxo)acetic acid;
[(2-bromo-4-{[(4-pentylbenzyl)amino]carbonyl}benzyl)(3-phenoxybenzyl)-amino] (oxo)acetic acid;
[[2,6-dibromo-4-({[2-(4-phenoxyphenyl)ethyl]amino}carbonyl)benzyl](3-phenoxy benzyl)amino](oxo)acetic acid;
[[4-({[2-(1,1'-biphenyl-4-yl)ethyl]amino}carbonyl)-2,6-dibromobenzyl](3-phenoxybenzyl)amino](oxo)acetic acid;
[(2,6-dibromo-4-{[(4-pentylbenzyl)amino]carbonyl}benzyl)(3-phenoxybenzyl)-amino] (oxo)acetic acid;
[{2,6-dibromo-4-[(dodecylamino)carbonyl]benzyl}(3-phenoxybenzyl)amino](oxo)-acetic acid;
oxo((3-phenoxybenzyl){[4'-({[2-(4-phenoxyphenyl)ethyl]amino}carbonyl)-1,1'-biphenyl-4-yl]methyl}amino)acetic acid;
oxo[[(4'-{[(4-pentylbenzyl)amino]carbonyl}-1,1'-biphenyl-4-yl)methyl](3-phenoxy benzyl)amino]acetic acid;
[({4'-[(dodecylamino)carbonyl]-1,1'-biphenyl-4-yl}methyl)(3-phenoxybenzyl)-amino] (oxo)acetic acid;
[[2-bromo-4-({[2-(4-phenoxyphenyl)ethyl]amino}carbonyl)benzyl](2-iodobenzyl)-amino](oxo)acetic acid;
[[4-({[2-(1,1'-biphenyl-4-yl)ethyl]amino}carbonyl)-2-bromobenzyl](2-iodobenzyl)-amino](oxo)acetic acid;
[(2-bromo-4-{[(4-pentylbenzyl)amino]carbonyl}benzyl)(2-iodobenzyl)amino]-(oxo) acetic acid;
[{2-bromo-4-[(dodecylamino)carbonyl]benzyl}(2-iodobenzyl)amino](oxo)acetic acid
([2-bromo-4-({[2-(4-phenoxyphenyl)ethyl]amino}carbonyl)benzyl]{[2'-(trifluoromethyl)-1,1'-biphenyl-4-yl]methyl}amino)(oxo)acetic acid;
([4-({[2-(1,1'-biphenyl-4-yl)ethyl]amino}carbonyl)-2-bromobenzyl]{[2'-(trifluoromethyl)-1,1'-biphenyl-4-yl]methyl}amino)(oxo)acetic acid;
((2-bromo-4-{[(4-pentylbenzyl)amino]carbonyl}benzyl){[2'-(trifluoromethyl)-1,1'-biphenyl-4-yl]methyl}amnino)(oxo)acetic acid;
((2-bromo-4-{[(4-pentylbenzyl)aimno]carbonyl}benzyl){[2'-(triftuofomethyl)-1,1'-biphenyl-4-yl]methyl}amino)(oxo)acetic acid;
({2-bromo-4-[(dodecylamino)carbonyl]benzyl}{[2'-(trifluoromethyl)-1,1'-biphenyl-4-yl]methyl}amino)(oxo)acetic acid;
([4-({[2-(1,1'-biphenyl-4-yl)ethyl]amino}carbonyl)-2,6-dibromobenzyl]{[2'-(tri-fluoromethyl)-1,1'-biphenyl-4-yl]methyl}amino)(oxo)acetic acid;
((2,6-dibromo-4-{[(4-pentylbenzyl)amino]carbonyl]benzyl}{[2'-(trifluoromethyl)-1,1'-biphenyl-4-yl]methyl}amino)(oxo)acetic acid;
({2,6-dibromo-4-[(dodecylamino)carbonyl]benzyl}{[2'-(trifluoromethyl)-1,1'-biphenyl-4-yl]methyl}amino)(oxo)acetic acid;
(({4'-[(dodecylamino)carbonyl]-1,1'-biphenyl-4-yl}methyl){[2'-(trifluoromethyl)-1,1'-biphenyl-4-yl]methyl}amino)(oxo)acetic acid;
[[4-({[2-(1,1'-biphenyl-4-yl)ethyl]amino}carbonyl)-2-bromobenzyl](1,1'-biphenyl-2-ylmethyl)amino](oxo)acetic acid;
[(1,1'-biphenyl-2-ylmethyl)(2-bromo-4-{[(4-pentylbenzyl)amino]carbonyl}benzyl)-amino](oxo)acetic acid;
((1,1'-biphenyl-2-ylmethyl){2-bromo-4-[(dodecylamino)carbonyl]benzyl}-amino)-(oxo)acetic acid;
{(1,1'-biphenyl-2-ylmethyl)[2,6-dibromo-4-({[2-(4-phenoxyphenyl)ethyl]amino}-carbonyl)benzyl]amino}(oxo)acetic acid;
[[4-({([2-(1,1'-biphenyl-4-yl)ethyl]amino}carbonyl)-2,6-dibromobenzyl](1,1'-biphenyl-2-ylmethyl)amino](oxo)acetic acid;
[(1,1'-biphenyl-2-ylmethyl)(2,6-dibromo-4-{[(4-pentylbenzyl)amino]carbonyl}-benzyl)amino](oxo)acetic acid;
((1,1'-biphenyl-2-ylmethyl){2,6-dihromo-4-[(dodecylatnino)carbonyl]benzyl}-amino) (oxo)acetic acid;
{(2-bromo-4-{[(4-pentylbenzyl)amino]carbonyl}benzyl)[4-(trifluoromethoxy)-benzyl]amino}(oxo)acetic acid;
{{2-bromo-4-[(dodecylamino)carbonyl]benzyl}[4-(trifluoromethoxy)benzyl]amino}-(oxo)acetic acid;
{(2,6-dibromo-4-{[(4-pentylbenzyl)amino]carbonyl}benzyl)[4-(trifluoromethoxy)-benzyl]amino}(oxo)acetic acid;
{(2-bromo-4-{[(4-pentylbeazyl)amino]carbonyl}benzyl)[3-(trifluoromethoxy)-benzyl] amino}(oxo)acetic acid;
{{2-bromo-4-[(dodecylamino)carbonyl]benzyl}[3-(trifluoromethoxy)benzyl]amino}-(oxo)acetic acid;
{(2,6-dibromo-4-{[(4-pentylbenzyl)amino]carbonyl}benzyl)[3-(trifluoromethoxy)-benzyl]amino}(oxo)acetic acid;
{{2,6-dibromo-4-[(dodecylamino)carbonyl]benzyl}[3-(trifluoromethoxy)benzyl]-amino}(oxo)acetic acid;
{({4'-[(dodecylamino)carbonyl]-1,1'-biphenyl-4-yl]methyl)[3-(trifluoromethoxy)-benzyl]amino}(oxo)acetic acid;
[[2-bromo-4-({[2-(4-phenoxyphenyl)ethyl]amino}carbonyl)benzyl](4-phenoxybenzyl)amino](oxo)acetic acid;
[[4-({[2-(1,1'-biphenyl-4-yl)ethyl]amino}carbonyl)-2-bromobenzyl](4-phenoxybenzyl)amino](oxo)acetic acid;
[(2-bromo-4-[(4-pentylbenzyl)amino]carbonyl}benzyl)(4-phenoxybenzyl)-amino] (oxo)acetic acid;
[{2-bromo-4-[(dodecylamino)carbonyl]benzyl}(4-phenoxybenzyl)amino](oxo)acetic acid;
[[4-({[2-(1,1'-biphenyl-4-yl)ethyl]amino}carbonyl)-2,6-dibromobenzyl](4-phenoxybenzyl)amino](oxo)acetic acid;
[(2,6-dibromo-4-{[(4-pentylbenzyl)amino]carbonyl}benzyl)(4-phenoxybenzyl)-amino] (oxo)acetic acid;
{[4-({[2-(1,1'-biphenyl-4-yl)ethyl]amino}carbonyl)-2-bromobenzyl][4-(trifluoromethyl)benzyl]amino}(oxo)acetic acid;
{(2-bromo-4-{[(4-pentylbenzyl)amino]carbonyl}benzyl)[4-(trifluoromethyl)-benzyl]-amino}(oxo)acetic acid;
{{2-bromo-4-[(dodecylamino)carbonyl]benzyl}[4-(trifluoromethyl)benzyl]amino}-(oxo)acetic acid;
{(2,6-dibromo-4-{[(4-pentylbenzyl)amino]carbonyl}benzyl)[4-(trifluoromethyl)-benzyl]amino}(oxo)acetic acid;
{{2,6-dibromo-4-[(dodecylamino)carbonyl]benzyl}[4-(trifluoromethyl)benzyl]-amino} (oxo)acetic acid;
oxo{[(4'-{[(4-pentylbenzyl)amino]carbonyl}-1,1'-biphenyl-4-yl)methyl][4-(trifluoromethyl)benzyl]amino}acetic acid;
{{2-bromo-4-[(dodecylamino)carbonyl]benzyl}[3-(trifluoromethyl)benzyl]-amino}(oxo)acetic acid;
{{2,6-dibromo-4-[(dodecylamino)carbonyl]benzyl}[3-(trifluoromethyl)benzyl]-amino} (oxo)acetic acid;
oxo{[(4'-{[(4-pentylbenzyl)amino]carbonyl}-1,1'-biphenyl-4-yl)methyl][3-(trifluoro methyl)benzyl]amino}acetic acid;
{(4-dibenzo[b,d]furan-4-ylbenzyl)[4-(trifluoromethyl)benzyl]amino}(oxo)acetic acid;
{(4-dibenzo[b,d]furan-4-ylbenzyl)[4-(trifluoromethyl)benzyl]amino}(oxo)acetic acid,
N-methyl-D-glucamine (i.e. 1-deoxy-1-(methylamino)glucitol) salt;
({4-[(dodecylamino)carbonyl]benzyl}{1-[4-(trifluoromethyl)phenyl]ethyl}amino)-(oxo)acetic acid;
({4-[(dodecylamino)carbonyl]benzyl}{1-[4-(trifluoromethyl)phenyl]ethyl}amino)-(oxo)acetic acid, N-methyl-D-glucamine (i.e. 1-deoxy-1-(methylamino)glucitol) salt;
{({4'-[(octylamino)carbonyl]-1,1'-biphenyl-4-yl}methyl)[4-(trifluoromethyl)benzyl]-amino}(oxo)acetic acid;
oxo{(4-tetradec-1-ynylbenzyl)[4-(trifluoromethyl)benzyl]amino}acetic acid;
{(4-dodec-1-ynylbenzyl)[4-(trifluoromethyl)benzyl]amino}(oxo)acetic acid;
{{4-[(dodecylamino)carbonyl]benzyl}[4-(tnfluoromethyl)phenyl]amino}(oxo)acetic acid;
[{4-[(dodecylamino)carbonyl]benzyl}(2-methoxyphenyl)amino](oxo)acetic acid;
((1,2-diphenylethyl){4-[(dodecylamino)carbonyl]benzyl}amino)(oxo)acetic acid;
N-(carboxycarbonyl)-N-{4-[(dodecylamino)carbonyl]benzyl}-L-phenylalanine;
[{4-[(dodecylamino)carbonyl]benzyl}(3-phenoxyphenyl)amino](oxo)acetic acid;
[{4-[(dodecylamino)carbonyl]benzyl}(2-isopropoxyphenyl)amino](oxo)acetic acid;
[{4-[(dodecylamino)carbonyl]benzyl}(4-iodophenyl)amino](oxo)acetic acid;
{{4-[(dodecylamino)carbonyl]benzyl}[3-fluoro-4-(trifluoromethyl)benzyl]-amino} (oxo)acetic acid;
((3-chloro-2-methylphenyl){4-[(dodecylamino)carbonyl]benzyl}amino)(oxo)acetic acid;
4'-((carboxycarbonyl){4-[(dodecylamino)carbonyl]benzyl}amimo)-1,1'-biphenyl-2-carboxylic acid;
((2,4-dichlorobenzyl){4-[(dodecylamino)carbonyl]benzyl}amino)(oxo)acetic acid;
[{4-[(dodecylamino)carbonyl]benzyl}(1-phenylpropyl)amino](oxo)acetic acid;
([2-(4-chlorophenyl)propyl]{4-[(dodecylamino)carbonyl]benzyl}amino)(oxo)acetic acid;
[{4-[(dodecylamino)carbonyl]benzyl}(4-isopropoxyphenyl)amino](oxo)acetic acid;
([4-(benzyloxy)phenyl]{4-[(dodecylamino)carbonyl]benzyl}amino)(oxo)acetic acid;
{{4-[(dodecylamino)carbonyl]benzyl}[2-(trifluoromethyl)benzyl]amino}(oxo)acetic acid;
[{4-[(dodecylamino)carbonyl]benzyl}(2-methoxybenzyl)amino](oxo)acetic acid;
([(1R)-1-(4-chlorophenyl)ethyl]{4-[(dodecylamino)carbonyl]benzyl}amino)-(oxo) acetic acid;
((3,4-dichlorobenzyl){4-[(dodecylamino)carbonyl]benzyl}amino)(oxo)acetic acid
((1-benzothien-3-ylmethyl){4-[(dodecylamino)carbonyl]benzyl}amino)(oxo)acetic acid;
([2-(2,6-dichlorophenyl)ethyl]{4-[(dodecylamino)carbonyl]benzyl}amino)(oxo)acetic acid;
({4-[(dodecylamino)carbonyl]benzyl}{2-[3-(trifluoromethyl)phenyl]ethyl}-amino)-(oxo)acetic acid;
{{4-[(dodecylamino)carbonyl]benzyl}[2-(3-fluorophenyl)ethyl]amino}(oxo)acetic acid;
([(1S)-1-(4-chlorophenyl)ethyl]{4-[(dodecylamino)carbonyl]benzyl}amino)(oxo)-acetic acid;
{{4-[(dodecylamino)carbonyl]benzyl}[(1S)-1-phenylethyl]amino}(oxo)acetic acid;
{{4-[(dodecylamino)carbonyl]benzyl}[(1R)-1-phenylethyl]amino}(oxo)acetic acid;
([3-(benzyloxy)phenyl]{4-[(dodecylamino)carbonyl]benzyl}amino)(oxo)acetic acid;
N-(carboxycarbonyl)-N-{4-[(dodecylamino)carbonyl]benzyl}-D-phenylalanine;
{{4-[(dodecylamino)carbonyl]phenyl}[4-(trifluoromethyl)benzyl]amino}(oxo)acetic acid;
{{4-[(dodecylamino)carbonyl]phenyl}[4-(trifluoromethyl)benzyl]amino}(oxo)acetic acid, N-methyl-D-glucamine (i.e. 1-deoxy-1-(methylamino)glucitol) salt;
oxo{{1-[4-(trifluoromethyl)phenyl]ethyl}[4-(3-undecyl-1,2,4-oxadiazol-5-yl)benzyl] amino}acetic acid;
oxo{{1-[4-(trifluoromethyl)phenyl]ethyl}[4-(3-undecyl-1,2,4-oxadiazol-5-yl)benzyl]
amino}acetic acid, N-methyl-D-glucamine (i.e. 1-deoxy-1-(methylamino)glucitol) salt;
([(2-butyl-1-benzofuran-3-yl)methyl]{4-[(dodecylamino)carbonyl]benzyl}-amino) (oxo)acetic acid;
{(1-{4-[(dodecylamino)carbonyl]phenyl}ethyl)[4-(trifluoromethyl)benzyl]amino}-(oxo)acetic acid;
{(1-{4-[(dodecylamino)carbonyl]phenyl}ethyl)[4-(trifluoromethyl)benzyl]amino}-(oxo)acetic acid, N-methyl-D-glucamine (i.e. 1-deoxy-1-(methylamino)glucitol) salt;
{(4-{[(4-octylphenyl)amino]carbonyl}benzyl)[4-(trifluoromethyl)benzyl]-amino} (oxo) acetic acid;
{(3-chlorobenzyl)[4-(3-undecyl-1,2,4-oxadiazol-5-yl)benzyl]amino}(oxo)acetic acid;
{(3-chlorobenzyl)[4-(3-undecyl-1,2,4-oxadiazol-5-yl)benzyl]amino}(oxo)acetic acid,
N-methyl-D-glucamine (i.e. 1-deoxy-1-(methylamino)glucitol) salt;
{{cyclopentyl[4-(trifluoromethyl)phenyl]methyl}[4-(tridecanoylamino)benzyl]-amino}(oxo)acetic acid;
oxo([4-(trifluoromethyl)benzyl]{[4-(3-undecyl-1,2,4-oxadiazol-5-yl)-1-naphthyl]-methyl}amino)acetic acid;
oxo([4-(trifluoromethyl)beazyl]{[4-(3-undecyl-1,2,4-oxadiazol-5-yl)-1-naphtbyl]-methyl}amino)acetic acid, N-methyl-D-glucamine (i.e. 1-deoxy-1-(methylamino)-glucitol) salt;
{{cyclopentyl[4-(trifluoromethyl)phenyl]methyl}[4-(3-undecyl-1,2,4-oxadiazol-5-yl)benzyl]amino}(oxo)acetic acid;
{{cyclopentyl[4-(trifluoromethyl)phenyl]methyl}[4-(3-undecyl-1,2,4-oxadiazol-5-yl)benzyl]amino}(oxo)acetic acid, N-methyl-D-glucamine (i.e. 1-deoxy-1-(methyl-amino)glucitol) salt;
{(4-dibenzo[b,d]furan-4-ylphenyl)[4-(trifluoromethyl)benzyl]amino}(oxo)acetic acid;
{(4-dibenzo[b,d]furan-4-ylphenyl)[4-(triiluoromethyl)benzyl]amino}(oxo)acetic acid,
N-methyl-D-glucamine (i.e. 1-deoxy-1-(methylamino)glucitol) salt;
{[4-(octyloxy)benzyl][4-(trifluoromethyl)benzyl]amino}(oxo)acetic acid;
{[4-(octyloxy)benzyl][4-(trifluoromethyl)benzyl]amino} (oxo)acetic acid, N-methyl-D-glucamine (i.e. 1-deoxy-1-(methylainino)glucitol) salt;
[[2-(3-chlorophenyl)ethyl](4-dec-1-ynylbenzyl)amino](oxo)acetic acid;
([2-(3-chlorophenyl)ethyl]{4-[(1Z)-dec-1-enyl]benzyl}amino)(oxo)acetic acid;
{[2-(3-chlorophenyl)ethyl][4-(3-undecyl-1,2,4-oxadiazol-5-yl)benzyl]amino}(oxo)-acetic acid;
{[2-(3-chlorophenyl)ethyl][4-(3-undecyl-1,2,4-oxadiazol-5-yl)benzyl]amino}(oxo)-acetic acid, N-methyl-D-glucamine (i.e. 1-deoxy-1-(methylamino)glucitol) salt;
oxo{(1R)-1-[4-(trifluoromethyl)phenyl]ethyl}[4-(3-undecyl-1,2,4-oxadiazol-5-yl)benzyl]amino}acetic acid;
oxo{{(1R)-1-[4-(trifluoromethyl)phenyl]ethyl}[4-(3-undecyl-1,2,4-oxadiazol-5-yl)benzyl]amino}acetic acid, N-methyl-D-glucamine (i.e. 1-deoxy-1-(methylamino)-glucitol) salt;
oxo {[4-(trifluoromethyl)phenyl][4-(3-undecyl-1,2,4-oxadiazol-5-yl)benzyl]amino}-acetic acid;
oxo{[4-(trifluoromethyl)phenyl][4-(3-undecyl-1,2,4-oxadiazol-5-yl)benzyl]amino}-acetic acid, N-methyl-D-glucamine (i.e. 1-deoxy-1-(methylamino)glucitol) salt;
oxo{{(1S)-1-[4-(trifluoromethyl)phenyl]ethyl}[4-(3-undecyl-1,2,4-oxadiazol-5-yl)benzyl]amino}acetic acid;
oxo{{(1S)-1-[4-(trifluoromethyl)phenyl]ethyl}[4-(3-undecyl-1,2,4-oxadiazol-5-yl)benzyl]amino}acetic acid, N-methyl-D-glucamine (i.e. 1-deoxy-1-(methylamino)-glucitol) salt;
[(3-chlorobenzyl)(4-dec-1-ynylbenzyl)amino](oxo)acetic acid;
[(3-chlorobenzyl)(4-dec-1-ynylbenzyl)amino](oxo)acetic acid, N-methyl-D-glucamine (i.e. 1-deoxy-1-(methylaamino)glucitol) salt;
([2-(3-chlorophenyl)ethyl](4-oct-1-ynylbenzyl)amino](oxo)acetic acid;
[[2-(3-chlorophenyl)ethyl](4-oct-1-ynylbenzyl)amino](oxo)acetic acid, N-methyl-D-glucamine (i.e. 1-deoxy-1-(methylamino)glucitol) salt;
{(4-dec-1-ynylbenzyl)[4-(trifluoromethyl)phenyl]amino}(oxo)acetic acid;
((4-dec-1-ynylbenzyl){1-[4-(trifluoromethyl)phenyl]ethyl}amino)(oxo)acetic acid;
((4-der-1-ynylbenzyl){1-[4-(trifluoromethyl)phenyl]ethyl}amino)(oxo)acetic acid, N-methyl-D-glucamine (i.e. 1-deoxy-1-(methylamino)glucitol) salt;
{{1-methyl-1-[4-(trifluoromethyl)phenyl]ethyl}[4-(3-undecyl-1,2,4-oxadiazol-5-yl) benzyl]amino}(oxo)acetic acid;
{{1-methyl-1-[4-(trifluoromethyl)phenyl]ethyl}[4-(3-undecyl-1,2,4-oxadiazol-5-yl) benzyl]amino}(oxo)acetic acid, N-methyl-D-glucamine (i.e. 1-deoxy-1-(methylamino)glucitol) salt;
{[2-(3-chlorophenyl)ethyl][4-(3-octyl-1,2,4-oxadiazol-5-yl)benzyl]amino}(oxo)acetic acid;
{[2-(3-chlorophenyl)ethyl][4-(3-octyl-1,2,4-oxadiazol-5-yl)benzyl]amino}(oxo)acetic acid, N-methyl-D-glucamine (i.e. 1-deoxy-1-(methylamino)glucitol) salt;
{[4-(3-octyl-1,2,4-oxadiazol-5-yl)benzyl][4-(trifluoromethyl)benzyl]amino}-(oxo) acetic acid;
{[4-(3-octyl-1,2,4-oxadiazol-5-yl)benzyl][4-(trifluoromethyl)benzyl]amino}-(oxo)-acetic acid, N-methyl-D-glucamine (i.e. 1-deoxy-1-(methylamino)glucitol) salt;
{{[4-(dodecyloxy)-1-naphthyl]methyl}[4-(trifluoromethyl)benzyl]amino}(oxo)acetic acid;
{{[4-(dodecyloxy)-1-naphthyl]methyl}[4-(trifluoromethyl)benzyl]amino}(oxo)acetic acid, N-methyl-D-glucamine (i.e. 1-deoxy-1-(methylamino)glucitol) salt [(4-bromobenzyl)(4-oct-1-ynylbenzyl)amino](oxo)acetic acid;
[{4-[(dodecylamino)carbonyl]benzyl}(2-hydroxy-1-phenylethyl)amino](oxo)acetic acid;
((4-dec-1-ynylbenzyl){1-methyl-1-[4-(trifluoromethyl)phenyl]ethyl}amino)(oxo)-acetic acid;
((4-dec-1-ynylbenzyl){1-methyl-1-[4-(trifluoromethyl)phenyl]ethyl}amino)(oxo)-acetic acid, N-methyl-D-glucamine (i.e. 1-deoxy-1-(methylamino)glucitol) salt;
oxo {{4-[(9Z)-tetradec-9-enoylamino]benzyl}[4-(trifluoromethyl)benzyl]amino}-acetic acid;
{(4-dec-1-ynylbenzyl)[4-(trifluoromethyl)benzyl]amino}(oxo)acetic acid;
oxo {[4-(trifluoromethyl)benzyl][3-(3-undecyl-1,2,4-oxadiazol-5-yl)benzyl]-amino}acetic acid;
oxo{[4-(trifluoromethyl)benzyl][3-(3-undecyl-1,2,4-oxadiazol-5-yl)benzyl]amino}-acetic acid, N-methyl-D-glucamine (i.e. 1-deoxy-1-(methylamino)glucitol) salt;
{(4-dodecylbenzyl)[4-(trifluoromethyl)benzyl]amino}(oxo)acetic acid;
{(4-dodecylbenzyl)[4-(trifluoromethyl)benzyl]amino}(oxo)acetic acid, N-methyl-D-glucamine (i.e. 1-deoxy-1-(methylamino)glucitol) salt;
{[4-({[(2-butyl-1-benzofuran-3-yl)methyl]amino}carbonyl)benzyl][4-(trifluoromethyl)benzyl]amino}(oxo)acetic acid;
{(4-{[4-(benzyloxy)benzoyl]amino}benzyl)[4-(trifluoromethyl)benzyl]amino}-(oxo) acetic acid;
{(3,5-dichlorobenzyl)[4-(tridecanoylamino)benzyl]amino}(oxo)acetic acid;
{(3,5-dichlorobenzyl)[4-(tridecanoylamino)benzyl]amino}(oxo)acetic acid, N-methyl-D-glucamine (i.e. 1-deoxy-1-(methylamino)glucitol) salt;
{{4-[(4-octylphenyl)ethynyl]benzyl}[4-(trifluoromethyl)benzyl]amino}(oxo)acetic acid;
oxo {[4-(trifluoromethyl)benzyl][4-(5-undecyl-1,2,4-oxadiazol-3-yl)benzyl]amino}-acetic acid;
oxo{[4-(trifluoromethyl)benzyl][4-(5-undecyl-1,2,4-oxadiazol-3-yl)benzyl]amino}-acetic acid, N-methyl-D-glucamine (i.e. 1-deoxy-1-(methylamino)glucitol) salt;
{{4-[2-(4-octylphenyl)ethyl]benzyl}[4-(trifluoromethyl)benzyl]amino}(oxo)acetic acid;
{(4-{[4-(heptyloxy)phenyl]ethynyl}benzyl)[4-(trifluoromethyl)benzyl]amino}-(oxo)acetic acid;
{{4-[(4-butylphenyl)ethynyl]benzyl}[4-(trifluoromethyl)benzyl]amino}(oxo)acetic acid;
{{4-[(4-hexylphenyl)ethynyl]benzyl}[4-(trifluoromethyl)benzyl]amino}(oxo)acetic acid;
{{4-[(4-hexylphenyl)ethynyl]benzyl}[4-(trifluoromethyl)benzyl]amino}(oxo)acetic acid, N-methyl-D-glucamine (i.e. 1-deoxy-1-(methylamino)glucitol) salt;
oxo{(4-{[4-(pentyloxy)phenyl]ethynyl}benzyl)[4-(trifluoromethyl)benzyl]-amino}-acetic acid;
oxo{{4-[(4-propylphenyl)ethynyl]benzyl}[4-(trifluoromethyl)benzyl]amino}acetic acid;
[[2-(3-chlorophenyl)ethyl](4-dodec-1-ynylbenzyl)amino](oxo)acetic acid;
[[2-(3-chlorophenyl)ethyl](4-dodec-1-ynylbenzyl)amino](oxo)acetic acid, N-methyl-D-glucamine (i.e. 1-deoxy-1-(methylamino)glucitol) salt;
{(4-oct-1-ynylbenzyl)[4-(trifluoromethyl)benzyl]amino}(oxo)acetic acid;
{[4-(11-hydroxyundec-1-ynyl)benzyl][4-(trifluoromethyl)benzyl]amino}(oxo)acetic acid;
{[4-(11-methoxy-11-oxoundec-1-ynyl)benzyl][4-(trifluoromethyl)benzyl]amino}-(oxo)acetic acid;
11-[4-({(carboxycarbonyl)[4-(trifluoromethyl)benzyl]amino}methyl)phenyl]undec-10-ynoic acid;
{(4-{[4-(benzyloxy)phenyl]ethynyl}benzyl)[4-(trifluoromethyl)benzyl]amino}-(oxo) acetic acid;
{(4-{2-[4-(heptyloxy)phenyl]ethyl}benzyl)[4-(trifluoromethyl)benzyl]amino}(oxo)-acetic acid;
{{4-[2-(4-butylphenyl)ethyl]benzyl}[4-(trifluoromethyl)benzyl]amino}(oxo)acetic acid;
{{4-[2-(4-hexylphenyl)ethyl]benzyl}[4-(trifluoromethyl)benzyl]amino}(oxo)acetic acid;
{{4-[2-(4-hexylphenyl)ethyl]benzyl}[4-(trifluoromethyl)benzyl]amino}(oxo)acetic acid, N-methyl-D-glucamine (i.e. 1-deoxy-1-(methylamino)glucitol) salt;
oxo{(4-{2-[4-(pentyloxy)phenyl]ethyl}benzyl)[4-(trifluoromethyl)benzyl]-amino} acetic acid;
oxo{{4-[2-(4-propylphenyl)ethyl]benzyl}[4-(trifluoromethyl)benzyl]amino}acetic acid;
11-[4-({(carboxycarbonyl)[4-(trifluoromethyl)benzyl]amino}methyl)phenyl]-undecanoic acid;
{[4-(11-hydroxyundecyl)benzyl][4-(trifluoromethyl)benzyl]amino}(oxo)acetic acid;
{(4-dodec-1-ynylbenzyl)[4-(trifluoromethyl)phenyl]amino}(oxo)acetic acid;
{(4-dodec-1-ynylbenzyl)[4-(trifluoromethyl)phenyl]amino}(oxo)acetic acid, N-methyl-D-glucamine (i.e. 1-deoxy-1-(methylamino)glucitol) salt;
oxo([4-(trifluoromethyl)benzyl]{4-[2-(3-undecyl-1,2,4-oxadiazol-5-yl)ethyl]benzyl}-amino)acetic acid;
oxo([4-(trifluoromethyl)benzyl]{4-[2-(3-undecyl-1,2,4-oxadiazol-5-yl)ethyl]benzyl}-amino)acetic acid, N-methyl-D-glucamine (i.e. 1-deoxy-1-(methylamino)glucitol) salt;
{{4-[2-(3-octyl-1,2,4-oxadiazol-5-yl)ethyl]benzyl}[4-(trifluoromethyl)benzyl]-amino} (oxo)acetic acid;
{{4-[2-(3-octyl-1,2,4-oxadiazol-5-yl)ethyl]benzyl}[4-(trifluoromethyl)benzyl]-amino}(oxo)acetic acid, N-methyl-D-glucamine (i.e. 1-deoxy-1-(methylamino) glucitol) salt;
{{4-[(4-octylbenzoyl)amino]benzyl}[4-(trifluoromethyl)benzyl]amino}(oxo)acetic acid;
{{4-[(4-octylbenzoyl)amino]benzyl}[4-(trifluoromethyl)benzyl]amino}(oxo)acetic acid, N-methyl-D-glucamine (i.e. 1-deoxy-1-(methylamino)glucitol) salt;
oxo{[(1-tridecanoylpiperidin-4-yl)methyl][4-(trifluoromethyl)benzyl]amino}acetic acid;
{{[1-(4-octylbenzoyl)piperidin-4-yl]methyl}[4-(trifluoromethyl)benzyl]-amino}-(oxo)acetic acid;
{{[1-(4-octylbenzoyl)piperidin-4-yl]methyl}[4-(trifluoromethyl)benzyl]amino}-(oxo) acetic acid, N-methyl-D-glucamine (i.e. 1-deoxy-1-(methylamino)glucitol) salt;
{[(3-dec-1-ynyl-1-benzofuran-5-yl)methyl][4-(trifluoromethyl)benzyl]amino}-(oxo) acetic acid;
{[(3-dodec-1-ynyl-1-benzofuran-5-yl)methyl][4-(trifluoromethyl)benzyl]amino]-(oxo) acetic acid;
oxo{({3-[(4-propylphenyl)ethynyl]-1-benzofuran-5-yl}methyl)[4-(trifluoromethyl)-benzyl]aminol acetic acid;
[(4-dodec-1-ynylbenzyl)(4-fluorobenzyl)amino](oxo)acetic acid;
[bis(4-oct-1-ynylbenzyl)amino](oxo)acetic acid;
{[(6-dodec-1-ynylpyridin-3-yl)methyl][4-(trifluoromethyl)benzyl]amino}(oxo)acetic acid;
{(3-dodec-1-ynylbenzyl)[4-(trifluoromethyl)benzyl]amino}(oxo)acetic acid;
{[2-(2-fluorophenyl)ethyl][4-(3-undecyl-1,2,4-oxadiazol-5-yl)benzyl]amino}-(oxo) acetic acid;
{[2-(2-fluorophenyl)ethyl][3-(3-undecyl-1,2,4-oxadiazol-5-yl)benzyl]amino}-(oxo) acetic acid;
{[2-(2-fluorophenyl)ethyl][4-(3-octyl-1,2,4-oxadiazol-5-yl)benzyl]amino}(oxo)acetic acid;
{[2-(3,4-dichlorophenyl)ethyl][4-(3-undecyl-1,2,4-oxadiazol-5-yl)benzyl]amino}-(oxo)acetic acid;
{[2-(3,4-dichlorophenyl)ethyl][3-(3-undecyl-1,2,4-oxadiazol-5-yl)benzyl]amino}-(oxo)acetic acid;
{[2-(3,4-dichlorophenyl)ethyl][4-(3-octyl-1,2,4-oxadiazol-5-yl)benzyl] amino}(oxo)acetic acid;
{[2-(1,1'-biphenyl-4-yl)ethyl][4-(3-undecyl-1,2,4-oxadiazol-5-yl)benzyl]amino}-(oxo) acetic acid;
{[2-(1,1'-biphenyl-4-yl)ethyl][3-(3-undecyl-1,2,4-oxadiazol-5-yl)benzyl]amino}-(oxo) acetic acid;
{[2-(1,1'-biphenyl-4-yl)ethyl][4-(3-octyl-1,2,4-oxadiazol-5-yl)benzyl]amino}-(oxo) acetic acid;
oxo{5,6,7,8-tetrahydronaphthalen-1-yl[4-(3-undecyl-1,2,4-oxadiazol-5-yl)benzyl]-amino} acetic acid;
oxo{5,6,7,8-tetrahydronaphthalen-1-yl[3-(3-undecyl-1,2,4-oxadiazol-5-yl)benzyl]-amino}acetic acid;
[[4-(3-octyl-1,2,4-oxadiazol-5-yl)benzyl](5,6,7,8-tetrahydronaphthalen-1-yl)amino]-(oxo)acetic acid;
{(1,1'-biphenyl-3-ylmethyl)[4-(3-undecyl-1,2,4-oxadiazol-5-yl)benzyl]amino}-(oxo) acetic acid;
{(1,1'-biphenyl-3-ylmethyl)[3-(3-undecyl-1,2,4-oxadiazol-5-yl)benzyl]amino}-(oxo) acetic acid;
{(1,1'-biphenyl-3-ylmethyl)[4-(3-octyl-1,2,4-oxadiazol-5-yl)benzyl]amino}-(oxo)-acetic acid;
{(1-benzothien-3-ylmethyl)[4-(3-undecyl-1,2,4-oxadiazol-5-yl)benzyl]amino}-(oxo)-acetic acid;
{(1-benzothien-3-ylmethyl)[3-(3-undecyl-1,2,4-oxadiazol-5-yl)benzyl]amino}(oxo)-acetic acid;
{(1-benzothien-3-ylmethyl)[4-(3-octyl-1,2,4-oxadiazol-5-yl)benryl]amino}(oxo)-acetic acid;
oxo{[2-(trifluoromethyl)benzyl][4-(3-undecyl-1,2,4-oxadiazol-5-yl)benzyl]amino}-acetic acid;
oxo {[2-(trifluoromethyl)benzyl][3-(3-undecyl-1,2,4-oxadiazol-5-yl)benzyl]amino} acetic acid;
{[4-(3-octyl-1,2,4-oxadiazol-5-yl)benzyl][2-(trifluoromethyl)benzyl]amino}(oxo)-acetic acid;
oxo{[3-(trifluoromethyl)benzyl][4-(3-undecyl-1,2,4-oxadiazol-5-yl)benzyl]-amino}-acetic acid;
oxo{[3-(trifluoromethyl)benzyl][3-(3-undecyl-1,2,4-oxadiazol-5-yl)benzyl]-amino}-acetic acid;
{[4-(3-octyl-1,2,4-oxadiazol-5-yl)benzyl][3-(trifluoromethyl)benzyl]amino}-(oxo)-acetic acid;
{(2-methoxybenzyl)[4-(3-undecyl-1,2,4-oxadiazol-5-yl)benzyl]amino}(oxo)acetic acid
{(2-methoxybenzyl)[3-(3-undecyl-1,2,4-oxadiazol-5-yl)benzyl]amino}(oxo)-acetic acid;
{(2-methoxybenzyl)[4-(3-octyl-1,2,4-oxadiazol-5-yl)benzyl]amino}(oxo)acetic acid;
oxo{{4-[(trifluoromethyl)sulfonyl]benzyl}[4-(3-undecyl-1,2,4-oxadiazol-5-yl)-benzyl] amino}acetic acid;
oxo{{4-[(trifluoromethyl)sulfonyl]benzyl}[3-(3-undecyl-1,2,4-oxadiazol-5-yl)-benzyl] amino}acetic acid;
([4-(3-octyl-1,2,4-oxadiazol-5-yl)benzyl] {4-[(trifluoromethyl)-sulfonyl]benzyl}-amino)(oxo)acetic acid;
{1,3-benzodioxol-5-yl[4-(3-undecyl-1,2,4-oxadiazol-5-yl)benzyl]amino}(oxo)acetic acid;
{1,3-benzodioxol-5-yl[3-(3-undecyl-1,2,4-oxadiazol-5-yl)benzyl]amino}(oxo)acetic acid;
{1,3-benzodioxol-5-yl[4-(3-octyl-1,2,4-oxadiazol-5-yl)benzyl]amino}(oxo)acetic acid;
{[(4-dodec-1-ynyl-1-naphthyl)methyl][4-(trifluoromethyl)benzyl]amino}(oxo)acetic acid;
{[(4-dec-1-ynyl-1-naphthyl)methyl][4-(trifluoromethyl)benzyl]amino}(oxo)acetic acid;
{[(4-dec-1-ynyl-1-naphthyl)methyl][4-(trifluoromethyl)benzyl]amino}(oxo)acetic acid;
oxo{[4-(trifluoromethyl)benzyl][4-(4-undecyl-1,3-thiazol-2-yl)benzyl]amino}acetic acid;
{(4-dec-1-ynylbenzyl)[2-(2-fluorophenyl)ethyl]amino}(oxo)acetic acid;
{(4-dodec-1-ynylbenzyl)[2-(2-fluorophenyl)ethyl]amino}(oxo)acetic acid;
{{[4-(dodecyloxy)-1-naphthyl]methyl}[2-(2-fluorophenyl)ethyl]amino}(oxo)acetic acid;
{[2-(2-fluorophenyl)ethyl][4-(octyloxy)benzyl]amino}(oxo)acetic acid;
{(4-dec-1-ynylbenzyl)[2-(trifluoromethyl)benzyl]amino}(oxo)acetic acid;
{(4-dodec-1-ynylbenzyl)[2-(trifluoromethyl)benzyl]amino}(oxo)acetic acid;
{{[4-(dodecyloxy)-1-naphthyl]methyl}[2-(trifluoromethyl)benzyl]amino}(oxo)acetic acid;
{[4-(octyloxy)benzyl][2-(trifluoromethyl)benzyl]arnino}(oxo)acetic acid;
{(4-dec-1-ynylbenzyl)[2-(3,4-dichlorophenyl)ethyl]amino}(oxo)acetic acid;
[[2-(3,4-dichlorophenyl)ethyl](4-dodec-1-ynylbenzyl)amino](oxo)acetic acid;
([2-(3,4-dichlorophenyl)ethyl]{[4-(dodecyloxy)-1-naphthyl]methyl}amino) (oxo) acetic acid;
{[2-(3,4-dichlorophenyl)ethyl][4-(octyloxy)benzyl]amino}(oxo)acetic acid;
({4-[(4-hexylphenyl)ethynyl]benzyl}{1-methyl-1-[4-(trifluoromethyl)phenyl] ethyl} amino)(oxo)acetic acid;
{[4-(5-cyclohexylpent-1-ynyl)benzyl][4-(trifluoromethyl)benzyl]amino}(oxo)acetic acid;
{{3-[(4-hexylphenyl)ethynyl]benzyl}[4-(trifluoromethyl)benzyl]amino}(oxo)acetic acid;
{[4-(4-ethyl-3-hydroxyoct-1-ynyl)benzyl][4-(trifluoromethyl)benzyl]amino}-(oxo)-acetic acid;
{(2-dec-1-ynylbenzyl)[4-(trifluoromethyl)benzyl]amino}(oxo)acetic acid;
{(4-dec-1-ynylbenzyl)[4-(trifluoromethyl)benzyl]amino}(oxo)acetic acid, L-lysine salt;
{(4-dec-1-ynylbenryl)[4-(trifluoromethyl)benzyl]amino}(oxo)acetic acid, tromethamine (i.e. (2-amino-2-hydroxymethyl)-1,3-propanediol) salt;
{(4-dec-1-ynyIbenzyl)[4-(trifluoromethyl)benzyl]anvno}(oxo)acetic acid, L-Arginine salt;
Sodium {(4-dec-1-ynylbenzyl)[4-(trifluoromethyl)benzyl]amino}(oxo)acetate.

17. Use according to any of the preceding claims wherein the cardiovascular disorder is heart failure.

18. Use according to any of the preceding claims wherein the cardiovascular disorder is chronic heart failure.

19. Use according to any of the preceding claims wherein the cardiovascular disorder is endothelial dysfunction.

## Patentansprüche

1. Verwendung eines Methylenamid-Derivats der Formel (I): 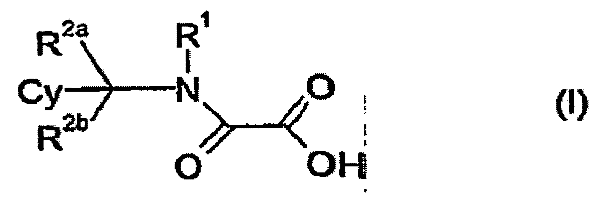 sowie deren geometrische Isomere, deren optisch aktive Formen, wie etwa Enantiomere, Diastereomere und deren Racematformen, als auch pharmazeutisch zulässige Salze und pharmazeutisch wirksame Derivate davon, worin
R¹ ausgewählt ist aus (C₁-C₁₅)-Alkyl, (C₂-C₁₂)-Alkenyl, (C₂-C₁₂)-Alkynyl, Aryl, Heteroaryl, (3-8-gliedrigem)-Cycloalkyl oder Heterocycloalkyl, (C₁-C₁₂)-Alkyl-aryl oder (C₁-C₁₂)-Alkyl-heteroaryl, (C₂-C₁₂)-Alkenyl-aryl oder -heteroaryl, (C₂-C₁₂)-Alkynyl-aryl und -heteroaryl;
R^{2a} und R^{2b} jeweils unabhängig voneinander aus H und (C₁-C₁₂)-Alkyl ausgewählt sind;
Cy aus D und E ausgewählt ist;
D ausgewählt ist aus substituiertem Thienyl und substituiertem Phenyl, worin die Substituenten aus Phenyl, Oxadiazol ausgewählt sind und 1 oder 2 Reste aus der Gruppe ausgewählt sind, die aus -NH-CO-R³, -SO₂-NR³R^{3'}, und -CO-NR³R^{3'} besteht;
E ausgewählt ist aus Aryl, Heteroaryl, (3-8-gliedrigem)-Cycloalkyl und -Heterocycloalkyl, worin Aryl, Heteroaryl, (3-8-gliedriges)-Cycloalkyl und -Heterocycloalkyl durch (C₂-C₁₈)Alkynyl substituiert sind;
R³, R^{3'} unabhängig voneinander ausgewählt sind aus H, (C₁-C₁₅)-Alkyl, (C₂-C₁₂)-Alkenyl, (C₂-C₁₂)-Alkynyl, Aryl, Heteroaryl, (3-8-gliedrigem)-Cycloalkyl oder - Heterocycloalkyl, (C₁-C₁₂)-Alkyl-aryl oder -heteroaryl, (C₂-C₁₂)-Alkenyl-aryl oder - heteroaryl, (C₂-C₁₂)-Alkynyl-aryl oder -heteroaryl;
für die Herstellung eines Medikaments zur Behandlung und/oder Vorbeugung kardiovaskulärer Erkrankungen.

2. Verwendung nach Anspruch 1, worin R^{2a} und R^{2b} jeweils H sind.

3. Verwendung nach Anspruch 1 oder 2, worin Cy D ist.

4. Verwendung nach einem der vorhergehenden Ansprüche, worin R^{3'} H ist und R³ ausgewählt ist aus der Gruppe bestehend aus Diphenyl-ethyl, Dodecyl, Octyl, 4-Pentyl-benzyl, 4-Phenoxy-phenethyl, Ethyl-thiophen-2-yl, Pentadecyl, Tridecyl, Hexyloxy-phenyl oder (2-Ethyl)-hexyl.

5. Verwendung nach Anspruch 1, worin Cy E ist.

6. Verwendung nach Anspruch 5, worin E Phenyl, Pyridinyl, eine Naphthyl- oder Benzofuranylgruppe ist, welche durch B-R⁴ substituiert ist, worin B eine Ethynylgruppe ist und R⁴ (C₆-C₁₆)Alkyl, (3-8-gliedriges) Cycloalkyl, (C₁-C₁₂)Alkyl-(3-8-gliedriges)cycloalkyl, Phenyl oder (C₁-C₁₂)Alkylphenyl ist.

7. Verwendung nach Anspruch 6, worin E Phenyl ist, welches durch B-R⁴ substituiert ist, worin B eine Ethynylgruppe und R⁴ (C₆-C₁₆)Alkyl ist.

8. Verwendung nach einem der vorhergehenden Ansprüche, worin R¹ ein -CH₂-A-, oder -CH₂-CH₂-A-Rest ist, worin A ein Aryl, Heteroaryl, (3-8-gliedriges)-Heterocycloalkyl oder (3-8-gliedriges)-Cycloalkyl ist.

9. Verwendung nach einem der Ansprüche 1 bis 7, worin R¹ A ist, worin A Aryl, Heteroaryl, (3-8-gliedriges)-Heterocycloalkyl oder (3-8-gliedriges)-Cycloalkyl ist.

10. Verwendung nach Anspruch 8 oder 9, worin A ausgewählt ist aus der Gruppe bestehend aus Phenyl, Pyridinyl, Benzo-1,3-dioxolenyl, Biphenyl, Naphthyl, Quinoxalinyl, Thiazolyl, Thienyl, Furanyl oder einer Piperidinylgruppe, welche gegebenenfalls substituiert ist durch 1 oder 2 Cyano, Halogen, NO₂, (C₁-C₆)-Alkoxy, Aryloxy oder Heteroaryloxy, (C₁-C₆)-Thioalkoxy, (C₁-C₁₂)-Alkyl, (C₁-C₁₂)-Alkyl-X, worin X Halogen ist, (C₂-C₁₂)-Alkenyl, (C₂-C₁₂)-Alkynyl, Aryl, Heteroaryl, (3-8-gliedriges)-Cykloalkyl oder -Heterocycloalkyl, (C₁-C₁₂)-Alkyl-aryl oder -heteroaryl, (C₂-C₁₂)-Alkenyl-aryl oder -heteroaryl, (C₂-C₁₂)-Alkynyl-aryl oder -heteroaryl, -COR³, -COOR³, -CO-NR³R^{3'}, -NHCOR³, worin R³ ein (C₁-C₁₂)-Alkyl oder (C₁-C₁₂)-Alkenyl, -SOR³, -SO₂R³, -SO₂NR³R^{3'}, ist, worin R³, R^{3'} unabhängig voneinander ausgewählt sind aus der Gruppe bestehend aus H, geradem oder verzweigtem (C₁-C₁₂)Alkyl, (C₂-C₁₂)Alkenyl, (C₂-C₁₂)Alkynyl, Aryl, Heteroaryl, (3-8-gliedriges)Cykloalkyl oder Heterocycloalkyl.

11. Verwendung nach einem der Ansprüche 1 bis 4 oder 8 bis 10, worin:
R^{2a} und R^{2b} jeweils H sind; R¹ -CH₂-A ist, worin A Phenyl oder Thienyl, gegebenenfalls substituiert ist durch Cyano, Halogen, Methoxy, Hydroxy, Phenoxy, -NO₂, Trifluoromethyl; Cy ein Thienyl, Phenyl und Biphenyl ist, welches durch -SO₂R³, -CO-NR³R^{3'} substituiert ist, worin R^{3'} H ist und R³ (C₇-C₁₂)Alkyl ist.

12. Verwendung nach einem der Ansprüche 1 bis 4 oder 8 bis 10, worin:
R^{2a} und R^{2b} jeweils H sind; R¹ -CH₂-A ist, worin A Phenyl oder Thienyl, gegebenenfalls substituiert ist durch Cyano, Halogen, Methoxy, Hydroxy, Phenoxy, -NO₂, Trifluoromethyl; Cy ein Thienyl, Phenyl und Biphenyl ist, welches durch -SO₂R³, -CO-NR³R^{3'} substituiert ist, worin R^{3'} H ist und R³ (C₇-C₁₅)Alkyl ist.

13. Verwendung nach einem der Ansprüche 1 bis 4 oder 8 bis 10, worin das Methylenamid-Derivat die Formel (I') aufweist: 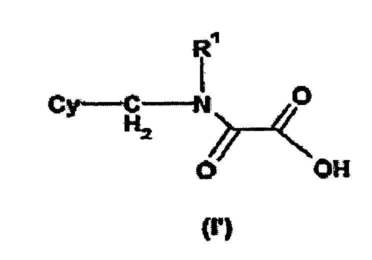 Worin
R¹ ausgewählt ist aus der Gruppe bestehend aus Phenyl, Benzyl, Phenethyl, 1-Methylbenzyl, welches durch eine (C₁-C₆)Alkylgruppe oder eine Cycloalkylgruppe substituiert sein kann; Cy eine Phenyl- oder eine Biphenylgruppe ist, welche mit einem Rest substituiert ist, der aus der Gruppe ausgewählt ist, die aus -NH-CO-R³, -CO-NH-R³, oder einer Oxadiazolgruppe, die mit R³ substituiert ist, worin R³ (C₇-C₁₅)Alkyl ist, besteht.

14. Verwendung nach Anspruch 13, worin R³ (C₈-C₁₅)Alkyl ist.

15. Verwendung nach Anspruch 13 und 14, worin R³ Dodecyl ist.

16. Verwendung nach einem der vorhergehenden Ansprüche, worin das Methylenamid-Derivat aus der folgenden Gruppe ausgewählt ist:
(benzyl {4-[(dodecylamino)carbonyl]benzyl}amino)(oxo)-Essigsäure;
oxo {{4-[(pentadecylamino)carbonyl]benzyl}[4-(trifluoromethyl)benzyl]amino}-Essigsäure;
(benzyl {4-[(pentadecylamino)carbonyl]benzyl} amino)(oxo)-Essigsäure;
(benzyl{4-[(tridecylamino)carbonyl]benzyl}amino)(oxo)-Essigsäure;
[benzyl(4-{[dodecyl(methyl)amino]carbonyl}benzyl)amino](oxo)-Essigsäure;
{(4-{[dodecyl(methyl)amino]carbonyl}benzyl)[4-(trifluoromethyl)benzyl]amino}-(oxo)-Essigsäure;
([1-(tert-butoxycarbonyl)-4-piperidinyl]{4-[(dodecylamino)carbonyl]benzyl}-amino)-(oxo)-Essigsäure;
{{4-[(dodecylamino)carbonyl]benzyl}[4-(trifluoromethyl)benzyl]amino}(oxo)-Essigsäure;
{{4-[(dodecylamino)carbonyl]benzyl}[3-(trifluoromethyl)benzyl]amino}(oxo)-Essigsäure;
({[1-(tert-butoxycarbonyl)-4-piperidinyl]methyl}{4-[(dodecylamino)carbonyl]-benzyl}amino)(oxo)-Essigsäure;
oxo{[4-(tridecanoylamino)benzyl][4-(trifluoromethyl)benzyl]amino}-Essigsäure;
[benzyl(4- {[4-(hexyloxy)benzoyl]amino}benzyl)amino](oxo)-Essigsäure;
oxo {[4-(trifluoromethyl)benzyl][4-(10-undecenoylamino)benzyl]amino}-Essigsäure;
oxo {{4-[(9E)-9-tetradecenoylamino]benzyl}[4-(trifluoromethyl)benzyl]amino}-Essigsäure;
{benzyl[4-(tridecanoylamino)benzyl]amino}(oxo)-Essigsäure;
{{4-[(2-hydroxydodecyl)amino]benzyl}[4-(trifluoromethyl)benzyl]amino}-(oxo)-Essigsäure;
oxo{[4-(trifluoromethyl)benzyl][4-(3-undecyl-1,2,4-oxadiazol-5-yl)benzyl]-amino}-Essigsäure;
{({5-[(dodecylamino)sulfonyl]-2-thienyl}methyl)[4-(trifluoromethyl)benzyl]amino}-(oxo)-Essigsäure;
[{4-[(dodecylamino)carbonyl]benzyl}({1-[(4-methoxyphenyl)sulfonyl]-4-piperidinyl}methyl)amino](oxo)-Essigsäure;
[{4-[(dodecylamino)carbonyl]benzyl}(2-carboxy-1-phenylethyl)amino](oxo)-Essigsäure;
[{4-[(dodecylamino)carbonyl]benzyl}(2-methoxy-1-methylethyl)amino](oxo)-Essigsäure;
(4-bromo {4-[(dodecylamino)carbonyl]benzyl}anilino)(oxo)-Essigsäure;
({4-[(dodecylamino)carbonyl]benzyl}anilino)(oxo)-Essigsäure;
([2-(3-chlorophenyl)ethyl]{4-[(dodecylamino)carbonyl]benzyl}amino)(oxo)-Essigsäure;
{{4-[(dodecylamino)carbonyl]benzyl}[2-(3-methoxyphenyl)ethyl]amino}(oxo)-Essigsäure;
{{4-[(dodecylamino)carbonyl]benzyl}[(d,1)-trans-2-phenylcyclopropyl]amino}-(oxo)-Essigsäure;
([(d,1)-trans-2-(benzyloxy)cyclopentyl]{4-[(dodecylamino)carbonyl]benzyl}-amino)-(oxo)-Essigsäure;
({4-[(dodecylamino)carbonyl]benzyl}-4-phenoxyanilino)(oxo)-Essigsäure;
[{4-[(dodecylamino)carbonyl]benzyl}(1,2,3,4-tetrahydro-1-naphthalenyl)amino]-(oxo)-Essigsäure;
((1-benzyl-4-piperidinyl){4-[(dodecylamino)carbonyl]benzyl}amino)(oxo)-Essigsäure;
{{4-[(dodecylamino)carbonyl]benzyl}[2-(4-phenoxyphenyl)ethyl]amino}(oxo)-Essigsäure;
{{4-[(dodecylamino)carbonyl]benzyl}[2-(2-phenoxyphenyl)ethyl]amino}(oxo)-Essigsäure;
((2-[1,1'-biphenyl]-4-ylethyl){4-[(dodecylamino)carbonyl]benzyl}amino)(oxo)-Essigsäure;
(([1,1'-biphenyl]-3-ylmethyl){4-[(dodecylamino)carbonyl]benzyl}amino)(oxo)-Essigsäure;
(3-(benzyloxy) {4-[(dodecylamino)carbonyl]benzyl}anilino)(oxo)-Essigsäure;
([4-(benzoylamino)benzyl]{4-[(dodecylamino)carbonyl]benzyl}amino)(oxo)-Essigsäure;
N-(carboxycarbonyl)-N- {4-[(dodecylamino)carbonyl]benzyl}-3-phenyl-beta-Alanin;
{{4-[(dodecylamino)carbonyl]benzyl}[4-(1,2,3-thiadiazol-4-yl)benzyl]amino}-(oxo)-Essigsäure;
[{4-[(dodecylamino)carbonyl]benzyl}(4-pentylbenzyl)amino](oxo)-Essigsäure;
[{4-[(dodecylamino)carbonyl]benzyl}(1-phenylethyl)amino](oxo)-Essigsäure;
{{4-[(dodecylamino)carbonyl]benzyl}[1-(1-naphthyl)ethyl]amino}(oxo)-Essigsäure;
(benzyl {3-[(dodecylamino)carbonyl]benzyl}amino)(oxo)-Essigsäure;
{{3-[(dodecylamino)carbonyl]benzyl}[4-(methylsulfonyl)benzyl] amino}(oxo)-Essigsäure;
((3-cyanobenzyl){3-[(dodecylamino)carbonyl]benzyl}amino)(oxo)-Essigsäure;
{{3-[(dodecylamino)carbonyl]benzyl}[4-(trifluoromethyl)benzyl]amino}(oxo)-Essigsäure;
[(4-chlorobenzyl)(3-{[(4-pentylbenzyl)amino]carbonyl}benzyl)amino](oxo)-Essigsäure;
oxo {[4-({[2-(2-thienyl)ethyl]amino}carbonyl)benzyl][4-(trifluoromethyl)-benzyl]amino}-Essigsäure;
{benzyl[(3'-{[(2,2-diphenylethyl)amino]carbonyl}[1,1'-biphenyl]-4-yl)methyl]-amino}(oxo)-Essigsäure;
{(3-cyanobenzyl)[(3'-{[(2,2-diphenylethyl)amino]carbonyl}[1,1'-biphenyl]-4-yl)methyl] amino}(oxo)-Essigsäure;
{(4-chlorobenzyl)[(3'-{[(2,2-diphenylethyl)amino]carbonyl}[1,1'-biphenyl]-4-yl)methyl] amino}(oxo)-Essigsäure;
{[(3'-{[(2,2-diphenylethyl)amino]carbonyl}[1,1'-biphenyl]-4-yl)methyl][4-(trifluoromethyl)benzyl] amino}(oxo)-Essigsäure;
((3-cyanobenzyl){[3'-({[2-(4-phenoxyphenyl)ethyl]amino}carbonyl)[1,1'-biphenyl]-4-yl]methyl} amino)(oxo)-Essigsäure;
oxo{{[3'-({[2-(4-phenoxyphenyl)ethyl]amino}carbonyl)[1,1'-biphenyl]-4-yl]methyl}-[4-(trifluoromethyl)benzyl]amino}-Essigsäure;
[(3-cyanobenzyl)({3'-[(octylamino)carbonyl][1,1'-biphenyl]-4-yl}methyl)amino]-(oxo)-Essigsäure;
[(4-chlorobenzyl)({3'-[(octylamino)carbonyl][1,1'-biphenyl]-4-yl}methyl)amino]-(oxo)-Essigsäure;
{({3'-[(octylamino)carbonyl][1,1'-biphenyl]-4-yl}methyl)[4-(trifluoromethyl)-benzyl] amino}(oxo)-Essigsäure;
{(3-cyanobenzyl)[(3'-{[(3-phenylpropyl)amino]carbonyl}[1,1'-biphenyl]-4-yl)methyl]amino}(oxo)-Essigsäure;
[(3-cyanobenzyl)({3'-[(dodecylamino)carbonyl][1,1'-biphenyl]-4-yl}methyl)-amino]-(oxo)-Essigsäure;
[(4-chlorobenzyl)({3'-[(dodecylamino)carbonyl][1,1'-biphenyl]-4-yl}methyl)-amino]-(oxo)-Essigsäure;
{({3'-[(dodecylamino)carbonyl][1,1'-biphenyl]-4-yl}methyl)[4-(trifluoromethyl)-benzyl] amino }(oxo)-Essigsäure;
{benzyl[(3'-{[(4-pentylbenzyl)amino]carbonyl}[1,1'-biphenyl]-4-yl)methyl]amino}-(oxo)-Essigsäure;
{(3-cyanobenzyl)[(3'- {[(4-pentylbenzyl)amino]carbonyl}[1,1'-biphenyl]-4-yl)-methyl]amino}(oxo)-Essigsäure;
{(4-chlorobenzyl)[(3'-{[(4-pentylbenzyl)amino]carbonyl}[1,1'-biphenyl]-4-yl)-methyl] amino}(oxo)-Essigsäure;
oxo {[(3'-{[(4-pentylbenzyl)amino]carbonyl}[1,1'-biphenyl]-4-yl)methyl][4-(trifluoromethyl)benzyl] amino}-Essigsäure;
oxo {[(3'- {[(4-phenylbutyl)amino]carbonyl}[1,1'-biphenyl]-4-yl)methyl][4-(trifluoromethyl)benzyl]-amino}Essigsäure;
{(3-cyanobenzyl)[(3'- {[(2-mesitylethyl)amino]carbonyl}[1,1'-biphenyl]-4-yl)-methyl]amino}(oxo)-Essigsäure;
{(4-chlorobenzyl)[(3'-{[(2-mesitylethyl)amino]carbonyl}[1,1'-biphenyl]-4-yl)-methyl]amino}(oxo)-Essigsäure;
{[(3'- {[(2-mesitylethyl)amino]carbonyl}[1,1'-biphenyl]-4-yl)methyl][4-(trifluoromethyl)benzyl]amino}(oxo)-Essigsäure;
((4-chlorobenzyl){[3'-({[2-(4-methoxyphenyl)ethyl]amino}carbonyl)[1,1'-biphenyl]-4-yl]methyl}amino)(oxo)-Essigsäure;
[{4-[(dodecylamino)carbonyl]benzyl}(4-methoxybenzyl)amino](oxo)-Essigsäure;
{{4-[(dodecylamino)carbonyl]benzyl}[4-(methylsulfonyl)benzyl]amino}(oxo)-Essigsäure;
[{3-[(dodecylamino)carbonyl]benzyl}(4-methoxybenzyl)amino](oxo)-Essigsäure;
{{3-[(dodecylamino)carbonyl]benzyl}[3-(trifluoromethyl)benzyl]amino}(oxo)-Essigsäure;
({4-[(dodecylamino)carbonyl]benzyl}{[6-(trifluoromethyl)-3-pyridinyl]methyl}-amino)(oxo)-Essigsäure;
4-[((carboxycarbonyl){3-[(dodecylamino)carbonyl]benzyl}amino)methyl]Benzoesäure;
({3-[(dodecylamino)carbonyl]benzyl}{4-[hydroxy(oxido)amino]benzyl}-amino)(oxo)-Essigsäure;
[{3-[(dodecylamino)carbonyl]benzyl}(2-fluorobenzyl)amino](oxo)-Essigsäure;
[{3-[(dodecylamino)carbonyl]benzyl}(2-pyridinylmethyl)amino](oxo)-Essigsäure;
[{3-[(dodecylamino)carbonyl]benzyl}(3-thienylmethyl)amino](oxo)-Essigsäure;
[{3-[(dodecylamino)carbonyl]benzyl}(4-hydroxybenzyl)amino](oxo)-Essigsäure;
[{3-[(dodecylamino)carbonyl]benzyl}(4-phenoxybenzyl)amino](oxo)-Essigsäure;
({3-[(dodecylamino)carbonyl]benzyl}{[6-(trifluoromethyl)-3-pyridinyl]methyl}-amino)(oxo)-Essigsäure;
3-[((carboxycarbonyl){3-[(dodecylamino)carbonyl]benzyl}amino)methyl]-Benzoesäure;
5-[((carboxycarbonyl){3-[(dodecylamino)carbonyl]benzyl}amino)methyl]-2-thiophen-Carbonsäure;
({4-[(dodecylamino)carbonyl]benzyl}{4-[hydroxy(oxido)amino]-benzyl}-amino)-(oxo)-Essigsäure;
((1,3-benzodioxol-5-ylmethyl){4-[(dodecylamino)carbonyl]-benzyl}amino)-(oxo)-Essigsäure;
[{4-[(dodecylamino)carbonyl]benzyl}(2-fluorobenzyl)amino](oxo)-Essigsäure;
[{4-[(dodecylamino)carbonyl]benzyl}(4-phenoxybenzyl)amino](oxo)-Essigsäure;
4-[((carboxycarbonyl){4-[(dodecylamino)carbonyl]-benzyl}amino)methyl]-Benzoesäure;
5-[((carboxycarbonyl){4-[(dodecylamino)carbonyl]benzyl}amino)methyl]-2-thiophen-Carbonsäure;
[{3-[(dodecylamino)carbonyl]benzyl}(2-thienylmethyl)amino](oxo)-Essigsäure;
[{4-[(dodecylamino)carbonyl]benzyl}(isopropyl)amino](oxo)-Essigsäure;
((3,5-dichlorobenzyl){4-[(dodecylamino)carbonyl]benzyl}amino)(oxo)-Essigsäure;
[(3,5-dichlorobenzyl)(4-{[(3,3-diphenylpropyl)amino]carbonyl}-benzyl)amino]-(oxo)-Essigsäure;
[(4- {[(2-[1,1'-biphenyl]-4-ylethyl)amino]carbonyl}benzyl)(3,5-dichlorobenzyl)-amino](oxo)-Essigsäure;
[(1,3-benzodioxol-5-ylmethyl)(4-{[(2-[1,1'-biphenyl]-4-ylethyl)amino]carbonyl}-benzyl)amino] (oxo)-Essigsäure;
(2,3-dihydro-1H-inden-1-yl{4-[(dodecylamino)carbonyl]benzyl}amino)(oxo)-Essigsäure;
{2,3-dihydro-1H-inden-1-yl[4-({[2-(4-phenoxyphenyl)ethyl]amino}-carbonyl)-benzyl]amino}(oxo)-Essigsäure;
[{4-[(dodecylamino)carbonyl]benzyl}(4-pyridinylmethyl)amino](oxo)-Essigsäure;
([4-(dimethylamino)benzyl]{4-[(dodecylamino)carbonyl]benzyl}amino)(oxo)-Essigsäure;
[{4-[(dodecylamino)carbonyl]benzyl}(3-pyridinylmethyl)amino](oxo)-Essigsäure;
((4-cyanobenzyl){4-[(dodecylamino)carbonyl]benzyl}amino)(oxo)-Essigsäure;
[{4-[(dodecylamino)carbonyl]benzyl}(1,3-thiazol-2-ylmethyl)amino](oxo)-Essigsäure;
({4-[(dodecylamino)carbonyl]benzyl} {[2-(4-morpholinyl)-1,3-thiazol-5-yl]methyl}-amino)(oxo)-Essigsäure;
[{3-[(dodecylamino)carbonyl]benzyl}(4-pyridinylmethyl)amino](oxo)-Essigsäure;
[{3-[(dodecylamino)carbonyl]benzyl}(3-pyridinylmethyl)amino](oxo)-Essigsäure;
[{3-[(dodecylamino)carbonyl]benzyl}(3-hydroxybenzyl)amino](oxo)-Essigsäure;
((4-cyanobenzyl){3-[(dodecylamino)carbonyl]benzyl}amino)(oxo)-Essigsäure;
[{3-[(dodecylamino)carbonyl]benzyl}(1,3-thiazol-2-ylmethyl)amino](oxo)-Essigsäure;
({3-[(dodecylamino)carbonyl]benzyl}{[2-(4-morpholinyl)-1,3-thiazol-5-yl]methyl}-amino)(oxo)-Essigsäure;
((1,3-benzodioxol-5-ylmethyl){3-[(dodecylamino)carbonyl]-benzyl}amino)-(oxo)-Essigsäure;
[{4-[(dodecylamino)carbonyl]benzyl}(2-thienylmethyl)amino](oxo)-Essigsäure;
[{4-[(dodecylamino)carbonyl]benzyl}(2-pyridinylmethyl)amino](oxo)-Essigsäure;
[{4-[(dodecylamino)carbonyl]benzyl}(3-thienylmethyl)amino](oxo)-Essigsäure;
[{4-[(dodecylamino)carbonyl]benzyl}(4-hydroxybenzyl)amino](oxo)-Essigsäure;
3-[((carboxycarbonyl) {4-[(dodecylamino)carbonyl]benzyl}amino)methyl]-Benzoesäure;
[cyclopentyl({5-[(dodecylamino)sulfonyl]-2-thienyl}methyl)amino](oxo)-Essigsäure;
[benzyl({5-[(dodecylamino)sulfonyl]-2-thienyl}methyl)amino](oxo)-Essigsäure;
(({5-[(dodecylamino)sulfonyl]-2-thienyl}methyl) {3-[hydroxy(oxido)amino]-benzyl}-amino)(oxo)-Essigsäure;
[({5-[(dodecylamino)sulfonyl]-2-thienyl}methyl)(4-methoxybenzyl)amino]-(oxo)-Essigsäure;
[({5-[(dodecylamino)sulfonyl]-2-thienyl}methyl)(2-fluorobenzyl)amino](oxo)-Essigsäure;
{({5-[(dodecylamino)sulfonyl]-2-thienyl}methyl)[4-(methylsulfonyl)-benzyl]-amino}(oxo)-Essigsäure;
[({5-[(dodecylamino)sulfonyl]-2-thienyl}methyl)(4-phenoxybenzyl)amino]-(oxo)-Essigsäure;
4-{[(carboxycarbonyl)({5-[(dodecylamino)sulfonyl]-2-thienyl}methyl)-amino]-methyl}-Benzoesäure;
(({5-[(dodecylamino)sulfonyl]-2-thienyl}methyl){[6-(trifluoromethyl)-3-pyridinyl]-methyl}amino)(oxo)-Essigsäure;
{({5-[(dodecylamino)sulfonyl]-2-thienyl}methyl)[3-(trifluoromethyl)benzyl]amino}-(oxo)-Essigsäure;
[(3-chlorobenzyl)({5-[(dodecylamino)sulfonyl]-2-thienyl}methyl)amino](oxo)-Essigsäure;
{[(5-{[(3,3-diphenylpropyl)amino]sulfonyl}-2-thienyl)methyl] [3-(trifluoromethyl)-benzyl]amino}(oxo)-Essigsäure;
{(3-chlorobenzyl)[(5-{[(3,3-diphenylpropyl)amino]sulfonyl}-2-thienyl)methyl]-amino}(oxo)-Essigsäure;
oxo {{[5-({[2-(4-phenoxyphenyl)ethyl]amino}sulfonyl)-2-thienyl]methyl}[3-(trifluoromethyl)benzyl]amino}-Essigsäure;
((3-chlorobenzyl) {[5-({[2-(4-phenoxyphenyl)ethyl]amino}sulfonyl)-2-thienyl]-methyl}amino)(oxo)-Essigsäure;
{[(5-{[(2-[1,1'-biphenyl]-4-ylethyl)amino]sulfonyl}-2-thienyl)methyl][3-(trifluoromethyl)benzyl]amino}(oxo)-Essigsäure;
(({1-[(cyclohexylamino)carbonyl]-4-piperidinyl}methyl) {4-[(dodecylamino)-carbonyl]benzyl}amino)(oxo)-Essigsäure;
([(1-{[4-(dimethylamino)anilino]carbonyl}-4-piperidinyl)methyl]{4-[(dodecyl-amino)carbonyl]benzyl}amino)(oxo)-Essigsäure;
{{4-[(dodecylamino)carbonyl]benzyl}[(1-hexanoyl-4-piperidinyl)methyl]-amino}-(oxo)-Essigsäure;
({4-[(dodecylamino)carbonyl]benzyl}{[1-(3-iodobenzoyl)-4-piperidinyl]methyl}-amino)(oxo)-Essigsäure;
{{4-[(dodecylamino)carbonyl]benzyl}[(1-{(2E)-3-(3-(trifluoromethyl)phenyl]-2-propenoyl}-4-piperidinyl)methyl] amino }(oxo)-Essigsäure;
({4-[(dodecylamino)carbonyl]benzyl}{(1-(2-quinoxalinylcarbonyl)-4-piperidinyl]-methyl}amino)(oxo)-Essigsäure;
[({1-[(4-methoxyphenyl)sulfonyl]-4-piperidinyl}methyl)(4- {[(4-phenoxybenzyl)amino]carbonyl}benzyl)amino](oxo)-Essigsäure;
[{[1-(3-iodobenzoyl)-4-piperidinyl]methyl}(4-{[(4-phenoxybenzyl)amino]-carbonyl}benzyl)amino](oxo)-Essigsäure;
oxo {(4- {[(4-phenoxybenzyl)amino]carbonyl}benzyl)[(1-{(2E)-3-[3-(trifluoromethyl)phenyl]-2-propenoyl}-4-piperidinyl)methyl]amino}-Essigsäure;
{{4-[(dodecylamino)carbonyl]phenyl}[2-(methoxycarbonyl)benzyl]-amino}(oxo)-Essigsäure; [[4-({[2-(1,1'-biphenyl-4-yl)ethyl]amino}carbonyl)-2-bromobenzyl](4-iodobenzyl)-amino](oxo)-Essigsäure;
[(2-bromo-4-{[(4-pentylbenzyl)amino]carbonyl}benzyl)(4-iodobenzyl)amino]-(oxo)-Essigsäure;
[{2-bromo-4-[(dodecylamino)carbonyl]benzyl}(4-iodobenzyl)amino](oxo)-Essigsäure;
[(2,6-dibromo-4-{[(4-pentylbenzyl)amino]carbonyl}benzyl)(4-iodobenzyl)amino]-(oxo)-Essigsäure;
((4-iodobenzyl){[4'-({[2-(4-phenoxyphenyl)ethyl]amino}carbonyl)-1,1'-biphenyl-4-yl]methyl}amino)(oxo)-Essigsäure;
{[2-bromo-4-({[2-(4-phenoxyphenyl)ethyl]amino}carbonyl)benzyl][(4'-fluoro-1,1'-biphenyl-3-yl)methyl]amino}(oxo)-Essigsäure;
{[4-({[2-(1,1'-biphenyl-4-yl)ethyl]amino}carbonyl)-2-bromobenzyl][(4'-fluoro-1,1'-biphenyl-3-yl)methyl]amino}(oxo)-Essigsäure;
{(2-bromo-4-{[(4-pentylbenzyl)amino]carbonyl}benzyl)[(4'-fluoro-1,1'-biphenyl-3-yl)methyl]amino}(oxo)-Essigsäure;
{[2,6-dibromo-4-({[2-(4-phenoxyphenyl)ethyl]amino}carbonyl)benzyl][(4'-fluoro-1,1'-biphenyl-3-yl)methyl] amino}(oxo)-Essigsäure;
{[4-({[2-(1,1'-biphenyl-4-yl)ethyl]amino}carbonyl)-2,6-dibromobenzyl][(4'-fluoro-1,1'-biphenyl-3-yl)methyl]aminol}(oxo)-Essigsäure;
{(2,6-dibromo-4- {[(4-pentylbenzyl)amino]carbonyl}benzyl)[(4'-fluoro-1,1'-biphenyl-3-yl)methyl] amino}(oxo)-Essigsäure;
{{2,6-dibromo-4-[(dodecylamino)carbonyl]benzyl}[(4'-fluoro-1,1'-biphenyl-3-yl)methyl]amino}(oxo)-Essigsäure;
([(4'-fluoro-1,1'-biphenyl-3-yl)methyl]{[4'-({[2-(4-phenoxyphenyl)ethyl]amino}-carbonyl)-1,1'-biphenyl-4-yl}methyl}amino)(oxo)-Essigsäure;
{({4'-[(dodecylamino)carbonyl]-1,1'-biphenyl-4-yl}methyl)[(4'-fluoro-1,1'-biphenyl-3-yl)methyl]amino}(oxo)-Essigsäure;
{(2-bromo-4-{[(4-pentylbenzyl)amino]carbonyl}benzyl)[2-(trifluoromethoxy)-benzyl] amino} (oxo)-Essigsäure;
{(2,6-dibromo-4- {[(4-pentylbenzyl)amino]carbonyl}benzyl)[2-(trifluoromethoxy)-benzyl]amino}(oxo)-Essigsäure;
oxo {{[4'-({[2-(4-phenoxyphenyl)ethyl]amino}carbonyl)-1,1'-biphenyl-4-yl]methyl}-[2-(trifluoromethoxy)benzyl]amino}-Essigsäure;
{({4'-[(dodecylamino)carbonyl]-1,1'-biphenyl-4-yl}methyl)[2-(trifluoromethoxy)-benzyl]amino}(oxo)-Essigsäure;
[[2-bromo-4-({[2-(4-phenoxyphenyl)ethyl]amino}carbonyl)benzyl](3-phenoxy-benzyl)amino](oxo)-Essigsäure;
[[4-({[2-(1,1'-biphenyl-4-yl)ethyl]amino}carbonyl)-2-bromobenzyl](3-phenoxybenzyl)amino](oxo)-Essigsäure;
[(2-bromo-4-{[(4-pentylbenzyl)amino]carbonyl}benzyl)(3-phenoxybenzyl)-amino](oxo)-Essigsäure;
[[2,6-dibromo-4-({[2-(4-phenoxyphenyl)ethyl]amino}carbonyl)benzyl](3-phenoxybenzyl)amino](oxo)-Essigsäure;
[[4-({[2-(1,1'-biphenyl-4-yl)ethyl}amino}carbonyl)-2,6-dibromobenzyl](3-phenoxy-benzyl)amino](oxo)-Essigsäure;
[(2,6-dibromo-4- {[(4-pentylbenzyl)amino]carbonyl}benzyl)(3-phenoxybenzyl)-amino](oxo)-Essigsäure;
[{2,6-dibromo-4-[(dodecylamino)carbonyl]benzyl}(3-phenoxybenzyl)amino](oxo)-Essigsäure;
oxo((3-phenoxybenzyl){[4'-({[2-(4-phenoxyphenyl)ethyl]amino}carbonyl)-1,1'-biphenyl-4-yl]methyl}amino)-Essigsäure;
oxo[[(4'-{[(4-pentylbenzyl)amino]carbonyl}-1,1'-biphenyl-4-yl)methyl](3-phenoxybenzyl)amino]-Essigsäure;
[({4'-[(dodecylamino)carbonyl]-1,1'-biphenyl-4-yl}methyl)(3-phenoxybenzyl)-amino](oxo)-Essigsäure;
[[2-bromo-4-({[2-(4-phenoxyphenyl)ethyl]amino}carbonyl)benzyl](2-iodobenzyl)-amino](oxo)-Essigsäure;
[[4-({[2-(1,1'-biphenyl-4-yl)ethyl]amino}carbonyl)-2-bromobenzyl](2-iodobenzyl)-amino] (oxo)-Essigsäure;
[(2-bromo-4-{[(4-pentylbenzyl)amino]carbonyl}benzyl)(2-iodobenzyl)amino]-(oxo)-Essigsäure;
[{2-bromo-4-[(dodecylamino)carbonyl]benzyl}(2-iodobenzyl)amino](oxo)-Essigsäure
([2-bromo-4-({[2-(4-phenoxyphenyl)ethyl]amino}carbonyl)benzyl]{[2'-(trifluoro-methyl)-1,1'-biphenyl-4-yl]methyl}amino)(oxo)-Essigsäure;
([4-({[2-(1,1'-biphenyl-4-yl)ethyl]amino}carbonyl)-2-bromobenzyl]{[2'-(trifluoro-methyl)-1,1'-biphenyl-4-yl]methyl}amino)(oxo)-Essigsäure;
((2-bromo-4-{[(4-pentylbenzyl)amino]carbonyl}benzyl){[2'-(trifluoromethyl)-1,1'-biphenyl-4-yl]methyl}amino)(oxo)-Essigsäure;
((2-bromo-4-{[(4-pentylbenzyl)amino]carbonyl}benzyl){[2'-(trifluoromethyl)-1,1'-biphenyl-4-yl]methyl}amino)(oxo)-Essigsäure;
({2-bromo-4-[(dodecylamino)carbonyl]benzyl}{[2'-(trifluoromethyl)-1,1'-biphenyl-4-yl]methyl} amino)(oxo)-Essigsäure;
([4-({[2-(1,1'-biphenyl-4-yl)ethyl]amino}carbonyl)-2,6-dibromobenzyl]{[2'-(trifluoromethyl)-1,1'-biphenyl-4-yl]methyl}amino)(oxo)-Essigsäure;
((2,6-dibromo-4- {[(4-pentylbenzyl)amino]carbonyl}benzyl){[2'-(trifluoromethyl)-1,1'-biphenyl-4-yl]methyl}amino)(oxo)-Essigsäure;
({2,6-dibromo-4-[(dodecylamino)carbonyl]benzyl}{[2'-(trifluoromethyl)-1,1'-biphenyl-4-yl]methyl}amino)(oxo)-Essigsäure;
(({4'-[(dodecylamino)carbonyl]-1,1'-biphenyl-4-yl}methyl){[2'-(trifluoromethyl)-1,1'-biphenyl-4-yl]methyl}amino)(oxo)-Essigsäure;
[[4-({[2-(1,1'-biphenyl-4-yl)ethyl]amino}carbonyl)-2-bromobenzyl](1,1'-biphenyl-2-ylmethyl)amino](oxo)-Essigsäure;
[(1,1'-biphenyl-2-ylmethyl)(2-bromo-4-{[(4-pentylbenzyl)amino]carbonyl}benzyl)-amino](oxo)-Essigsäure;
((1,1'-biphenyl-2-ylmethyl){2-bromo-4-[(dodecylamino)carbonyl]benzyl}-amino)-(oxo)-Essigsäure;
((1,1'-biphenyl-2-ylmethyl)[2,6-dibromo-4-({[2-(4-phenoxyphenyl)ethyl]amino}-carbonyl)benzyl]amino}(oxo)-Essigsäure;
[[4-({[2-(1,1'-biphenyl-4-yl)ethyl]amino}carbonyl)-2,6-dibromobenzyl](1,1'-biphenyl-2-ylmethyl)amino](oxo)-Essigsäure;
[(1,1'-biphenyl-2-ylmethyl)(2,6-dibromo-4-{[(4-pentylbenzyl)amino]carbonyl}-benzyl)amino](oxo)-Essigsäure;
((1,1'-biphenyl-2-ylmethyl){2,6-dibromo-4-[(dodecylamino)carbonyl]benzyl}-amino)(oxo)-Essigsäure;
{(2-bromo-4-{[(4-pentylbenzyl)amino]carbonyl}benzyl)[4-(trifluoromethoxy)-benzyl]amino}(oxo)-Essigsäure;
{{2-bromo-4-[(dodecylamino)carbonyl]benzyl}[4-(trifluoromethoxy)benzyl]amino}-(oxo)-Essigsäure;
{(2,6-dibromo-4-{[(4-pentylbenzyl)amino]carbonyl}benzyl)[4-(trifluoromethoxy)-benzyl]amino}(oxo)-Essigsäure;
{(2-bromo-4-{[(4-pentylbenzyl)amino]carbonyl}benzyl)[3-(trifluoromethoxy)-benzyl]amino}(oxo)-Essigsäure;
{{2-bromo-4-[(dodecylamino)carbonyl]benzyl}[3-(trifluoromethoxy)benzyl]amino}-(oxo)-Essigsäure;
{(2,6-dibromo-4- {[(4-pentylbenzyl)amino]carbonyl}benzyl)[3-(trifluoromethoxy)-benzyl]amino}(oxo)-Essigsäure;
{{2,6-dibromo-4-[(dodecylamino)carbonyl]benzyl}[3-(trifluoromethoxy)benzyl]-amino}(oxo)-Essigsäure;
{({4'-[(dodecylamino)carbonyl]-1,1'-biphenyl-4-yl}methyl)[3-(trifluoromethoxy)-benzyl]amino}(oxo)-Essigsäure;
[[2-bromo-4-({[2-(4-phenoxyphenyl)ethyl]amino}carbonyl)benzyl](4-phenoxy-benzyl)amino](oxo)-Essigsäure;
[[4-({[2-(1,1'-biphenyl-4-yl)ethyl]amino}carbonyl)-2-bromobenzyl](4-phenoxy-benzyl)amino](oxo)-Essigsäure;
[(2-bromo-4-{[(4-pentylbenzyl)amino]carbonyl}benzyl)(4-phenoxybenzyl)-amino](oxo)-Essigsäure;
[{2-bromo-4-[(dodecylamino)carbonyl]benzyl}(4-phenoxybenzyl)amino](oxo)-Essigsäure;
[[4-({[2-(1,1'-biphenyl-4-yl)ethyl]amino}carbonyl)-2,6-dibromobenzyl](4-phenoxy-benzyl)amino](oxo)-Essigsäure;
[(2,6-dibromo-4-{[(4-pentylbenzyl)amino]carbonyl}benzyl)(4-phenoxybenzyl)-amino](oxo)-Essigsäure;
{[4-({[2-(1,1'-biphenyl-4-yl)ethyl]amino}carbonyl)-2-bromobenzyl][4-(trifluoromethyl)benzyl]amino}(oxo)-Essigsäure;
{(2-bromo-4-{[(4-pentylbenzyl)amino]carbonyl}benzyl)[4-(trifluoromethyl)-benzyl]-amino}(oxo)-Essigsäure;
{{2-bromo-4-[(dodecylamino)carbonyl]benzyl}[4-(trifluoromethyl)benzyl]amino}-(oxo)-Essigsäure;
{(2,6-dibromo-4-{[(4-pentylbenzyl)amino]carbonyl}benzyl)[4-(trifluoromethyl)-benzyl]amino}(oxo)-Essigsäure;
{{2,6-dibromo-4-[(dodecylamino)carbonyl]benzyl}[4-(trifluoromethyl)benzyl]-amino}(oxo)-Essigsäure;
oxo {[(4'-{[(4-pentylbenzyl)amino]carbonyl}-1,1'-biphenyl-4-yl]methyl][4-(trifluoromethyl)benzyl]amino}-Essigsäure;
{{2-bromo-4-[(dodecylamino)carbonyl]benzyl}[3-(trifluoromethyl)benzyl]-amino}(oxo)-Essigsäure;
{{2,6-dibromo-4-[(dodecylamino)carbonyl]benzyl}[3-(trifluoromethyl)benzyl]-amino}(oxo)-Essigsäure;
oxo {[(4'-{[(4-pentylbenzyl)amino]carbonyl}-1,1'-biphenyl-4-yl)methyl][3-(trifluoromethyl)benzyl]amino}-Essigsäure;
{(4-dibenzo[b,d]furan-4-ylbenzyl)[4-(trifluoromethyl)benzyl]amino}(oxo)-Essigsäure;
{(4-dibenzo[b,d]furan-4-ylbenzyl)[4-(trifluoromethyl)benzyl]amino}(oxo)-Essigsäure, N-methyl-D-glucamin(d.h.1-deoxy-1-(methylamino)glucitol)-Salz;
({4-[(dodecylamino)carbonyl)benzyl}{1-[4-(trifluoromethyl)phenyl]ethyl}amino)-(oxo)-Essigsäure;
({4-[(dodecylamino)carbonyl]benzyl}{1-[4-(trifluoromethyl)phenyl]ethyl}amino)-(oxo)-Essigsäure, N-methyl-D-glucamin(d.h.1-deoxy-1-(methylamino)glucitol)-Salz;
{({4'-[(octylamino)carbonyl]-1,1'-biphenyl-4-yl}methyl}[4-(trifluoromethyl)benzyl]-amino}(oxo)-Essigsäure;
oxo {(4-tetradec-1-ynylbenzyl)[4-(trifluoromethyl)benzyl]amino}-Essigsäure;
{(4-dodec-1-ynylbenzyl)[4-(trifluoromethyl)benzyl]amino}(oxo)-Essigsäure;
{{4-[(dodecylamino)carbonyl]benzyl}[4-(trifluoromethyl)phenyl]amino}(oxo)-Essigsäure;
[{4-[(dodecylamino)carbonyl]benzyl}(2-methoxyphenyl)amino](oxo)-Essigsäure;
((1,2-diphenylethyl){4-[(dodecylamino)carbonyl]benzyl}amino)(oxo)-Essigsäure;
N-(carboxycarbonyl)-N- {4-[(dodecylamino)carbonyl]benzyl}-L-Phenylalanin;
[{4-[(dodecylamino)carbonyl]benzyl}(3-phenoxyphenyl)amino](oxo)-Essigsäure;
[{4-[(dodecylamino)carbonyl]benzyl}(2-isopropoxyphenyl)amino](oxo)-Essigsäure;
[{4-[(dodecylamino)carbonyl]benzyl}(4-iodophenyl)amino](oxo)-Essigsäure;
{{4-[(dodecylamino)carbonyl]benzyl}[3-fluoro-4-(trifluoromethyl)benzyl]-amino}(oxo)-Essigsäure;
((3-chloro-2-methylphenyl){4-[(dodecylamino)carbonyl]benzyl}amino)(oxo)-Essigsäure;
4'-((carboxycarbonyl){4-[(dodecylamino)carbonyl]benzyl}amino)-1,1'-biphenyl-2-Carbonsäure;
((2,4-dichlorobenzyl){4-[(dodecylamino)carbonyl]benzyl}amino)(oxo)-Essigsäure;
[{4-[(dodecylamino)carbonyl]benzyl}(1-phenylpropyl)amino](oxo)-Essigsäure;
([2-(4-chlorophenyl)propyl] {4-[(dodecylamino)carbonyl]benzyl}amino)(oxo)-Essigsäure;
[{4-[(dodecylamino)carbonyl]benzyl}(4-isopropoxyphenyl)amino](oxo)-Essigsäure;
([4-(benzyloxy)phenyl]{4-[(dodecylamino)carbonyl]benzyl}amino)(oxo)-Essigsäure;
{{4-[(dodecylamino)carbonyl]benzyl}[2-(trifluoromethyl)benzyl]amino}(oxo)-Essigsäure;
[{4-[(dodecylamino)carbonyl]benzyl}(2-methoxybenzyl)amino](oxo)-Essigsäure;
([(1R)-1-(4-chlorophenyl)ethyl]{4-[(dodecylamino)carbonyl]benzyl}amino)-(oxo)-Essigsäure;
((3,4-dichlorobenzyl){4-[(dodecylamino)carbonyl]benzyl}amino)(oxo)-Essigsäure
((1-benzothien-3-ylmethyl){4-[(dodecylamino)carbonyl]benzyl}ammo)(oxo)-Essigsäure;
([2-(2,6-dichlorophenyl)ethyl] {4-[(dodecylamino)carbonyl]benzyl}amino)(oxo)-Essigsäure;
({4-[(dodecylamino)carbonyl]benzyl}{2-[3-(trifluoromethyl)phenyl]ethyl}-amino)-(oxo)-Essigsäure;
{{4-[(dodecylamino)carbonyl]benzyl}[2-(3-fluorophenyl)ethyl]amino}(oxo)-Essigsäure;
([(1S)-1-(4-chlorophenyl)ethyl]{4-[(dodecylamino)carbonyl]benzyl}amino)(oxo)-Essigsäure;
{{4-[(dodecylamino)carbonyl]benzyl}[(1S)-1-phenylethyl]amino}(oxo)-Essigsäure;
{{4-[(dodecylamino)carbonyl]benzyl}[(1R)-1-phenylethyl]amino}(oxo)-Essigsäure;
([3-(benzyloxy)phenyl] {4-[(dodecylamino)carbonyl]benzyl}amino)(oxo)-Essigsäure;
N-(carboxycarbonyl)-N- {4-[(dodecylamino)carbonyl]benzyl}-D-Phenylalanin;
{{4-[(dodecylamino)carbonyl]phenyl}[4-(trifluoromethyl)benzyl]amino}(oxo)-Essigsäure;
{{4-[(dodecylamino)carbonyl]phenyl}[4-(trifluoromethyl)benzyl]amino}(oxo)-Essigsäure, N-methyl-D-glucamin(d.h.1-deoxy-1-(methylamino)glucitol)-Salz;
oxo {{1-[4-(trifluoromethyl)phenyl]ethyl}[4-(3-undecyl-1,2,4-oxadiazol-5-yl)benzyl]amino}-Essigsäure;
oxo {{1-[4-(trifluoromethyl)phenyl]ethyl}[4-(3-undecyl-1,2,4-oxadiazol-5-yl)benzyl]amino}-Essigsäure, N-methyl-D-glucamin(d.h.1-deoxy-1-(methylamino)glucitol)-Salz;
([(2-butyl-1-benzofuran-3-yl)methyl]{4-[(dodecylamino)carbonyl]benzyl}-amino)-(oxo)-Essigsäure;
{(1-{4-[(dodecylamino)carbonyl]phenyl}ethyl)[4-(trifluoromethyl)benzyl]amino)-(oxo)-Essigsäure;
{(1-{4-[(dodecylamino)carbonyl]phenyl}ethyl)[4-(trifluoromethyl)benzyl]amino}(oxo)-Essigsäure, N-methyl-D-glucamin(d.h.1-deoxy-1-(methylamino)glucitol)-Salz;
{(4-{[(4-octylphenyl)amino]carbonyl}benzyl)[4-(trifluoromethyl)benzyl]-amino}(oxo)-Essigsäure;
{(3-chlorobenzyl)[4-(3-undecyl-1,2,4-oxadiazol-5-yl)benzyl]amino}(oxo)-Essigsäure;
{(3-chlorobenzyl)[4-(3-undecyl-1,2,4-oxadiazol-5-yl)benzyl]amino)(oxo)-Essigsäure, N-methyl-D-glucamin(d.h.1-deoxy-1-(methylamino)glucitol)-Salz;
{{cyclopentyl[4-(trifluoromethyl)phenyl]methyl}[4-(tridecanoylamino)benzyl]-amino}(oxo)-Essigsäure;
oxo([4-(trifluoromethyl)benzyl]{[4-(3-undecyl-1,2,4-oxadiazol-5-yl)-1-naphthyl]-methyl}amino)-Essigsäure;
oxo([4-(trifluoromethyl)benzyl]{[4-(3-undecyl-1,2,4-oxadiazol-5-yl)-1-naphthyl]-methyl}amino)-Essigsäure, N-methyl-D-glucamin(d.h.1-deoxy-1-(methylamino)-glucitol)-Salz;
{{cyclopentyl[4-(trifluoromethyl)phenyl]methyl}[4-(3-undecyl-1,2,4-oxadiazol-5-yl)benzyl] amino}(oxo)-Essigsäure;
{{cyclopentyl[4-(trifluoromethyl)phenyl]methyl}[4-(3-undecyl-1,2,4-oxadiazol-5-yl)benzyl]amino}(oxo)-Essigsäure, N-methyl-D-glucamin(d.h. 1-deoxy-1-(methyl-amino)glucitol)-Salz;
{(4-dibenzo[b,d]furan-4-ylphenyl)[4-(trifluoromethyl)benzyl]amino}(oxo)-Essigsäure;
{(4-dibenzo[b,d]furan-4-ylphenyl)[4-(trifluoromethyl)benzyl]amino}(oxo)-Essigsäure, N-methyl-D-glucwnin(d.h.1-deoxy-1-(methylamino)glucitol)-Salz;
{[4-(octyloxy)benzyl][4-(trifluoromethyl)benzyl]amino}(oxo)-Essigsäure;
{[4-(octyloxy)benzyl][4-(trifluoromethyl)benzyl]amino}(oxo)-Essigsäure, N-methyl-D-glucamin(d.h.1-deoxy-1-(methylamino)glucitol)-Salz;
[[2-(3-chlorophenyl)ethyl](4-dec-1-ynylbenzyl)amino](oxo)-Essigsäure;
([2-(3-chlorophenyl)ethyl] {4-[(1Z)-dec-1-enyl]benzyl}amino)(oxo)-Essigsäure;
{[2-(3-chlorophenyl)ethyl][4-(3-undecyl-1,2,4-oxadiazol-5-yl)benzyl]amino}(oxo)-Essigsäure;
{[2-(3-chlorophenyl)ethyl][4-(3-undecyl-1,2,4-oxadiazol-5-yl)benzyl]amino}(oxo)-Essigsäure, N-methyl-D-glucamin(d.h.l-deoxy-1-(methylamino)glucitol)-Salz;
oxo{{(1R)-1-[4-(trifluoromethyl)phenyl]ethyl}[4-(3-undecyl-1,2,4-oxadiazol-5-yl)benzyl]amino}-Essigsäure;
oxo{{(1R)-1-[4-(trifluoromethyl)phenyl]ethyl}[4-(3-undecyl-1,2,4-oxadiazol-5-yl)benzyl]amino}-Essigsäure, N-methyl-D-glucamin(d.h. 1-deoxy-1-(methylamino)-glucitol)-Salz;
oxo{[4-(trifluoromethyl)phenyl][4-(3-undecyl-1,2,4-oxadiazol-5-yl)benzyl]amino}-Essigsäure;
oxo{[4-(trifluoromethyl)phenyl][4-(3-undecyl-1,2,4-oxadiazol-5-yl)benzyl]amino}-Essigsäure, N-methyl-D-glucamin(d.h.1-deoxy-1-(methylamino)glucitol)-Salz;
oxo {{(1S)-1-[4-(trifluoromethyl)phenyl]ethyl)}[4-(3-undecyl-1,2,4-oxadiazol-5-yl)benzyl]amino}-Essigsäure;
oxo {{(1S)-1-[4-(trifluoromethyl)phenyl]ethyl}[4-(3-undecyl-1,2,4-oxadiazol-5-yl)benzyl]amino}-Essigsäure, N-methyl-D-glucamin(d.h.1-deoxy-1-(methylamino)-glucitol)-Salz;
[(3-chlorobenzyl)(4-dec-1-ynylbenzyl)amino](oxo)-Essigsäure;
[(3-chlorobenzyl)(4-dec-1-ynylbenzyl)amino](oxo)-Essigsäure, N-methyl-D-glucamin(d.h. 1-deoxy-1-(methylamino)glucitol)-Salz;
[[2-(3-chlorophenyl)ethyl](4-oct-1-ynylbenzyl)amino](oxo)-Essigsäure;
[[2-(3-chlorophenyl)ethyl](4-oct-1-ynylbenzyl)amino](oxo)-Essigsäure, N-methyl-D-glucamin(d.h.1-deoxy-1-(methylamino)glucitol)-Salz;
{(4-dec-1-ynylbenzyl)[4-(trifluoromethyl)phenyl]amino}(oxo)-Essigsäure;
((4-dec-1-ynylbenzyl){1-[4-(trifluoromethyl)phenyl]ethyl}amino)(oxo)-Essigsäure;
((4-dec-1-ynylbenzyl){1-[4-(trifluoromethyl)phenyl]ethyl}amino)(oxo)-Essigsäure, N-methyl-D-glucamin(d.h.1-deoxy-1-(methylamino)glucitol)-Salz;
{{1-methyl-1-[4-(trifluoromethyl)phenyl]ethyl}[4-(3-undecyl-1,2,4-oxadiazol-5-yl)benzyl]amino}(oxo)-Essigsäure;
{{1-methyl-1-[4-(trifluoromethyl)phenyl]ethyl}[4-(3-undecyl-1,2,4-oxadiazol-5-yl)benzyl]amino}(oxo)-Essigsäure, N-methyl-D-glucamin(d.h.1-deoxy-1-(methylamino)glucitol)-Salz;
{[2-(3-chlorophenyl)ethyl] [4-(3-octyl-1,2,4-oxadiazol-5-yl)benzyl]amino}(oxo)-Essigsäure;
{[2-(3-chlorophenyl)ethyl][4-(3-octyl-1,2,4-oxadiazol-5-yl)benzyl]amino}(oxo)-Essigsäure, N-methyl-D-glucamin(d.h.1-deoxy-1-(methylamino)glucitol)-Salz;
{[4-(3-octyl-1,2,4-oxadiazol-5-yl)benzyl][4-(trifluoromethyl)benzyl]amino}-(oxo)-Essigsäure;
{[4-(3-octyl-1,2,4-oxadiazol-5-yl)benzyl][4-(trifluoromethyl)benzyl]amino}-(oxo)-Essigsäure, N-methyl-D-glucamin(d.h. 1-deoxy-1-(methylamino)glucitol)-Salz;
{{[4-(dodecyloxy)-1-naphthyl]methyl}[4-(trifluoromethyl)benzyl]amino}(oxo)-Essigsäure;
{{[4-(dodecyloxy)-1-naphthyl]methyl}[4-(trifluoromethyl)benzyl]amino}(oxo)-Essigsäure, N-methyl-D-glucamin(d.h.1-deoxy-1-(methylamino)glucitol)-Salz
[(4-bromobenzyl)(4-oct-1-ynylbenzyl)amino](oxo)-Essigsäure;
[{4-[(dodecylamino)carbonyl]benzyl}(2-hydroxy-1-phenylethyl)amino](oxo)-Essigsäure;
((4-dec-1-ynylbenzyl){1-methyl-1-[4-(trifluoromethyl)phenyl]ethyl}amino)(oxo)-Essigsäure;
((4-dec-1-ynylbenzyl){1-methyl-1-[4-(trifluoromethyl)phenyl]ethyl}amino)(oxo)-Essigsäure,
N-methyl-D-glucamin(d.h.1-deoxy-1-(methylamino)glucitol)-Salz;
oxo{{4-[(9Z)-tetradec-9-enoylamino]benzyl}[4-(trifluoromethyl)benzyl]amino}-Essigsäure;
{[4-dec-1-ynylbenzyl)[4-(trifluoromethyl)benzyl]amino}(oxo)-Essigsäure;
oxo {[4-(trifluoromethyl)benzyl] [3-(3-undecyl-1,2,4-oxadiazol-5-yl)benzyl]-amino}-Essigsäure;
oxo {[4-(trifluoromethyl)benzyl][3-(3-undecyl-1,2,4-oxadiazol-5-yl)benzyl]amino}-Essigsäure, N-methyl-D-glucamin(d.h.1-deoxy-1-(methylamino)glucitol)-Salz;
{(4-dodecylbenzyl)[4-(trifluoromethyl)benzyl]amino}(oxo)-Essigsäure;
{(4-dodecylbenzyl)[4-(trifluoromethyl)benzyl]amino}(oxo)-Essigsäure, N-methyl-D-glucamin(d.h.1-deoxy-1-(methylamino)glucitol)-Salz;
{[4-({[(2-butyl-1-benzofuran-3-yl)methyl]amino}carbonyl)benzyl][4-(trifluoromethyl)benzyl]amino}(oxo)-Essigsäure;
{(4-{[4-(benzyloxy)benzoyl]amino}benzyl)[4-(trifluoromethyl)benzyl]amino}-(oxo)-Essigsäure;
{(3,5-dichlorobenzyl)[4-(tridecanoylamino)benzyl]amino}(oxo)-Essigsäure;
{(3,5-dichlorobenzyl)[4-(tridecanoylamino)benzyl]amino}(oxo)-Essigsäure, N-methyl-D-glucamin(d.h.1-deoxy-1-(methylamino)glucitol)-Salz;
{ {4-[(4-octylphenyl)ethynyl]benzyl}[4-(trifluoromethyl)benzyl]amino}(oxo)-Essigsäure;
oxo {[4-(trifluoromethyl)benzyl][4-(5-undecyl-1,2,4-oxadiazol-3-yl)benzyl]amino}-Essigsäure;
oxo {[4-(trifluoromethyl)benzyl][4-(5-undecyl-1,2,4-oxadiazol-3-yl)benzyl]amino}-Essigsäure, N-methyl-D-glucamin(d.h. 1-deoxy-1-(methylamino)glucitol)-Salz;
{{4-[2-(4-octylphenyl)ethyl]benzyl}[4-(trifluoromethyl)benzyl]amino}(oxo)-Essigsäure;
{(4-{[4-(heptyloxy)phenyl]ethynyl}benzyl)[4-(trifluoromethyl)benzyl]amino}-(oxo)-Essigsäure;
{{4-[(4-butylphenyl)ethynyl]benzyl}[4-(trifluoromethyl)benzyl]amino}(oxo)-Essigsäure;
{{4-[(4-hexylphenyl)ethynyl]benzyl}[4-(trifluoromethyl)benzyl]amino} (oxo)-Essigsäure;
{{4-[(4-hexylphenyl)ethynyl]benzyl}[4-(trifluoromethyl)benzyl]amino}(oxo)-Essigsäure, N-methyl-D-glucamin(d.h.1-deoxy-1-(methylamino)glucitol)-Salz;
oxo {(4-{[4-(pentyloxy)phenyl]ethynyl}benzyl)[4-(trifluoromethyl)benzyl]-amino}-Essigsäure;
oxo {{4-[(4-propylphenyl)ethynyl]benzyl}[4-(trifluoromethyl)benzyl]amino}-Essigsäure;
[[2-(3-chlorophenyl)ethyl](4-dodec-1-ynylbenzyl)amino](oxo)-Essigsäure;
[[2-(3-chlorophenyl)ethyl](4-dodec-1-ynylbenzyl)amino](oxo)-Essigsäure, N-methyl-D-glucamin(d.h.1-deoxy-1-(methylamino)glucitol)-Salz;
{(4-oct-1-ynylbenzyl)[4-(trifluoromethyl)benzyl]amino}(oxo)-Essigsäure;
{[4-(11-hydroxyundec-1-ynyl)benzyl][4-(trifluoromethyl)benzyl]amino }(oxo)-Essigsäure;
{[4-(11-methoxy-11-oxoundec-1-ynyl)benzyl][4-(trifluoromethyl)benzyl]amino}-(oxo)-Essigsäure;
11-[4-({(carboxycarbonyl)[4-(trifluoromethyl)benzyl]amino}methyl)phenyl]Undec-10-insäure;
{(4-{[4-(benzyloxy)phenyl]ethynyl}benzyl)[4-(trifluoromethyl)benzyl]amino}-(oxo)-Essigsäure;
{(4-{2-[4-(heptyloxy)phenyl]ethyl}benzyl)[4-(trifluoromethyl)benzyl]amino}(oxo)-Essigsäure;
{{4-[2-(4-butylphenyl)ethyl]benzyl}[4-(trifluoromethyl)benzyl]amino}(oxo)-Essigsäure;
{{4-[2-(4-hexylphenyl)ethyl]benzyl}[4-(trifluoromethyl)benzyl]amino}(oxo)-Essigsäure;
{{4-[2-(4-hexylphenyl)ethyl]benzyl}[4-(trifluoromethyl)benzyl]amino}(oxo)-Essigsäure, N-methyl-D-glucamin(d.h.1-deoxy-1-(methylamino)glucitol)-Salz;
oxo{(4-2-[4-(pentyloxy)phenyl]ethyl}benzyl)[4-(trifluoromethyl)benzyl]-amino}-Essigsäure;
oxo {{4-[2-(4-propylphenyl)ethyl]benzyl}[4-(trifluoromethyl)benzyl]amino}-Essigsäure;
11-[4-({(carboxycarbonyl)[4-(trifluoromethyl)benzyl]amino}methyl)phenyl]-Undecansäure;
{[4-(11-hydroxyundecyl)benzyl][4-(trifluoromethyl)benzyl]amino}(oxo)-Essigsäure;
{(4-dodec-1-ynylbenzyl)[4-(trifluoromethyl)phenyl]amino}(oxo)-Essigsäure;
{(4-dodec-1-ynylbenzyl)[4-(trifluoromethyl)phenyl]amino}(oxo)-Essigsäure, N-methyl-D-glucamin(d.h.1-deoxy-1-(methylamino)glucitol)-Salz;
oxo([4-(trifluoromethyl)benzyl]{4-[2-(3-undecyl-1,2,4-oxadiazol-5-yl)ethyl]benzyl}-amino)-Essigsäure;
oxo([4-(trifluoromethyl)benzyl]{4-[2-(3-undecyl-1,2,4-oxadiazol-5-yl)ethyl]benzyl}-amino)-Essigsäure, N-methyl-D-glucamin(d.h.1-deoxy-1-(methylamino)glucitol)-Salz;
{{4-[2-(3-octyl-1,2,4-oxadiazol-5-yl)ethyl]benzyl}[4-(trifluoromethyl)benzyl]-amino}(oxo)-Essigsäure;
{{4-[2-(3-octyl-1,2,4-oxadiazol-5-yl)ethyl]benzyl}[4-(trifluoromethyl)benzyl]-amino}(oxo)-Essigsäure, N-methyl-D-glucamin(d.h.1-deoxy-1-(methylamino)glucitol)-Salz;
{{4-[(4-octylbenzoyl)amino]benzyl}[4-(trifluoromethyl)benzyl]amino}(oxo)-Essigsäure;
{{4-[(4-octylbenzoyl)amino]benzyl}[4-(trifluoromethyl)benzyl]amino}(oxo)-Essigsäure, N-methyl-D-glucamin(d.h.1-deoxy-1-(methylamino)glucitol)-Salz;
oxo {[(1-tridecanoylpiperidin-4-yl)methyl][4-(trifluoromethyl)benzyl]amino}-Essigsäure;
{{[1-(4-octylbenzoyl)piperidin-4-yl]methyl}[4-(trifluoromethyl)benzyl]-amino}-(oxo)-Essigsäure;
{{[1-(4-octylbenzoyl)piperidin-4-yl]methyl}[4-(trifluoromethyl)benzyl]amino}-(oxo)-Essigsäure, N-methyl-D-glucamin(d.h.1-deoxy-1-(methylamino)glucitol)-Salz;
{[(3-dec-1-ynyl-1-benzofuran-5-yl)methyl][4-(trifluoromethyl)benzyl]amino}-(oxo)-Essigsäure;
{[(3-dodec-1-ynyl-1-benzofuran-5-yl)methyl][4-(trifluoromethyl)benzyl]amino}-(oxo)-Essigsäure;
oxo{({3-[(4-propylphenyl)ethynyl]-1-benzofuran-5-yl}methyl)[4-(trifluoromethyl)-benzyl]amino}-Essigsäure;
[(4-dodec-1-ynylbenzyl)(4-fluorobenzyl)amino](oxo)-Essigsäure;
[bis(4-oct-1-ynylbenzyl)amino](oxo)-Essigsäure;
{[(6-dodec-1-ynylpyridin-3-yl)methyl][4-(trifluoromethyl)benzyl]amino}(oxo)-Essigsäure;
{(3-dodec-1-ynylbenzyl)[4-(trifluoromethyl)benzyl]amino}(oxo)-Essigsäure;
{[2-(2-fluorophenyl)ethyl][4-(3-undecyl-1,2,4-oxadiazol-5-yl)benzyl]amino}-(oxo)-Essigsäure;
{[2-(2-fluorophenyl)ethyl][3-(3-undecyl-1,2,4-oxadiazol-5-yl)benzyl]amino}-(oxo)-Essigsäure;
{[2-(2-fluorophenyl)ethyl][4-(3-octyl-1,2,4-oxadiazol-5-yl)benzyl]amino}(oxo)-Essigsäure;
{[2-(3,4-dichlorophenyl)ethyl][4-(3-undecyl-1,2,4-oxadiazol-5-yl)benzyl]amino}-(oxo)-Essigsäure;
{[2-(3,4-dichlorophenyl)ethyl][3-(3-undecyl-1,2,4-oxadiazol-5-yl)benzyl]amino}-(oxo)-Essigsäure;
{[2-(3,4-dichlorophenyl)ethyl][4-(3-octyl-1,2,4-oxadiazol-5-yl)benzyl]amino}(oxo)-Essigsäure;
{[2-(1,1'-biphenyl-4-yl)ethyl][4-(3-undecyl-1,2,4-oxadiazol-5-yl)benzyl]amino}-(oxo)-Essigsäure;
{[2-(1,1'-biphenyl-4-yl)ethyl][3-(3-undecyl-1,2,4-oxadiazol-5-yl)benzyl]amino}-(oxo)-Essigsäure;
{[2-(1,1'-biphenyl-4-yl)ethyl][4-(3-octyl-1,2,4-oxadiazol-5-yl)benzyl]amino}-(oxo)-Essigsäure;
oxo {5,6,7,8-tetrahydronaphthalen-1-yl[4-(3-undecyl-1,2,4-oxadiazol-5-yl)benzyl]-amino}-Essigsäure;
oxo {5,6,7,8-tetrahydronaphthalen-1-yl[3-(3-undecyl-1,2,4-oxadiazol-5-yl)benzyl]-amino}-Essigsäure;
[[4-(3-octyl-1,2,4-oxadiazol-5-yl)benzyl](5,6,7,8-tetrahydronaphthalen-1-yl)amino]-(oxo)-Essigsäure;
{(1,1'-biphenyl-3-ylmethyl)[4-(3-undecyl-1,2,4-oxadiazol-5-yl)benzyl]amino}-(oxo)-Essigsäure;
{(1,1'-biphenyl-3-ylmethyl)[3-(3-undecyl-1,2,4-oxadiazol-5-yl)benzyl]amino}-(oxo)-Essigsäure;
{(1,1'-biphenyl-3-ylmethyl)[4-(3-octyl-1,2,4-oxadiazol-5-yl)benzyl]amino}-(oxo)-Essigsäure;
{(1-benzothien-3-ylmethyl)[4-(3-undecyl-1,2,4-oxadiazol-5-yl)benzyl]amino}-(oxo)-Essigsäure;
{(1-benzothien-3-ylmethyl)[3-(3-undecyl-1,2,4-oxadiazol-5-yl)benzyl]amino}(oxo)-Essigsäure;
{(1-benzothien-3-ylmethyl)[4-(3-octyl-1,2,4-oxadiazol-5-yl)benzyl]amino}(oxo)-Essigsäure;
oxo {[2-(trifluoromethyl)benzyl][4-(3-undecyl-1,2,4-oxadiazol-5-yl)benzyl]amino}-Essigsäure;
oxo {[2-(trifluoromethyl)benzyl] [3-(3-undecyl-1,2,4-oxadiazol-5-yl)benzyl]amino}-Essigsäure;
{[4-(3-octyl-1,2,4-oxadiazol-5-yl)benzyl][2-(trifluoromethyl)benzyl]amino}(oxo)-Essigsäure;
oxo{[3-(trifluoromethyl)benzyl][4-(3-undecyl-1,2,4-oxadiazol-5-yl)benzyl]-amino}-Essigsäure;
oxo{[3-(trifluoromethyl)benzyl][3-(3-undecyl-1,2,4-oxadiazol-5-yl)benzyl]-amino}-Essigsäure;
{[4-(3-octyl-1,2,4-oxadiazol-5-yl)benzyl][3-(trifluoromethyl)benzyl]amino}-(oxo)-Essigsäure;
{(2-methoxybenzyl)[4-(3-undecyl-1,2,4-oxadiazol-5-yl)benzyl]amino}(oxo)-Essigsäure
{(2-methoxybenzyl)[3-(3-undecyl-1,2,4-oxadiazol-5-yl)benzyl]amino}(oxo)-Essigsäure;
{[2-(methoxybenzyl)[4-(3-octyl-1,2,4-oxadiazol-5-yl)benzyl]amino}(oxo)-Essigsäure;
oxo {{4-[(trifluoromethyl)sulfonyl]benzyl}[4-(3-undecyl-1,2,4-oxadiazol-5-yl)-benzyl]amino}-Essigsäure;
oxo {{4-[(trifluoromethyl)sulfonyl]benzyl}[3-(3-undecyl-1,2,4-oxadiazol-5-yl)-benzyl]amino}-Essigsäure;
([4-(3-octyl-1,2,4-oxadiazol-5-yl)benzyl] {4-[(trifluoromethyl)-sulfonyl]benzyl}-amino)(oxo)-Essigsäure;
{1,3-benzodioxol-5-yl[4-(3-undecyl-1,2,4-oxadiazol-5-yl)benzyl]amino}(oxo)-Essigsäure;
{1,3-benzodioxol-5-yl[3-(3-undecyl-1,2,4-oxadiazol-5-yl)benzyl]amino}(oxo)-Essigsäure;
{1,3-benzodioxol-5-yl[4-(3-octyl-1,2,4-oxadiazol-5-yl)benzyl]amino}(oxo)-Essigsäure;
{[(4-dodec-1-ynyl-1-naphthyl)methyl][4-(trifluoromethyl)benzyl]amino}(oxo)-Essigsäure;
{[(4-dec-1-ynyl-1-naphthyl)methyl][4-(trifluoromethyl)benzyl]amino}(oxo)-Essigsäure;
{[(4-dec-1-ynyl-1-naphthyl)methyl][4-(trifluoromethyl)benzyl]amino}(oxo)-Essigsäure;
oxo{[4-(trifluoromethyl)benzyl][4-(4-undecyl-1,3-thiazol-2-yl)benzyl]amino}-Essigsäure;
{(4-dec-1-ynylbenzyl)[2-(2-fluorophenyl)ethyl]amino}(oxo)-Essigsäure;
{(4-dodec-1-ynylbenzyl)[2-(2-fluorophenyl)ethyl]amino}(oxo)-Essigsäure;
{{[4-(dodecyloxy)-1-naphthyl]methyl}[2-(2-fluorophenyl)ethyl]amino}(oxo)-Essigsäure;
{[2-(2-fluorophenyl)ethyl][4-(octyloxy)benzyl]amino}(oxo)-Essigsäure;
{(4-dec-1-ynylbenzyl)[2-(trifluoromethyl)benzyl]amino}(oxo)-Essigsäure;
{(4-dodec-1-ynylbenzyl)[2-(trifluoromethyl)benzyl]amino}(oxo)-Essigsäure;
{{[4-(dodecyloxy)-1-naphthyl]methyl}[2-(trifluoromethyl)benzyl]amino}(oxo)-Essigsäure;
{[4-(octyloxy)benzyl][2-(trifluoromethyl)benzyl]amino}(oxo)-Essigsäure;
{(4-dec-1-ynylbenzyl)[2-(3,4-dichlorophenyl)ethyl]amino}(oxo)-Essigsäure;
[[2-(3,4-dichlorophenyl)ethyl](4-dodec-1-ynylbenzyl)amino](oxo)-Essigsäure;
([2-(3,4-dichlorophenyl)ethyl] {[4-(dodecyloxy)-1-naphthyl]methyl}amino)(oxo)-Essigsäure;
{[2-(3,4-dichlorophenyl)ethyl][4-(octyloxy)benzyl]amino}(oxo)-Essigsäure;
({4-[(4-hexylphenyl)ethynyl]benzyl}{1-methyl-1-[4-(trifluoromethyl)phenyl]ethyl}amino) (oxo)-Essigsäure;
{[4-(5-cyclohexylpent-1-ynyl)benzyl][4-(trifluoromethyl)benzyl]amino}(oxo)-Essigsäure;
{{3-[(4-hexylphenyl)ethynyl]benzyl}[4-(trifluoromethyl)benzyl]amino}(oxo)-Essigsäure;
{[4-(4-ethyl-3-hydroxyoct-1-ynyl)benzyl][4-(trifluoromethyl)benzyl]amino}-(oxo)-Essigsäure;
{(2-dec-1-ynylbenzyl)[4-(trifluoromethyl)benzyl]amino}(oxo)-Essigsäure;
{(4-dec-1-ynylbenzyl)[4-(trifluoromethyl)benzyl]amino}(oxo)-Essigsäure, L-Lysinsalz;
{(4-dec-1-ynylbenzyl)[4-(trifluoromethyl)benzyl]amino}(oxo)-Essigsäure,
tromethamin(d.h.(2-amino-2-hydroxymethyl)-1,3-propandiol)-Salz;
{(4-dec-1-ynylbenzyl)[4-(trifluoromethyl)benzyl]amino}(oxo)-Essigsäure, L-Argininsalz;
Natrium{(4-dec-1-ynylbenzyl)[4-(trifluoromethyl)benzyl]amino}(oxo)Acetat.

17. Verwendung nach einem der vorhergehenden Ansprüche, wobei die kardiovaskuläre Erkrankung Herzinsuffizienz ist.

18. Verwendung nach einem der vorhergehenden Ansprüche, wobei die kardiovaskuläre Erkrankung chronische Herzinsuffizienz ist.

19. Verwendung nach einem der vorhergehenden Ansprüche, wobei die kardiovaskuläre Erkrankung Endotheldysfunktion ist.

## Revendications

1. Utilisation d'un dérivé de méthylène-amide de formule (I) : 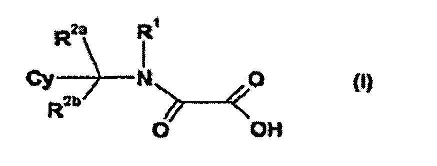 ainsi que ses isomères géométriques, ses formes optiquement actives en tant qu'énantiomères, diastéréoisomères et racémates, ainsi que les sels pharmaceutiquement acceptables et les dérivés pharmaceutiquement actifs de ceux-ci, dans laquelle
R¹ est choisi parmi les groupes alkyle en C₁-C₁₅, alcényle en C₂-C₁₂, alcynyle en C₂-C₁₂, aryle, hétéroaryle, cycloalkyle ou hétérocycloalkyle de 3 à 8 éléments, arylalkyle en C₁-C₁₂ ou hétéroarylalkyle en C₁-C₁₂, aryl- ou hétéroarylalcényle en C₂-C₁₂, et aryl- ou hétéroarylalcynyle en C₂-C₁₂ ;
R^{2a} et R^{2b} sont chacun indépendamment l'un de l'autre choisis parmi H et un groupe alkyle en C₁-C₁₂ ;
Cy est choisi parmi D et E ;
D est choisi parmi les groupes thiényle substitué et phényle substitué où les substituants sont choisis parmi les groupes phényle, oxadiazole et 1 ou 2 fragments choisis dans les groupes constitués par -NH-CO-R³, -SO₂-NR³R^{3'} et -CO-NR³R^{3'} ;
E est choisi parmi les groupes aryle, hétéroaryle, cycloalkyle et hétérocycloalkyle de 3 à 8 éléments où les groupes aryle, hétéroaryle, cycloalkyle et hétérocycloalkyle de 3 à 8 éléments sont substitués par un groupe alcynyle en C₂-C₁₈ ;
R³ et R^{3'} sont indépendamment choisis parmi H, les groupes alkyle en C₁-C₁₅, alcényle en C₂-C₁₂, alcynyle en C₂-C₁₂, aryle, hétéroaryle, cycloalkyle ou hétérocycloalkyle de 3 à 8 éléments, arylalkyle en C₁-C₁₂ ou hétéroarylalkyle en C₁-C₁₂, aryl- ou hétéroarylalcényle en C₂-C₁₂, et aryl- ou hétéroarylalcynyle en C₂-C₁₂ ;
pour la préparation d'un médicament destiné au traitement et/ou à la prévention de troubles cardiovasculaires.

2. Utilisation selon la revendication 1, dans laquelle R^{2a} et R^{2b} sont chacun H.

3. Utilisation selon la revendication 1 ou 2, dans laquelle Cy est D.

4. Utilisation selon l'une quelconque des revendications précédentes, dans laquelle R^{3'} est H et R³ est choisi parmi les groupes constitués par diphényléthyle, dodécyle, octyle, 4-pentyl-benzyle, 4-phénoxy-phénéthyle, éthyl-thiophén-2-yle, pentadécyle, tridécyle, hexyloxy-phényle ou (2-éthyl)-hexyle.

5. Utilisation selon la revendication 1, dans laquelle Cy est E.

6. Utilisation selon la revendication 5, dans laquelle E est un groupe phényle, pyridinyle, naphtyle ou benzofuranyle, substitué par B-R⁴ où B est un groupe éthynyle et R⁴ est un groupe alkyle en C₆-C₁₆, cycloalkyle de 3 à 8 éléments, (alkyle en C₁-C₁₂)-cycloalkyle de 3 à 8 éléments, phényle ou (alkyle en C₁-C₁₂)-phényle.

7. Utilisation selon la revendication 6, dans laquelle E est un groupe phényle substitué par B-R⁴ où B est un groupe éthynyle et R⁴ est un groupe alkyle en C₆-C₁₆.

8. Utilisation selon l'une quelconque des revendications précédentes, dans laquelle R¹ est un fragment -CH₂-A ou -CH₂-CH₂-A, A étant un groupe aryle, hétéroaryle, hétérocycloalkyle de 3 à 8 éléments ou cycloalkyle de 3 à 8 éléments.

9. Utilisation selon l'une quelconque des revendications 1 à 7, dans laquelle R¹ est A, A étant un groupe aryle, hétéroaryle, hétérocycloalkyle de 3 à 8 éléments ou cycloalkyle de 3 à 8 éléments.

10. Utilisation selon la revendication 8 ou 9, dans laquelle A est choisi dans le groupe constitué par phényle, pyridinyle, benzo-1,3-dioxolényle, biphényle, naphtyle, quinoxalinyle, thiazolyle, thiényle, furanyle ou pipéridinyle, éventuellement substitué par 1 ou 2 groupes cyano, halogène, NO₂, alcoxy en C₁-C₆, aryloxy ou hétéroaryloxy, thioalcoxy en C₁-C₆, alkyle en C₁-C₁₂, X-alkyle en C₁-C₁₂ dans lequel X est un atome d'halogène, un groupe alcényle en C₂-C₁₂, alcynyle en C₂-C₁₂, aryle, hétéroaryle, cycloalkyle ou hétérocycloalkyle de 3 à 8 éléments, aryl- ou hétéroarylalkyle en C₁-C₁₂, aryl- ou hétéroarylalcényle en C₂-C₁₂, aryl- ou hétéroarylalcynyle en C₂-C₁₂, -COR³, -COOR³, -CO-NR³R^{3'}, -NHCOR³, où R³ est un groupe alkyle en C₁-C₁₂ ou alcényle en C₂-C₁₂, -SOR³, -SO₂R³, -SO₂NR³R^{3'} où R³ et R³' sont indépendamment l'un de l'autre choisis dans les groupes constitués par H, les groupes alkyle en C₁-C₁₂ linéaire ou ramifié, alcényle en C₂-C₁₂, alcynyle en C₂-C₁₂, aryle, hétéroaryle, cycloalkyle et hétérocycloalkyle de 3 à 8 éléments.

11. Utilisation selon l'une quelconque des revendications 1 à 4 ou 8 à 10, dans laquelle
R^{2a} et R^{2b} sont chacun H, R¹ est -CH₂-A, A étant un groupe phényle ou thiényle, éventuellement substitué par un groupe cyano, halogène, méthoxy, hydroxy, phénoxy, -NO₂ ou trifluorométhyle ; Cy est un groupe thiényle, phényle ou biphényle substitué par -SO₂R³, - CO-NR³R^{3'} où R^{3'} est H et R³ est un groupe alkyle en C₇-C₁₂.

12. Utilisation selon l'une quelconque des revendications 1 à 4 ou 8 à 10, dans laquelle
R^{2a} et R^{2b} sont chacun H, R¹ est -CH₂-A, A étant un groupe phényle ou thiényle, éventuellement substitué par un groupe cyano, halogène, méthoxy, hydroxy, phénoxy, -NO₂ ou trifluorométhyle ; Cy est un groupe thiényle, phényle ou biphényle substitué par -SO₂R³, - CO-NR³R³' où R³' est H et R³ est un groupe alkyle en C₇-C₁₅.

13. Utilisation selon l'une quelconque des revendications 1 à 4 ou 8 à 10, dans laquelle le dérivé de méthylène-amide est de formule (I') : 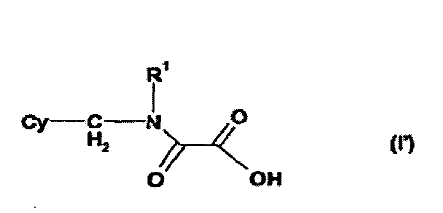 dans laquelle
R¹ est choisi dans les groupes constitués par phényle, benzyle, phénéthyle, 1-méthylbenzyle qui peuvent être substitués par un groupe alkyle en C₁-C₆ ou un groupe cycloalkyle ; Cy est un groupe phényle ou biphényle substitué avec un fragment choisi dans le groupe constitué par -NH-CO-R³ -CO-NH-R³ ou un groupe oxadiazole substitué par R³, où R³ est un groupe alkyle en C₇-C₁₅.

14. Utilisation selon la revendication 13, dans laquelle R³ est un groupe alkyle en C₈-C₁₅.

15. Utilisation selon la revendication 13 ou 14, dans laquelle R³ est un groupe dodécyle.

16. Utilisation selon l'une quelconque des revendications précédentes, dans laquelle le dérivé de méthylène-amide est choisi dans le groupe suivant :
- l'acide (benzyl{4-[(dodécylamino)carbonyl]benzyl} amino)(oxo)-acétique ;
- l'acide oxo{{4-[(pentadécylamino)carbonyl]benzyl}[4-(trifluoro-méthyl)benzyl] amino}-acétique ;
- l'acide (benzyl{4-[(pentadécylamino)carbonyl]benzyl} amino)(oxo)-acétique ;
- l'acide (benzyl{4-[(tridécylamino)carbonyl]benzyl} amino)(oxo)-acétique ;
- l'acide [benzyl(4-{[dodécyl(méthyl)amino]carbonyl} benzyl)amino) (oxo)acétique ;
- l'acide {(4-{[dodécyl(méthyl)amino]carbonyl} benzyl) [4-(trifluoro-méthyl)benzyl] amino}-(oxo)acétique ;
- l'acide ([1-(tert-butoxycarbonyl)-4-pipéridinyl]{4-[(dodécylamino)-carbonyl]benzyl} amino)-(oxo) acétique ;
- l'acide {{4-[(dodécylamino)carbonyl]benzyl}[4-(trifluorométhyl)-benzyl] amino}(oxo)-acétique ;
- l'acide {{4-[(dodécylamino)carbonyl]benzyl}[3-(trifluorométhyl)-benzyl] amino}(oxo)-acétique
- l'acide ({[1-(tert-butoxycarbonyl)-4-pipéridinyl] méthyl}{4-[(dodécyl-amino)carbonyl]benzyl} amino) (oxo) acétique ;
- l'acide oxo{[4-[(tridécanoylamino)benzyl][4-(trifluorométhyl)benzyl]-amino} acétique ;
- l'acide [benzyl(4-{[4-[(hexyloxy)benzoyl]amino} benzyl)amino](oxo)-acétique ;
- l'acide oxo{[4-[(tridécanoylamino)benzyl][4-(10-undécénoylamino)-benzyl] amino} acétique ;
- l'acide oxo{{4-[(9E)-9-tétradécénoylamino]benzyl}[4-(trifluoro-méthyl)benzyl]-amino} acétique ;
- l'acide {benzyl[4-(tridécanoylamino)benzyl] amino} (oxo) acétique ;
- l'acide {{4-[(2-hydroxydodécyl)amino]benzyl}[4-(trifluorométhyl)-benzyl] amino} (oxo)-acétique ;
- l'acide oxo{[4-(trifluorométhyl)benzyl][4-(3-undécyl-1,2,4-oxadiazol-5-yl)benzyl]-amino} acétique ;
- l'acide {({5-[(dodécylamino)sulfonyl]-2-thiényl} méthyl)[4-(trifluoro-méthyl)benzyl] amino} (oxo) acétique ;
- l'acide [{4-[(dodécylamino)carbonyl]benzyl}({1-[(4-méthoxyphényl)-sulfonyl]-4-pipéridinyl}méthyl)amino] (oxo) acétique ;
- l'acide [{4-[(dodécylamino)carbonyl]benzyl}(2-carboxy-1-phényl-éthyl)amino](oxo) acétique ;
- l'acide (4-bromo{4-[(dodécylamino)carbonyl]benzyl} anilino)(oxo)-acétique ;
- l'acide ({4-[(dodécylamino)carbonyl]benzyl} amino)(oxo) acétique ;
- l'acide ([2-(3-chlorophényl)éthyl]{4-[(dodécylamino) carbonyl]-benzyl)amino)(oxo) acétique ;
- l'acide {{4-[(dodécylamino)carbonyl]benzyl}[2-(3-méthoxyphényl)-éthyl]amino} (oxo) acétique ;
- l'acide {{4-[(dodécylamino)carbonyl]benzyl}[(d,1)-trans-2-phényl-cyclopropyl]amino} (oxo) acétique ;
- l'acide ([(d,1)-trans-2-(benzyloxy)cyclopentyl]{4-[(dodécylamino)-carbonyl]benzyl} amino)-(oxo) acétique ;
- l'acide ({4-[(dodécylamino)carbonyl]benzyl}-4-phénoxyanilino)(oxo)-acétique ;
- l'acide [{4-[(dodécylamino)carbonyl]benzyl}(1,2,3,4-tétrahydro-1-naphtalényl)amino]-(oxo) acétique ;
- l'acide ((1-benzyl-4-pipéridinyl){4-[(dodécylamino) carbonyl]benzyl}-amino)(oxo) acétique ;
- l'acide {{4-[(dodécylamino)carbonyl]benzyl}[2-(4-phénoxyphényl)-éthyl]amino} (oxo) acétique ;
- l'acide {{4-[(dodécylamino)carbonyl]benzyl}[2-(2-phénoxyphényl)-éthyl]amino} (oxo) acétique ;
- l'acide ((2-[1,1'-biphényl]-4-yléthyl){4-[(dodécyl-amino)carbonyl]-benzyl} amino)(oxo) acétique ;
- l'acide (([1,1'-biphényl]-3-ylméthyl){4-[(dodécyl-amino)carbonyl]-benzyl} amino)(oxo) acétique ;
- l'acide (3-(benzyloxy){4-[(dodécylamino)carbonyl] benzyl}anilino)-(oxo) acétique ;
- l'acide ([4-(benzoylamino)benzyl]{4-[(dodécylamine) carbonyl]-benzyl} amino)(oxo) acétique ;
- l'acide N-(carboxycarbonyl)-N-{4-[(dodécylamine) carbonyl] benzyl}-3-phényl-β-alanine;
- l'acide {{4-[(dodécylamino)carbonyl]benzyl}[4-(1,2,3-thiadiazol-4-yl)benzyl] amino} (oxo) acétique ;
- l'acide [{4-[(dodécylamino)carbonyl]benzyl}(4-pentylbenzyl)amino]-(oxo) acétique ;
- l'acide [{4-[(dodécylamino)carbonyl]benzyl}(1-phényléthyl)amino]-(oxo) acétique ;
- l'acide {{4-[(dodécylamino)carbonyl]benzyl}[1-(1-naphtyl)éthyl]-amino} (oxo) acétique ;
- l'acide (benzyl{3-[(dodécylamino)carbonyl]benzyl} amino)(oxo)-acétique ;
- l'acide {{3-[(dodécylamino)carbonyl]benzyl}[4-(méthylsulfonyl)-benzyl] amino} (oxo) acétique ;
- l'acide ((3-cyanobenzyl){3-[(dodécylamino)carbonyl] benzyl} amino)-(oxo) acétique ;
- l'acide {{3-[(dodécylamino)carbonyl] benzyl}[4-(tri-fluorométhyl)-benzyl] amino} (oxo) acétique ;
- l'acide [(4-chlorobenzyl)(3-{[(4-pentylbenzyl)amino] carbonyl)-benzyl)amino](oxo) acétique ;
- l'acide oxo{[4-({[2-(2-thiényl)éthyl]amino}carbonyl) benzyl][4-(trifluorométhyl)benzyl] amino} acétique ;
- l'acide {benzyl[(3'-{[(2,2-diphényléthyl)amino] carbonyl}[1,1'-biphényl]-4-yl)méthyl]-amino} (oxo) acétique ;
- l'acide {(3-cyanobenzyl)[(3'-{[(2,2-diphényléthyl) amino]carbonyl}-[1,1'-biphényl]-4-yl)méthyl] amino} (oxo) acétique ;
- l'acide {(4-chlorobenzyl)[(3'-{[(2,2-diphényl-éthyl)amino]carbonyl}-[1,1'-biphényl]-4-yl) méthyl]amino} (oxo) acétique ;
- l'acide {[(3'-{[(2,2-diphényléthyl)amino]carbonyl} [1,1'-biphényl]-4-yl)méthyl][4-(trifluorométhyl) benzyl] amino} (oxo) acétique ;
- l'acide ((3-cyanobenzyl){[3'-({[2-(4-phénoxyphényl) éthyl]amino}-carbonyl)[1,1'-biphényl]-4-yl] méthyl} amino)(oxo) acétique ;
- l'acide oxo{{[3'-({[2-(4-phénoxyphényl)éthyl]amino} carbonyl)[1,1'-biphényl]-4-yl]méthyl}-[4-(trifluorométhyl)benzyl] amino} acétique ;
- l'acide [(3-cyanobenzyl)({3'-[(octylamino)carbonyl] [1,1'-biphényl]-4-yl}méthyl)amino]-(oxo) acétique ;
- l'acide [(4-chlorobenzyl)({3'-[(octylamino)carbonyl] [1,1'-biphényl]-4-yl}méthyl)amino]-(oxo) acétique ;
- l'acide {({3'-[(octylamino)carbonyl][1,1'-biphényl]-4-yl}méthyl)[4-(trifluorométhyl)-benzyl] amino} (oxo) acétique ;
- l'acide {(3-cyanobenzyl)[(3'-{[(3-phénylpropyl) amino]carbonyl}[1,1'-biphényl]-4-yl)méthyl]amino} (oxo) acétique ;
- l'acide [(3-cyanobenzyl)({3'-[(dodécylamino) carbonyl][1,1'-biphényl]-4-yl}méthyl)-amino]-(oxo) acétique ;
- l'acide [(4-chlorobenzyl)({3'-[(dodécylamino) carbonyl][1,1'-biphényl]-4-yl}méthyl)-amino]-(oxo) acétique ;
- l'acide {({3'-[(dodécylamino) carbonyl][1,1'-biphényl]-4-yl}méthyl)[4-(trifluorométhyl)-benzyl] amino} (oxo) acétique ;
- l'acide {benzyl[(3'-{[(4-pentylbenzyl)amino] carbonyl}[1,1'-biphényl]-4-yl)méthyl]amino}-(oxo) acétique ;
- l'acide {(3-cyanobenzyl)[(3'-{[(4-pentylbenzyl) amino]carbonyl}[1,1'-biphényl]-4-yl)-méthyl]amino} (oxo) acétique ;
- l'acide {(4-chlorobenzyl)[(3'-{[(4-pentylbenzyl) amino]carbonyl}[1,1'-biphényl]-4-yl)-méthyl]amino} (oxo) acétique ;
- l'acide oxo{[(3'-{[(4-pentylbenzyl)amino]carbonyl} [1,1'-biphényl]-4-yl)méthyl][4-(trifluorométhyl) benzyl] amino} acétique ;
- l'acide oxo{[(3'-{[(4-phénylbutyl)amino]carbonyl} [1,1'-biphényl]-4-yl)méthyl][4-(trifluorométhyl) benzyl] amino} acétique ;
- l'acide {(3-cyanobenzyl)[(3'-{[(2-mésityléthyl) amino]carbonyl}[1,1'-biphényl]-4-yl)-méthyl]amino} (oxo) acétique ;
- l'acide {(4-chlorobenzyl)[(3'-{[(2-mésityléthyl) amino]carbonyl}[1,1'-biphényl]-4-yl)-méthyl]amino} (oxo) acétique ;
- l'acide {[(3'-{[(2-mésityléthyl)amino]carbonyl} [1,1'-biphényl]-4-yl)-méthyl][4-(trifluorométhyl) benzyl] amino} (oxo) acétique ;
- l'acide ((4-chlorobenzyl){(3'-({[2-(4-méthoxyphényl) éthyl]amino}-carbonyl)[1,1'-biphényl]-4-yl]méthyl} amino)(oxo) acétique ;
- l'acide [{4-[(dodécylamino) carbonyl] benzyl}(4-méthoxybenzyl)-amino](oxo) acétique ;
- l'acide {{4-[(dodécylamino) carbonyl] benzyl}[4-(méthylsulfonyl)-benzyl] amino} (oxo) acétique ;
- l'acide [{3-[(dodécylamino) carbonyl] benzyl}(4-méthoxybenzyl)-amino](oxo) acétique ;
- l'acide {{3-[(dodécylamino) carbonyl] benzyl}[3-(trifluorométhyl)-benzyl] amino} (oxo) acétique ;
- l'acide ({4-[(dodécylamino) carbonyl] benzyl}{[6-(trifluorométhyl)-3-pyridinyl]méthyl}amino)(oxo) acétique ;
- l'acide 4-[((carboxycarbonyl){3-[(dodécylamino) carbonyl] benzyl}-amino)méthyl]benzoïque ;
- l'acide ({3-[(dodécylamino) carbonyl] benzyl}{4-[hydroxy(oxydo)-amino]benzyl} amino)(oxo) acétique ;
- l'acide [{3-[(dodécylamino) carbonyl] benzyl}(2-fluorobenzyl)amino] (oxo) acétique ;
- l'acide [{3-[(dodécylamino) carbonyl] benzyl}(2-pyridinylméthyl)-amino](oxo) acétique ;
- l'acide [{3-[(dodécylamino) carbonyl] benzyl}(3-thiénylméthyl)amino]-(oxo) acétique ;
- l'acide [{3-[(dodécylamino) carbonyl] benzyl}(4-hydroxybenzyl)-amino](oxo) acétique ;
- l'acide [{3-[(dodécylamino) carbonyl] benzyl}(4-phénoxybenzyl)-amino](oxo) acétique ;
- l'acide ({3-[(dodécylamino) carbonyl] benzyl}{[6-(trifluorométhyl)-3-pyridinyl]méthyl}amino)(oxo) acétique ;
- l'acide 3-[((carboxycarbonyl){3-[(dodécylamino) carbonyl] benzyl}-amino)méthyl]benzoïque ;
- l'acide 5-[((carboxycarbonyl){3-[(dodécylamino) carbonyl] benzyl}-amino)méthyl]-2-thiophène-carboxylique ;
- l'acide ({4-[(dodécylamino) carbonyl] benzyl}{4-[hydroxy(oxydo)-amino]benzyl} amino)(oxo) acétique ;
- l'acide ((1,3-benzodioxol-5-ylméthyl){4-[(dodécylamino) carbonyl]-benzyl} amino)(oxo) acétique ;
- l'acide [{4-[(dodécylamino) carbonyl] benzyl}(2-fluorobenzyl)amino]-(oxo) acétique ;
- l'acide [{4-[(dodécylamino) carbonyl] benzyl}(4-phénoxybenzyl)-amino](oxo) acétique ;
- l'acide 4-[((carboxycarbonyl){4-[(dodécylamino) carbonyl] benzyl}-amino)méthyl]benzoïque ;
- l'acide 5-[((carboxycarbonyl){4-[(dodécylamino) carbonyl] benzyl}-amino)méthyl]-2-thiophéne-carboxylique ;
- l'acide [{3-[(dodécylamino) carbonyl] benzyl}(2-thiénylméthyl)amino]-(oxo) acétique ;
- l'acide [{4-[(dodécylamino) carbonyl] benzyl} (isopropyl)amino](oxo)-acétique ;
- l'acide ((3,5-dichlorobenzyl){4-[(dodécylamino) carbonyl] benzyl}-amino)(oxo) acétique ;
- l'acide [(3,5-dichlorobenzyl)(4-{[(3,3-diphényl-propyl)amino]-carbonyl} benzyl)amino](oxo) acétique ;
- l'acide [(4-{[(2-[1,1'-biphényl]-4-yléthyl)amino] carbonyl} benzyl)(3,5-dichlorobenzyl)-amino](oxo) acétique ;
- l'acide [(1,3-benzodioxol-5-ylméthyl)(4-{[(2-[1,1'-biphényl]-4-yléthyl)-amino]carbonyl}-benzyl)amino] ( oxo) acétique ;
- l'acide (2,3-dihydro-1H-indén-1-yl{4-[(dodécylamino) carbonyl]-benzyl} amino)(oxo) acétique ;
- l'acide {2,3-dihydro-1H-indén-1-yl[4-({[2-(4-phénoxyphényl)éthyl]-amino}-carbonyl)-benzyl] amino} (oxo) acétique ;
- l'acide [{4-[(dodécylamino) carbonyl] benzyl}(4-pyridinylméthyl)-amino](oxo) acétique ;
- l'acide ([4-(diméthylamino)benzyl]{4-[(dodécylamino) carbonyl]-benzyl} amino)(oxo) acétique ;
- l'acide [{4-[(dodécylamino) carbonyl] benzyl}(3-pyridinylméthyl)-amino](oxo) acétique ;
- l'acide ((4-cyanobenzyl){4-[(dodécylamino) carbonyl] benzyl} amino) (oxo) acétique ;
- l'acide [{4-[(dodécylamino) carbonyl] benzyl}(1,3-thiazol-2-ylméthyl}-amino](oxo) acétique ;
- l'acide ({4-[(dodécylamino) carbonyl] benzyl}{[2-(4-morpholinyl)-1,3-thiazol-5-yl]méthyl}amino)(oxo) acétique ;
- l'acide [{3-[(dodécylamino) carbonyl] benzyl}(4-pyridinylméthyl)-amino](oxo) acétique ;
- l'acide [{3-[(dodécylamino) carbonyl] benzyl}(3-pyridinylméthyl)-amino](oxo) acétique ;
- l'acide [{3-[(dodécylamino) carbonyl] benzyl}(3-hydroxybenzyl)-amino](oxo) acétique ;
- l'acide ((4-cyanobenzyl){3-[(dodécylamino) carbonyl] benzyl}-amino)(oxo) acétique ;
- l'acide [{3-[(dodécylamino) carbonyl] benzyl}(1,3-thiazol-2-ylméthyl)-amino](oxo) acétique ;
- l'acide ({3-[(dodécylamino) carbonyl] benzyl}{[2-(4-morpholinyl)-1,3-thiazol-5-yl]méthyl}-amino)(oxo) acétique ;
- l'acide ((1,3-benzodioxol-5-ylméthyl){3-[(dodécyl-amino) carbonyl]-benzyl}amino)-(oxo)acétique ;
- l'acide [{4-[(dodécylamino)carbonyl] benzyl}(2-thiénylméthyl)-amino](oxo) acétique ;
- l'acide [{4-[(dodécylamino) carbonyl] benzyl}(2-pyridinylméthyl)-amino](oxo) acétique ;
- l'acide [{4-[(dodécylamino) carbonyl] benzyl}(3-thiénylméthyl)amino] (oxo) acétique ;
- l'acide [{4-[(dodécylamino) carbonyl] benzyl}(4-hydroxybenzyl)amino] (oxo) acétique ;
- l'acide 3-[((carboxycarbonyl){4-[(dodécylamino) carbonyl] benzyl}-amino)méthyl]benzoïque ;
- l'acide [cyclopentyl({5-[(dodécylamino)sulfonyl]-2-thiényl}méthyl)-amino](oxo) acétique ;
- l'acide [benzyl({5-[(dodécylamino)sulfonyl]-2-thiényl}méthyl)amino]-(oxo) acétique ;
- l'acide (({5-[(dodécylamino)sulfonyl]-2-thiényl} méthyl){3-[hydroxy (oxydo)amino]-benzyl} amino)(oxo) acétique ;
- l'acide [({5-[(dodécylamino)sulfonyl]-2-thiényl} méthyl)(4-méthoxy-benzyl)amino]-(oxo)-acétique ;
- l'acide [({5-[(dodécylamino)sulfonyl]-2-thiényl} méthyl)(2-fluoro-benzyl)amino](oxo) acétique ;
- l'acide {({5-[(dodécylamino)sulfonyl]-2-thiényl} méthyl)[4-(méthyl-sulfonyl)benzyl]-amino} (oxo) acétique ;
- l'acide [({5-[(dodécylamino)sulfonyl]-2-thiényl} méthyl)(4-phénoxy-benzyl)amino](oxo) acétique ;
- l'acide 4-{[(carboxycarbonyl)({5-[(dodécyl-amino)sulfonyl]-2-thiényl}-méthyl)amino]méthyl} benzoïque ;
- l'acide (({5-[(dodécylamino)sulfonyl]-2-thiényl} méthyl){[6-(trifluoro-méthyl)-3-pyridinyl]-méthyl}amino)(oxo) acétique ;
- l'acide {({5-[(dodécylamino)sulfonyl]-2-thiényl} méthyl)[3-(trifluoro-méthyl)benzyl] amino} (oxo) acétique ;
- l'acide [(3-chlorobenzyl)({5-[(dodécylamino) sulfonyl]-2-thiényl}-méthyl)amino](oxo) acétique ;
- l'acide {[(5-{[(3,3-diphénylpropyl)amino]sulfonyl}-2-thiényl)méthyl][3-(trifluorométhyl)-benzyl] amino} (oxo) acétique ;
- l'acide {(3-chlorobenzyl)[(5-{[(3,3-diphénylpropyl) amino]sulfonyl}-2-thiényl)méthyl]amino} (oxo) acétique ;
- l'acide oxo{{[5-({[2-(4-phénoxyphényl)éthyl]amino} sulfonyl)-2-thiényl]méthyl}[3-(trifluorométhyl) benzyl] amino} acétique ;
- l'acide ((3-chlorobenzyl){[5-({[2-(4-phénoxyphényl) éthyl]amino}-sulfonyl)-2-thiényl]méthyl}amino)(oxo) acétique ;
- l'acide {[(5-{[(2-[1,1'-biphényl]-4-yléthyl)amino] sulfonyl}-2-thiényl)-méthyl][3-(trifluorométhyl) benzyl] amino} (oxo) acétique ;
- l'acide (({1-[(cyclohexylamino) carbonyl]-4-pipéridinyl}méthyl){4-[(dodécylamino)-carbonyl] benzyl} amino)(oxo) acétique ;
- l'acide ([(1-{[4-(diméthylamino)anilino]carbonyl}-4-pipéridinyl)-méthyl]{4-[(dodécyl-amino) carbonyl] benzyl} amino)(oxo) acétique ;
- l'acide {{4-[(dodécylamino) carbonyl] benzyl}[(1-hexanoyl-4-pipéridinyl)méthyl]amino} (oxo) acétique ;
- l'acide ({4-[(dodécylamino) carbonyl] benzyl}{[1-(3-iodobenzoyl)-4-pipéridinyl]méthyl}amino)(oxo) acétique ;
- l'acide {{4-[(dodécylamino) carbonyl] benzyl}[(1-{(2E)-3-[3-(trifluoro-méthyl)phényl]-2-propénoyl}-4-pipéridinyl)méthyl]amino} (oxo) acétique ;
- l'acide ({4-[(dodécylamino) carbonyl] benzyl}{[1-(1,2-quinoxalinyl-carbonyl)-4-pipéridinyl]méthyl} amino)(oxo) acétique ;
- l'acide [({1-[(4-méthoxyphényl)sulfonyl]-4-pipéridinyl}méthyl)(4-{[(4-phénoxybenzyl)amino] carbonyl} benzyl)amino](oxo) acétique ;
- l'acide [{[1-(3-iodobenzoyl)-4-pipéridinyl]méthyl} (4-{[(4-phénoxy-benzyl)amino]-carbonyl} benzyl) amino](oxo) acétique ;
- l'acide oxo{(4-{[(4-phénoxybenzyl)amino]carbonyl} benzyl)[(1-{(2E)-3-[3-(trifluorométhyl)phényl]-2-propénoyl}-4-pipéridinyl)méthyl]amino}-acétique ;
- l'acide {{4-[(dodécylamino) carbonyl]phényl}[2-(méthoxycarbonyl)-benzyl]-amino} (oxo) acétique ;
- l'acide [[4-({[2-(1,1'-biphényl-4-yl)éthyl]amino} carbonyl)-2-bromo-benzyl](4-iodobenzyl)-amino](oxo) acétique ;
- l'acide [(2-bromo-4-{[(4-pentylbenzyl)amino] carbonyl}benzyl)(4-iodobenzyl)amino]-(oxo) acétique ;
- l'acide [{2-bromo-4-[(dodécylamino) carbonyl] benzyl}(4-iodobenzyl)-amino](oxo) acétique ;
- l'acide [(2,6-dibromo-4-{[(4-pentylbenzyl)amino] carbonyl} benzyl)(4-iodobenzyl)amino]-(oxo) acétique ;
- l'acide ((4-iodobenzyl){[4'-({[2-(4-phénoxyphényl) éthyl]amino}-carbonyl)-1,1'-biphényl-4-yl]méthyl}amino)(oxo) acétique ;
- l'acide {[2-bromo-4-({[2-(4-phénoxyphényl)éthyl] amino} carbonyl)-benzyl][(4'-fluoro-1,1'-biphényl-3-yl)méthyl]amino} (oxo) acétique ;
- l'acide {[4-({[2-(1,1'-biphényl-4-yl)éthyl]amino} carbonyl)-2-bromo-benzyl][(4'-fluoro-1,1'-biphényl-3-yl)méthyl]amino} (oxo) acétique ;
- l'acide {(2-bromo-4-{[(4-pentylbenzyl)amino] carbonyl} benzyl)[(4'-fluoro-1,1'-biphényl-3-yl)méthyl]amino} (oxo) acétique ;
- l'acide {[2,6-dibromo-4-({[2-(4-phénoxyphényl)éthyl] amino}-carbonyl)benzyl][(4'-fluoro-1,1'-biphényl-3-yl)méthyl] amino} (oxo) acétique ;
- l'acide {[4-({[2-(1,1'-biphényl-4-yl)éthyl] amino} carbonyl)-2,6-dibromobenzyl][(4'-fluoro-1,1'-biphényl-3-yl)méthyl] amino} (oxo) acétique ;
- l'acide {(2,6-dibromo-4-{[(4-pentylbenzyl)amino] carbonyl} benzyl)[(4'-fluoro-1,1'-biphényl-3-yl)méthyl] amino} (oxo) acétique ;
- l'acide {{2,6-dibromo-4-[(dodécylamino) carbonyl] benzyl}[(4'-fluoro-1,1'-biphényl-3-yl)méthyl] amino} (oxo) acétique ;
- l'acide ([(4'-fluoro-1,1'-biphényl-3-yl)méthyl]{[4'-({[2-(4-phénoxy-phényl)éthyl] amino}carbonyl)-1,1'-biphényl-4-yl]méthyl}amino)(oxo) acétique ;
- l'acide {({4'-[(dodécylamino) carbonyl]-1,1'-biphényl-4-yl}méthyl)[(4'-fluoro-1,1'-biphényl-3-yl)méthyl] amino} (oxo) acétique ;
- l'acide {(2-bromo-4-{[(4-pentylbenzyl)amino] carbonyl} benzyl)[2-(trifluorométhoxy)-benzyl] amino} (oxo) acétique
- l'acide {(2,6-dibromo-4-{[(4-pentylbenzyl)amino] carbonyl} benzyl)[2-(trifluorométhoxy)-benzyl] amino} (oxo) acétique ;
- l'acide oxo{{[4'-({[2-(4-phénoxyphényl)éthyl] amino}carbonyl)-1,1'-biphényl-4-yl]méthyl}-[2-(trifluorométhoxy)benzyl] amino} acétique ;
- l'acide {({4'-[(dodécylamino) carbonyl]-1,1'-biphényl-4-yl}méthyl)[2-(trifluorométhoxy)-benzyl] amino} (oxo) acétique ;
- l'acide [[2-bromo-4-({[2-(4-phénoxyphényl)éthyl] amino}carbonyl)-benzyl](3-phénoxybenzyl)amino](oxo) acétique ;
- l'acide [[4-([{2-(1,1'-biphényl-4-yl)éthyl] amino}carbonyl)-2-bromo-benzyl](3-phénoxybenzyl) amino](oxo) acétique ;
- l'acide [(2-bromo-4-{[(4-pentylbenzyl)amino] carbonyl}benzyl)(3-phénoxybenzyl)amino](oxo) acétique ;
- l'acide [[2,6-dibromo-4-({[2-(4-phénoxyphényl)éthyl] amino}-carbonyl)benzyl](3-phénoxybenzyl)amino](oxo) acétique ;
- l'acide [[4-({[2-(1,1'-biphényl-4-yl)éthyl] amino} carbonyl)-2,6-dibromobenzyl](3-phénoxy-benzyl) amino](oxo) acétique ;
- l'acide [(2,6-dibromo-4-{[(4-pentylbenzyl)amino] carbonyl} benzyl)(3-phénoxybenzyl)-amino](oxo) acétique;
- l'acide [{2,6-dibromo-4-[(dodécylamino) carbonyl] benzyl}(3-phénoxy-benzyl)amino](oxo)-acétique ;
- l'acide oxo((3-phénoxybenzyl){[4'-({[2-(4-phénoxy-phényl)éthyl]-amino}carbonyl)-1,1'-biphényl-4-yl] méthyl}amino)acétique ;
- l'acide oxo[[(4'-{[(4-pentylbenzyl)amino] carbonyl}-1,1'-biphényl-4-yl)méthyl](3-phénoxybenzyl) amino] acétique ;
- l'acide [({4'-[(dodécylamino)carbonyl]-1,1'-biphényl-4-yl}méthyl)(3-phénoxybenzyl)-amino](oxo) acétique ;
- l'acide [[2-bromo-4-({[2-(4-phénoxyphényl)éthyl] amino}carbonyl)-benzyl](2-iodobenzyl)-amino](oxo) acétique ;
- l'acide [[4-({[2-(1,1'-biphényl-4-yl)éthyl] amino} carbonyl)-2-bromo-benzyl](2-iodobenzyl)-amino](oxo) acétique ;
- l'acide [(2-bromo-4-{[(4-pentylbenzyl)amino] carbonyl} benzyl)(2-iodobenzyl)amino]-(oxo) acétique ;
- l'acide [{2-bromo-4-[(dodécylamino) carbonyl] benzyl}(2-iodobenzyl)-amino](oxo) acétique ;
- l'acide ([2-bromo-4-({[2-(4-phénoxyphényl)éthyl] amino}carbonyl)-benzyl]{[2'-(trifluorométhyl)-1,1'-biphényl-4-yl]méthyl}amino)(oxo) acétique ;
- l'acide ([4-({[2-(1,1'-biphényl-4-yl)éthyl] amino}carbonyl)-2-bromo-benzyl]{[2'-(trifluorométhyl)-1,1'-biphényl-4-yl]méthyl}amino)(oxo) acétique ;
- l'acide ((2-bromo-4-{[(4-pentylbenzyl)amino] carbonyl} benzyl){[2'-(trifluorométhyl)-1,1'-biphényl-4-yl]méthyl}amino)(oxo) acétique ;
- l'acide ((2-bromo-4-{[(4-pentylbenzyl)amino] carbonyl} benzyl){[2'-(trifluorométhyl)-1,1'-biphényl-4-yl]méthyl}amino)(oxo) acétique ;
- l'acide ({2-bromo-4-[(dodécylamino) carbonyl] benzyl}{[2'-(trifluoro-méthyl)-1,1'-biphényl-4-yl]méthyl}amino)(oxo) acétique ;
- l'acide ([4-({[2-(1,1'-biphényl-4-yl)éthyl] amino}carbonyl)-2,6-dibromobenzyl]{[2'-(trifluorométhyl)-1,1'-biphényl-4-yl]méthyl}amino)(oxo) acétique ;
- l'acide ((2,6-dibromo-4-{[(4-pentylbenzyl)amino] carbonyl} benzyl)-{[2'-(trifluorométhyl)-1,1'-biphényl-4-yl]méthyl}amino)(oxo) acétique ;
- l'acide ({2,6-dibromo-4-[(dodécylamino) carbonyl] benzyl}{[2'-(trifluorométhyl)-1,1'-biphényl-4-yl]méthyl}amino)(oxo) acétique ;
- l'acide (({4'-[(dodécylamino) carbonyl]-1,1'-biphényl-4-yl}méthyl){[2'-(trifluorométhyl)-1,1'-biphényl-4-yl]méthyl}amino)(oxo) acétique ;
- l'acide [[4-({[2-(1,1'-biphényl-4-yl)éthyl] amino}carbonyl)-2-bromo-benzyl](1,1'-biphényl-2-ylméthyl)amino](oxo) acétique ;
- l'acide [(1,1'-biphényl-2-ylméthyl)(2-bromo-4-{[(4-pentylbenzyl)-amino] carbonyl} benzyl)-amino](oxo) acétique ;
- l'acide ((1,1'-biphényl-2-ylméthyl){2-bromo-4-[(dodécylamino)-carbonyl] benzyl} amino)(oxo) acétique ;
- l'acide {(1,1'-biphényl-2-ylméthyl)[2,6-dibromo-4-({[2-(4-phénoxy-phényl)éthyl] amino}carbonyl)benzyl] amino} (oxo) acétique ;
- l'acide [[4-({[2-(1,1'-biphényl-4-yl)éthyl] amino} carbonyl)-2,6-dibromobenzyl](1,1'-biphényl-2-ylméthyl)amino](oxo) acétique ;
- l'acide [(1,1'-biphényl-2-ylméthyl)(2,6-dibromo-4-{[(4-pentylbenzyl)-amino] carbonyl} benzyl)amino] (oxo) acétique ;
- l'acide ((1,1'-biphényl-2-ylméthyl){2,6-dibromo-4-[(dodécylamino)-carbonyl] benzyl}-amino)(oxo) acétique ;
- l'acide {(2-bromo-4-{[(4-pentylbenzyl)amino] carbonyl}benzyl)[4-(trifluorométhoxy)benzyl]amino} (oxo) acétique ;
- l'acide {{2-bromo-4-[(dodécylamino) carbonyl] benzyl}[4-(trifluoro-méthoxy)benzyl] amino} (oxo) acétique ;
- l'acide {(2,6-dibromo-4-{[(4-pentylbenzyl)amino] carbonyl}benzyl)[4-(trifluorométhoxy)benzyl] amino} (oxo) acétique ;
- l'acide {(2-bromo-4-{[(4-pentylbenzyl)amino] carbonyl} benzyl)[3-(trifluorométhoxy)benzyl] amino} (oxo) acétique ;
- l'acide {{2-bromo-4-[(dodécylamino) carbonyl] benzyl}[3-(trifluoro-méthoxy)benzyl] amino} (oxo) acétique ;
- l'acide {(2,6-dibromo-4-{[(4-pentylbenzyl)amino] carbonyl} benzyl)[3-(trifluorométhoxy)-benzyl] amino} (oxo) acétique ;
- l'acide {{2,6-dibromo-4-[(dodécylamino) carbonyl] benzyl}[3-(trifluoro-méthoxy)benzyl]-amino} (oxo) acétique ;
- l'acide {({4'-[(dodécylamino) carbonyl]-1,1'-biphényl-4-yl}méthyl)[3-(trifluorométhoxy)-benzyl] amino} (oxo) acétique ;
- l'acide [[2-bromo-4-({[2-(4-phénoxyphényl)éthyl] amino}carbonyl)-benzyl](4-phénoxy-benzyl)amino](oxo) acétique ;
- l'acide [[4-({[2-(1,1'-biphényl-4-yl)éthyl] amino} carbonyl)-2-bromo-benzyl](4-phénoxybenzyl)amino] (oxo) acétique ;
- l'acide [(2-bromo-4-{[(4-pentylbenzyl)amino] carbonyl} benzyl)(4-phénoxybenzyl)amino](oxo) acétique;
- l'acide [{2-bromo-4-[(dodécylamino) carbonyl] benzyl}(4-phénoxy-benzyl)amino](oxo) acétique ;
- l'acide [[4-({[2-(1,1'-biphényl-4-yl)éthyl] amino}carbonyl)-2,6-dibromobenzyl](4-phénoxy-benzyl)amino](oxo) acétique ;
- l'acide [(2,6-dibromo-4-{[(4-pentylbenzyl)amino] carbonyl}benzyl)(4-phénoxybenzyl)-amino](oxo) acétique ;
- l'acide {[4-({[2-(1,1'-biphényl-4-yl)éthyl] amino}carbonyl)-2-bromo-benzyl][4-(trifluoro-méthyl)benzyl] amino} (oxo) acétique ;
- l'acide {(2-bromo-4-{[(4-pentylbenzyl)amino] carbonyl} benzyl)[4-(trifluorométhyl)benzyl] amino} (oxo) acétique ;
- l'acide {{2-bromo-4-[(dodécylamino) carbonyl] benzyl}[4-(trifluoro-méthyl)benzyl] amino} (oxo) acétique ;
- l'acide {(2,6-dibromo-4-{[(4-pentylbenzyl)amino] carbonyl} benzyl)[4-(trifluorométhyl)benzyl] amino} (oxo) acétique ;
- l'acide {{2,6-dibromo-4-[(dodécylamino)carbonyl] benzyl}[4-(trifluoro-méthyl)benzyl]-amino} (oxo) acétique ;
- l'acide oxo{[(4'-{[(4-pentylbenzyl)amino] carbonyl}-1,1'-biphényl-4-yl)méthyl][4-(trifluoro-méthyl)benzyl] amino} acétique ;
- l'acide {{2-bromo-4-[(dodécylamino) carbonyl] benzyl}[3-(trifluoro-méthyl)benzyl] amino} (oxo) acétique ;
- l'acide {{2,6-dibromo-4-[(dodécylamino) carbonyl] benzyl}[3-(trifluoro-méthyl)benzyl]-amino} (oxo) acétique ;
- l'acide oxo{[(4'-{[(4-pentylbenzyl)amino] carbonyl}-1,1'-biphényl-4-yl)méthyl][3-(trifluorométhyl) benzyl] amino} acétique ;
- l'acide {(4-dibenzo[b,d]furan-4-ylbenzyl)[4-(trifluorométhyl)benzyl]-amino} (oxo) acétique ;
- l'acide {(4-dibenzo[b,d]furan-4-ylbenzyl)[4-(trifluorométhyl)benzyl]-amino} (oxo) acétique, sel de N-méthyl-D-glucamine (c'est-à-dire 1-désoxy-1-(méthylamino) glucitol) ;
- l'acide ({4-[(dodécylamino) carbonyl] benzyl}{1-[4-(trifluorométhyl)-phényl] éthyl}amino)(oxo) acétique ;
- l'acide ({4-[(dodécylamino) carbonyl] benzyl}{1-[4-(trifluorométhyl)-phényl] éthyl}amino)(oxo) acétique, sel de N-méthyl-D-glucamine (c'est-à-dire 1-désoxy-1-(méthylamino) glucitol) ;
- l'acide {({4'-[(octylamino) carbonyl]-1,1'-biphényl-4-yl}méthyl)[4-(trifluorométhyl)benzyl] amino} (oxo) acétique ;
- l'acide oxo{(4-tétradéc-1-ynylbenzyl)[4-(trifluorométhyl)benzyl]-amino} acétique ;
- l'acide {(4-dodéc-1-ynylbenzyl)[4-(trifluorométhyl)benzyl] amino} (oxo) acétique ;
- l'acide {{4-[(dodécylamino) carbonyl] benzyl}[4-(trifluorométhyl)-phényl]amino} (oxo) acétique ;
- l'acide [{4-[(dodécylamino) carbonyl] benzyl}(2-méthoxyphényl)-amino](oxo) acétique ;
- l'acide ((1,2-diphényléthyl){4-[(dodécylamino) carbonyl] benzyl}-amino)(oxo) acétique ;
- la N-(carboxycarbonyl)-N-{4-[(dodécylamino) carbonyl] benzyl}-L-phénylalanine ;
- l'acide [{4-[(dodécylamino) carbonyl] benzyl}(3-phénoxyphényl)-amino](oxo) acétique ;
- l'acide [{4-[(dodécylamino) carbonyl] benzyl}(2-isopropoxyphényl)-amino](oxo) acétique ;
- l'acide [{4-[(dodécylamino) carbonyl] benzyl}(4-iodophényl)amino]-(oxo) acétique ;
- l'acide {{4-[(dodécylamino) carbonyl] benzyl}[3-fluoro-4-(trifluoro-méthyl)benzyl]-amino} (oxo) acétique ;
- l'acide ((3-chloro-2-méthylphényl){4-[(dodécylamino) carbonyl]-benzyl} amino} (oxo) acétique ;
- l'acide 4'-((carboxycarbonyl){4-[(dodécylamino) carbonyl] benzyl}-amino)-1,1'-biphényl-2-carboxylique ;
- l'acide ((2,4-dichlorobenzyl){4-[(dodécylamino) carbonyl] benzyl}-amino)(oxo) acétique ;
- l'acide [{4-[(dodécylamino) carbonyl] benzyl}(1-phénylpropyl)amino]-(oxo) acétique ;
- l'acide ([2-(4-chlorophényl)propyl]{4-[(dodécyl-amino) carbonyl]-benzyl} amino} (oxo) acétique ;
- l'acide [{4-[(dodécylamino) carbonyl] benzyl}(4-isopropoxyphényl)-amino](oxo) acétique ;
- l'acide ([4-(benzyloxy)phényl]{4-[(dodécylamino) carbonyl] benzyl}-amino)(oxo) acétique ;
- l'acide {{4-[(dodécylamino) carbonyl] benzyl}[2-(trifluorométhyl)-benzyl] amino} (oxo) acétique ;
- l'acide [{4-[(dodécylamino) carbonyl] benzyl}(2-méthoxybenzyl)-amino](oxo) acétique ;
- l'acide ([(1R)-1-(4-chlorophényl)éthyl]{4-[(dodécyl-amino) carbonyl]-benzyl} amino)-(oxo) acétique
- l'acide ((3,4-dichlorobenzyl){4-[(dodécylamino) carbonyl] benzyl}-amino)(oxo) acétique ;
- l'acide (1-benzothién-3-ylméthyl){4-[(dodécylamino) carbonyl]-benzyl}amino)(oxo) acétique ;
- l'acide ([2-(2,6-dichlorophényl)éthyl]{4-[(dodécyl-amino) carbonyl]-benzyl} amino)(oxo) acétique ;
- l'acide ({4-[(dodécylamino) carbonyl] benzyl}{2-[3-(trifluorométhyl)-phényl] éthyl}-amino)-(oxo) acétique ;
- l'acide {{4-[(dodécylamino) carbonyl] benzyl}[2-(3-fluorophényl)-éthyl] amino} (oxo) acétique ;
- l'acide ([(1S)-1-(4-chlorophényl)éthyl]{4-[(dodécyl-amino) carbonyl]-benzyl} amino)(oxo) acétique ;
- l'acide {{4-[(dodécylamino) carbonyl] benzyl}[(1S)-1-phényléthyl]-amino} (oxo) acétique ;
- l'acide {{4-[(dodécylamino) carbonyl] benzyl}[(1R)-1-phényléthyl]-amino} (oxo) acétique ;
- l'acide ([3-(benzyloxy)phényl]{4-[(dodécylamino) carbonyl] benzyl}-amino)(oxo) acétique ;
- la N-(carboxycarbonyl)-N-{4-[(dodécylamino) carbonyl] benzyl}-D-phénylalanine;
- l'acide {{4-[(dodécylamino) carbonyl]phényl}[4-(trifluorométhyl)-benzyl] amino} (oxo) acétique ;
- l'acide {{4-[(dodécylamino) carbonyl]phényl}[4-(trifluorométhyl)-benzyl] amino} (oxo) acétique, sel de N-méthyl-D-glucamine (c'est-à-dire 1-désoxy-1-(méthylamino) glucitol) ;
- l'acide oxo{{1-[4-(trifluorométhyl)phényl] éthyl}[4-(3-undécyl-1,2,4-oxadiazol-5-yl)benzyl] amino} acétique ;
- l'acide oxo{{1-[4-(trifluorométhyl)phényl]éthyl}[4-(3-undécyl-1,2,4-oxadiazol-5-yl)benzyl]amino) acétique, sel de N-méthyl-D-glucamine (c'est-à-dire 1-désoxy-1-(méthylamino) glucitol) ;
- l'acide ([(2-butyl-1-benzofuran-3-yl)méthyl]{4-[(dodécylamino)-carbonyl] benzyl}-amino)-(oxo) acétique ;
- l'acide {(1-{4-[(dodécylamino) carbonyl]phényl} éthyl)[4-(trifluoro-méthyl)benzyl] amino}-(oxo) acétique ;
- l'acide {(1-{4-[(dodécylamino) carbonyl]phényl} éthyl)[4-(trifluoro-méthyl)benzyl] amino} (oxo) acétique, sel de N-méthyl-D-glucamine (c'est-à-dire 1-désoxy-1-(méthylamino) glucitol) ;
- l'acide {(4-{[(4-octylphényl)amino] carbonyl} benzyl)[4-(trifluoro-méthyl)benzyl]-amino} (oxo) acétique ;
- l'acide {(3-chlorobenzyl)[4-(3-undécyl-1,2,4-oxadiazol-5-yl)benzyl]-amino} (oxo) acétique ;
- l'acide {(3-chlorobenzyl)[4-(3-undécyl-1,2,4-oxadiazol-5-yl)benzyl]-amino} (oxo) acétique, sel de N-méthyl-D-glucamine (c'est-à-dire 1-désoxy-1-(méthylamino) glucitol) ;
- l'acide {{cyclopentyl[4-(trifluorométhyl)phényl] méthyl}[4-(tridécanoyl-amino)benzyl]amino} (oxo) acétique ;
- l'acide oxo([4-(trifluorométhyl)benzyl]{[4-(3-undécyl-1,2,4-oxadiazol-5-yl)-1-naphtyl]-méthyl} amino)acétique ;
- l'acide oxo([4-(trifluorométhyl)benzyl]{[4-(3-undécyl-1,2,4-oxadiazol-5-yl)-1-naphtyl]-méthyl} amino)acétique, sel de N-méthyl-D-glucamine (c'est-à-dire 1-désoxy-1-(méthylamino)glucitol) ;
- l'acide {{cyclopentyl[4-(trifluorométhyl)phényl] méthyl}[4-(3-undécyl-1,2,4-oxadiazol-5-yl)benzyl] amino} (oxo) acétique ;
- l'acide {{cyclopentyl[4-(trifluorométhyl)phényl] méthyl}[4-(3-undécyl-1,2,4-oxadiazol-5-yl)benzyl] amino} (oxo) acétique, sel de N-méthyl-D-glucamine (c'est-à-dire 1-désoxy-1-(méthylamino) glucitol) ;
- l'acide {(4-dibenzo[b,d]furan-4-ylphényl)[4-(trifluorométhyl)benzyl]-amino} (oxo) acétique ;
- l'acide {(4-dibenzo[b,d]furan-4-ylphényl)[4-(trifluorométhyl)benzyl]-amino} (oxo) acétique, sel de N-méthyl-D-glucamine (c'est-à-dire 1-désoxy-1-(méthylamino) glucitol) ;
- l'acide {[4-(octyloxy)benzyl][4-(trifluorométhyl) benzyl] amino} (oxo)-acétique ;
- l'acide {[4-(octyloxy)benzyl][4-(trifluorométhyl) benzyl] amino} (oxo)-acétique, sel de N-méthyl-D-glucamine (c'est-à-dire 1-désoxy-1-(méthyl-amino) glucitol) ;
- l'acide [[2-(3-chlorophényl)éthyl](4-déc-1-ynylbenzyl)amino](oxo)-acétique ;
- l'acide ([2-(3-chlorophényl)éthyl]{4-[(1Z)-déc-1-ényl]benzyl} amino}-(oxo) acétique ;
- l'acide {[2-(3-chlorophényl)éthyl][4-(3-undécyl-1,2,4-oxadiazol-5-yl)-benzyl] amino} (oxo)-acétique ;
- l'acide {[2-(3-chlorophényl)éthyl][4-(3-undécyl-1,2,4-oxadiazol-5-yl)-benzyl] amino} (oxo)-acétique, sel de N-méthyl-D-glucamine (c'est-à-dire 1-désoxy-1-(méthylamino) glucitol) ;
- l'acide oxo{{(1R)-1-[4-(trifluorométhyl)phényl] éthyl}[4-(3-undécyl-1,2,4-oxadiazol-5-yl)benzyl] amino} acétique ;
- l'acide oxo{{(1R)-1-[4-(trifluorométhyl)phényl] éthyl}[4-(3-undécyl-1,2,4-oxadiazol-5-yl)benzyl] amino} acétique, sel de N-méthyl-D-glucamine (c'est-à-dire 1-désoxy-1-(méthylamino) glucitol) ;
- l'acide oxo{[4-(trifluorométhyl)phényl][4-(3-undécyl-1,2,4-oxadiazol-5-yl)benzyl] amino}-acétique ;
- l'acide oxo{[4-(trifluorométhyl)phényl][4-(3-undécyl-1,2,4-oxadiazol-5-yl)benzyl] amino)-acétique, sel de N-méthyl-D-glucamine (c'est-à-dire 1-désoxy-1-(méthylamino) glucitol) ;
- l'acide oxo{{(1S)-1-[4-(trifluorométhyl)phényl] éthyl}[4-(3-undécyl-1,2,4-oxadiazol-5-yl)benzyl] amino} acétique ;
- l'acide oxo{{(1S)-1-[4-(trifluorométhyl)phényl] éthyl}[4-(3-undécyl-1,2,4-oxadiazol-5-yl)benzyl] amino} acétique, sel de N-méthyl-D-glucamine (c'est-à-dire 1-désoxy-1-(méthylamino) glucitol) ;
- l'acide [(3-chlorobenzyl)(4-déc-1-ynylbenzyl)amino] (oxo) acétique ;
- l'acide [(3-chlorobenzyl)(4-déc-1-ynylbenzyl)amino] (oxo) acétique, sel de N-méthyl-D-glucamine (c'est-à-dire 1-désoxy-1-(méthylamino)-glucitol-) ;
- l'acide [[2-(3-chlorophényl)éthyl](4-oct-1-ynylbenzyl)amino](oxo)-acétique ;
- l'acide [[2-(3-chlorophényl)éthyl](4-oct-1-ynylbenzyl)amino](oxo)-acétique, sel de N-méthyl-D-glucamine (c'est-à-dire 1-désoxy-1-(méthylamino) glucitol) ;
- l'acide {(4-déc-1-ynylbenzyl)[4-(trifluorométhyl) phényl]amino} (oxo)-acétique ;
- l'acide ((4-déc-1-ynylbenzyl){1-[4-(trifluorométhyl) phényl] éthyl}-amino)(oxo) acétique ;
- l'acide ((4-déc-1-ynylbenzyl){1-[4-(trifluorométhyl) phényl] éthyl}-amino)(oxo) acétique, sel de N-méthyl-D-glucamine (c'est-à-dire 1-désoxy-1-(méthylamino) glucitol) ;
- l'acide {{1-méthyl-1-[4-(trifluorométhyl)phényl] éthyl}[4-(3-undécyl-1,2,4-oxadiazol-5-yl)benzyl] amino} (oxo) acétique ;
- l'acide {{1-méthyl-1-[4-(trifluorométhyl)phényl] éthyl}[4-(3-undécyl-1,2,4-oxadiazol-5-yl)benzyl] amino} (oxo) acétique, sel de N-méthyl-D-glucamine (c'est-à-dire 1-désoxy-1-(méthylamino) glucitol) ;
- l'acide {[2-(3-chlorophényl)éthyl][4-(3-octyl-1,2,4-oxadiazol-5-yl)-benzyl] amino} (oxo) acétique ;
- l'acide {[2-(3-chlorophényl)éthyl][4-(3-octyl-1,2,4-oxadiazol-5-yl)-benzyl] amino} (oxo) acétique, sel de N-méthyl-D-glucamine (c'est-à-dire 1-désoxy-1-(méthylamino) glucitol) ;
- l'acide {[4-(3-octyl-1,2,4-oxadiazol-5-yl)benzyl][4-(trifluorométhyl)-benzyl] amino} (oxo) acétique ;
- l'acide {[4-(3-octyl-1,2,4-oxadiazol-5-yl)benzyl][4-(trifluorométhyl)-benzyl] amino} (oxo) acétique, sel de N-méthyl-D-glucamine (c'est-à-dire 1-désoxy-1-(méthylamino) glucitol) ;
- l'acide {{[4-(dodécyloxy)-1-naphtyl]méthyl}[4-(trifluorométhyl)-benzyl] amino} (oxo) acétique ;
- l'acide {{[4-(dodécyloxy)-1-naphtyl]méthyl}[4-(trifluorométhyl)-benzyl] amino} (oxo) acétique, sel de N-méthyl-D-glucamine (c'est-à-dire 1-désoxy-1-(méthylamino) glucitol) ;
- l'acide [(4-bromobenzyl)(4-oct-1-ynylbenzyl) amino] (oxo) acétique ;
- l'acide [{4-[(dodécylamino) carbonyl] benzyl}(2-hydroxy-1-phényl-éthyl)amino](oxo) acétique ;
- l'acide ((4-déc-1-ynylbenzyl){1-méthyl-1-[4-(trifluorométhyl)phényl]-éthyl}amino} (oxo)-acétique ;
- l'acide ((4-déc-1-ynylbenzyl){1-méthyl-1-[4-(trifluorométhyl)phényl]-éthyl}amino)(oxo)-acétique, sel de N-méthyl-D-glucamine (c'est-à-dire 1-désoxy-1-(méthylamino) glucitol) ;
- l'acide oxo{{4-[(9Z)-tétradéc-9-énoylamino]benzyl} [4-(trifluoro-méthyl)benzyl] amino} acétique ;
- l'acide {(4-déc-1-ynylbenzyl)[4-(trifluorométhyl) benzyl] amino} (oxo)-acétique ;
- l'acide oxo{[4-(trifluorométhyl)benzyl][3-(3-undécyl -1,2,4-oxadiazol-5-yl)benzyl] amino} acétique ;
- l'acide oxo{[4-(trifluorométhyl)benzyl][3-(3-undécyl -1,2,4-oxadiazol-5-yl)benzyl] amino} acétique, sel de N-méthyl-D-glucamine (c'est-à-dire 1-désoxy-1-(méthylamino) glucitol) ;
- l'acide {(4-dodécylbenzyl)[4-(trifluorométhyl) benzyl] amino} (oxo)-acétique ;
- l'acide {(4-dodécylbenzyl)[4-(trifluorométhyl) benzyl] amino} (oxo)-acétique, sel de N-méthyl-D-glucamine (c'est-à-dire 1-désoxy-1-(méthylamino) glucitol) ;
- l'acide {[4-({[(2-butyl-1-benzofuran-3-yl)méthyl] amino}carbonyl)-benzyl][4-(trifluorométhyl)benzyl] amino} (oxo) acétique ;
- l'acide {(4-{[4-(benzyloxy)benzoyl]amino} benzyl)[4-(trifluorométhyl)-benzyl] amino} (oxo) acétique ;
- l'acide {(3,5-dichlorobenzyl)[4-(tridécanoylamino) benzyl] amino}-(oxo) acétique ;
- l'acide {(3,5-dichlorobenzyl)[4-(tridécanoylamino) benzyl] amino}-(oxo) acétique, sel de N-méthyl-D-glucamine (c'est-à-dire 1-désoxy-1-(méthylamino) glucitol) ;
- l'acide {{4-[(4-octylphényl)éthynyl]benzyl}[4-(trifluorométhyl)benzyl]-amino} (oxo) acétique ;
- l'acide oxo{[4-(trifluorométhyl)benzyl][4-(5-undécyl-1,2,4-oxadiazol-3-yl)benzyl] amino} acétique ;
- l'acide oxo{[4-(trifluorométhyl)benzyl][4-(5-undécyl-1,2,4-oxadiazol-3-yl)benzyl] amino} acétique, sel de N-méthyl-D-glucamine (c'est-à-dire 1-désoxy-1-(méthylamino) glucitol) ;
- l'acide {{4-[2-(4-octylphényl)éthyl]benzyl}[4-(trifluorométhyl)benzyl]-amino} (oxo) acétique ;
- l'acide {(4-{[4-(heptyloxy)phényl]éthynyl}benzyl)[4-(trifluorométhyl)-benzyl] amino} (oxo) acétique ;
- l'acide {{4-[(4-butylphényl)éthynyl]benzyl}[4-(trifluorométhyl)benzyl]-amino} (oxo) acétique ;
- l'acide {{4-[(4-hexylphényl)éthynyl]benzyl}[4-(trifluorométhyl)benzyl]-amino} (oxo) acétique ;
- l'acide {{4-[(4-hexylphényl)éthynyl]benzyl}[4-(trifluorométhyl)benzyl]-amino} (oxo) acétique, sel de N-méthyl-D-glucamine (c'est-à-dire 1-désoxy-1-(méthylamino) glucitol) ;
- l'acide oxo{(4-{[4-(pentyloxy)phényl]éthynyl} benzyl)[4-(trifluoro-méthyl)benzyl] amino)acétique ;
- l'acide oxo{{4-[(4-propylphényl)éthynyl]benzyl}[4-(trifluorométhyl)-benzyl] amino} acétique ;
- l'acide [[2-(3-chlorophényl)éthyl](4-dodéc-1-ynylbenzyl)amino](oxo)-acétique ;
- l'acide [[2-(3-chlorophényl)éthyl](4-dodéc-1-ynylbenzyl)amino](oxo)-acétique, sel de N-méthyl-D-glucamine (c'est-à-dire 1-désoxy-1-(méthyl-amino) glucitol) ;
- l'acide {(4-oct-1-ynylbenzyl)[4-(trifluorométhyl) benzyl] amino} (oxo)-acétique ;
- l'acide {[4-(11-hydroxyundéc-1-ynyl)benzyl][4-(trifluorométhyl)-benzyl] amino} (oxo) acétique ;
- l'acide {[4-(11-méthoxy-11-oxoundéc-1-ynyl) benzyl][4-(trifluoro-méthyl)benzyl] amino} (oxo) acétique ;
- l'acide 11-[4-({(carboxycarbonyl)[4-(trifluorométhyl)benzyl] amino}-méthyl)phényl]undéc-10-ynoïque ;
- l'acide {(4-{[4-(benzyloxy)phényl]éthynyl}benzyl)[4-(trifluorométhyl)-benzyl] amino}-(oxo) acétique ;
- l'acide {(4-{2-[4(heptyloxy)phényl] éthyl}benzyl)[4-(trifluorométhyl)-benzyl] amino} (oxo) acétique ;
- l'acide {{4-[2-(4-butylphényl)éthyl]benzyl}[4-(trifluorométhyl)benzyl]-amino} (oxo) acétique ;
- l'acide {{4-[2-(4-hexylphényl)éthyl]benzyl}[4-(trifluorométhyl)benzyl]-amino} (oxo) acétique ;
- l'acide {{4-[2-(4-hexylphényl)éthyl]benzyl}[4-(trifluorométhyl)benzyl]-amino} (oxo) acétique, sel de N-méthyl-D-glucamine (c'est-à-dire 1-désoxy-1-(méthylamino) glucitol) ;
- l'acide oxo{(4-{2-[4-(pentyloxy)phényl] éthyl} benzyl)[4-(trifluoro-méthyl)benzyl] amino} acétique ;
- l'acide oxo{{4-[2-(4-propylphényl)éthyl]benzyl}[4-(trifluorométhyl)-benzyl] amino} acétique ;
- l'acide 11-[4-({(carboxycarbonyl)[4-(trifluorométhyl) benzyl] amino}-méthyl)phényl]-undécanoïque
- l'acide {[4-(11-hydroxyundécyl)benzyl][4-(trifluorométhyl) benzyl]-amino} (oxo) acétique ;
- l'acide {(4-dodéc-1-ynylbenzyl)[4-(trifluorométhyl) phényl]amino}-(oxo) acétique ;
- l'acide {(4-dodéc-1-ynylbenzyl)[4-(trifluorométhyl) phényl]amino}-(oxo) acétique, sel de N-méthyl-D-glucamine (c'est-à-dire 1-désoxy-1-(méthylamino) glucitol) ;
- l'acide oxo([4-(trifluorométhyl)benzyl]{4-[2-(3-undécyl-1,2,4-oxadiazol-5-yl)éthyl]benzyl} amino) acétique ;
- l'acide oxo([4-(trifluorométhyl)benzyl]{4-[2-(3-undécyl-1,2,4-oxadiazol-5-yl)éthyl]benzyl}-amino)acétique, sel de N-méthyl-D-glucamine (c'est-à-dire 1-désoxy-1-(méthylamino) glucitol) ;
- l'acide {{4-[2-(3-octyl-1,2,4-oxadiazol-5-yl)éthyl] benzyl}[4-(trifluoro-méthyl)benzyl]-amino} (oxo) acétique ;
- l'acide {{4-[2-(3-octyl-1,2,4-oxadiazol-5-yl)éthyl] benzyl}[4-(trifluoro-méthyl)benzyl] amino} (oxo) acétique, sel de N-méthyl-D-glucamine (c'est-à-dire 1-désoxy-1-(méthylamino) glucitol) ;
- l'acide {{4-[(4-octylbenzoyl)amino]benzyl}[4-(trifluorométhyl)benzyl]-amino} (oxo) acétique ;
- l'acide {{4-[(4-octylbenzoyl)amino]benzyl}[4-(trifluorométhyl)benzyl]-amino} (oxo) acétique, sel de N-méthyl-D-glucamine (c'est-à-dire 1-désoxy-1-(méthylamino) glucitol) ;
- l'acide oxo{[(1-tridécanoylpipéridin-4-yl)méthyl][4-(trifluorométhyl)-benzyl] amino} acétique ;
- l'acide {{[1-(4-octylbenzoyl)pipéridin-4-yl]methyl} [4-(trifluorométhyl)-benzyl]-amino} (oxo) acétique ;
- l'acide {{[1-(4-octylbenzoyl)pipéridin-4-yl]méthyl} [4-(trifluorométhyl)-benzyl] amino} (oxo) acétique, sel de N-méthyl-D-glucamine (c'est-à-dire 1-désoxy-1-(méthylamino) glucitol) ;
- l'acide {[(3-déc-1-ynyl-1-benzofuran-5-yl)méthyl][4-(trifluorométhyl)-benzyl] amino} (oxo) acétique ;
- l'acide {[(3-dodéc-1-ynyl-1-benzofuran-5-yl)méthyl] [4-(trifluoro-méthyl)benzyl] amino} (oxo) acétique;
- l'acide oxo{({3-[(4-propylphényl)éthynyl]-1-benzofuran-5-yl}méthyl)-[4-(trifluorométhyl)-benzyl] amino} acétique ;
- l'acide [(4-dodéc-1-ynylbenzyl)(4-fluorobenzyl) amino](oxo)-acétique ;
- l'acide [bis(4-oct-1-ynylbenzyl)amino](oxo) acétique ;
- l'acide {[(6-dodéc-1-ynylpyridin-3-yl)méthyl][4-(trifluorométhyl)-benzyl] amino} (oxo) acétique ;
- l'acide {(3-dodéc-1-ynylbenzyl)[4-(trifluorométhyl) benzyl] amino}-(oxo) acétique ;
- l'acide {[2-(2-fluorophényl)éthyl][4-(3-undécyl-1,2,4-oxadiazol-5-yl)-benzyl] amino} (oxo) acétique ;
- l'acide {[2-(2-fluorophényl)éthyl][3-(3-undécyl-1,2,4-oxadiazol-5-yl)-benzyl] amino}-(oxo) acétique ;
- l'acide {[2-(2-fluorophényl)éthyl][4-(3-octyl-1,2,4-oxadiazol-5-yl)-benzyl] amino} (oxo) acétique ;
- l'acide {[2-(3,4-dichlorophényl)éthyl][4-(3-undécyl-1,2,4-oxadiazol-5-yl)benzyl] amino} (oxo) acétique ;
- l'acide {[2-(3,4-dichlorophényl)éthyl][3-(3-undécyl-1,2,4-oxadiazol-5-yl)benzyl] amino} (oxo) acétique ;
- l'acide {[2-(3,4-dichlorophényl)éthyl][4-(3-octyl-1,2,4-oxadiazol-5-yl)benzyl] amino} (oxo) acétique ;
- l'acide {[2-(1,1'-biphényl-4-yl)éthyl][4-(3-undécyl-1,2,4-oxadiazol-5-yl)benzyl] amino} (oxo) acétique ;
- l'acide {[2-(1,1'-biphényl-4-yl)éthyl][3-(3-undécyl-1,2,4-oxadiazol-5-yl)benzyl] amino} (oxo) acétique
- l'acide {[2-(1,1'-biphényl-4-yl)éthyl][4-(3-octyl-1,2,4-oxadiazol-5-yl)benzyl] amino} (oxo) acétique ;
- l'acide oxo{5,6,7,8-tétrahydronaphtalén-1-yl[4-(3-undécyl-1,2,4-oxadiazol-5-yl)benzyl]-amino} acétique ;
- l'acide oxo{5,6,7,8-tétrahydronaphtalén-1-yl[3-(3-undécyl-1,2,4-oxadiazol-5-yl)benzyl]-amino} acétique ;
- l'acide [[4-(3-octyl-1,2,4-oxadiazol-5-yl)benzyl] (5,6,7,8-tétrahydro-naphtalén-1-yl)amino](oxo) acétique ;
- l'acide {(1,1'-biphényl-3-ylméthyl)[4-(3-undécyl-1,2,4-oxadiazol-5-yl)benzyl] amino} (oxo) acétique ;
- l'acide {(1,1'-biphényl-3-ylméthyl)[3-(3-undécyl-1,2,4-oxadiazol-5-yl)benzyl] amino} (oxo) acétique ;
- l'acide {(1,1'-biphényl-3-ylméthyl)[4-(3-octyl-1,2,4-oxadiazol-5-yl)-benzyl] amino} (oxo)acétique ;
- l'acide {(1-benzothién-3-ylméthyl)[4-(3-undécyl-1,2,4-oxadiazol-5-yl)benzyl] amino} (oxo) acétique ;
- l'acide {(1-benzothién-3-ylméthyl)[3-(3-undécyl-1,2,4-oxadiazol-5-yl)benzyl] amino} (oxo) acétique ;
- l'acide {(1-benzothién-3-ylméthyl)[4-(3-octyl-1,2,4-oxadiazol-5-yl)-benzyl] amino} (oxo) acétique ;
- l'acide oxo{[2-(trifluorométhyl)benzyl][4-(3-undécyl -1,2,4-oxadiazol-5-yl)benzyl] amino} acétique ;
- l'acide oxo{[2-(trifluorométhyl)benzyl][3-(3-undécyl -1,2,4-oxadiazol-5-yl)benzyl] amino} acétique ;
- l'acide {[4-(3-octyl-1,2,4-oxadiazol-5-yl)benzyl][2-(trifluorométhyl)-benzyl] amino} (oxo) acétique ;
- l'acide oxo{[3-(trifluorométhyl)benzyl][4-(3-undécyl-1,2,4-oxadiazol-5-yl)benzyl] amino} acétique ;
- l'acide oxo{[3-(trifluorométhyl)benzyl][3-(3-undécyl -1,2,4-oxadiazol-5-yl)benzyl] amino} acétique ;
- l'acide {[4-(3-octyl-1,2,4-oxadiazol-5-yl)benzyl][3-(trifluorométhyl)-benzyl] amino} (oxo) acétique ;
- l'acide {(2-méthoxybenzyl)[4-(3-undécyl-1,2,4-oxadiazol-5-yl)-benzyl] amino} (oxo) acétique ;
- l'acide {(2-méthoxybenzyl)[3-(3-undécyl-1,2,4-oxadiazol-5-yl)-benzyl] amino} (oxo) acétique ;
- l'acide {(2-méthoxybenzyl)[4-(3-octyl-1,2,4-oxadiazol-5-yl)benzyl]-amino} (oxo) acétique ;
- l'acide oxo{{4-[(trifluorométhyl)sulfonyl]benzyl}[4-(3-undécyl-1,2,4-oxadiazol-5-yl)-benzyl] amino} acétique ;
- l'acide oxo{{4-[(trifluorométhyl)sulfonyl]benzyl}[3-(3-undécyl-1,2,4-oxadiazol-5-yl)-benzyl] amino} acétique ;
- l'acide ([4-(3-octyl-1,2,4-oxadiazol-5-yl)benzyl]{4-[(trifluorométhyl)-sulfonyl]benzyl} amino)(oxo) acétique ;
- l'acide {1,3-benzodioxol-5-yl[4-(3-undécyl-1,2,4-oxadiazol-5-yl)-benzyl] amino} (oxo) acétique ;
- l'acide {1,3-benzodioxol-5-yl[3-(3-undécyl-1,2,4-oxadiazol-5-yl)-benzyl] amino} (oxo) acétique ;
- l'acide {1,3-benzodioxol-5-yl[4-(3-octyl-1,2,4-oxadiazol-5-yl)benzyl]-amino} (oxo) acétique ;
- l'acide {[(4-dodéc-1-ynyl-1-naphtyl)méthyl][4-(trifluorométhyl)-benzyl] amino} (oxo) acétique ;
- l'acide {[(4-déc-1-ynyl-1-naphtyl)méthyl][4-(trifluorométhyl)benzyl]-amino} (oxo) acétique ;
- l'acide {[(4-déc-1-ynyl-1-naphtyl)méthyl][4-(trifluorométhyl)benzyl]-amino} (oxo) acétique ;
- l'acide oxo{[4-(trifluorométhyl)benzyl][4-(4-undécyl -1,3-thiazol-2-yl)-benzyl] amino} acétique ;
- l'acide {(4-déc-1-ynylbenzyl)[2-(2-fluorophényl) éthyl] amino} (oxo) acétique ;
- l'acide {(4-dodéc-1-ynylbenzyl)[2-(2-fluorophényl) éthyl] amino} (oxo) acétique ;
- l'acide {{[4-(dodécyloxy)-1-naphtyl]méthyl}[2-(2-fluorophényl)éthyl]-amino} (oxo) acétique-;
- l'acide {[2-(2-fluorophényl)éthyl][4-(octyloxy) benzyl] amino} (oxo) acétique ;
- l'acide {(4-déc-1-ynylbenzyl)[2-(trifluorométhyl) benzyl] amino} (oxo) acétique ;
- l'acide {(4-dodéc-1-ynylbenzyl)[2-(trifluorométhyl) benzyl] amino}-(oxo) acétique ;
- l'acide {{[4-(dodécyloxy)-1-naphtyl]méthyl}[2-(trifluorométhyl)-benzyl] amino} (oxo) acétique ;
- l'acide {[4-(octyloxy)benzyl][2-(trifluorométhyl) benzyl] amino} (oxo) acétique ;
- l'acide {(4-déc-1-ynylbenzyl)[2-(3,4-dichlorophényl) éthyl] amino}-(oxo) acétique ;
- l'acide [[2-(3,4-dichlorophényl)éthyl](4-dodéc-1-ynylbenzyl)amino]-(oxo) acétique ;
- l'acide ([2-(3,4-dichlorophényl)éthyl]{[4-(dodécyloxy)-1-naphtyl]-méthyl}amino)(oxo) acétique ;
- l'acide {[2-(3,4-dichlorophényl)éthyl][4-(octyloxy) benzyl] amino} (oxo) acétique ;
- l'acide ({4-[(4-hexylphényl)éthynyl]benzyl}{1-méthyl -1-[4-(trifluoro-méthyl)phényl] éthyl}amino) (oxo) acétique ;
- l'acide {[4-(5-cyclohexylpent-1-ynyl)benzyl][4-(trifluorométhyl)-benzyl] amino} (oxo) acétique ;
- l'acide {{3-[(4-hexylphényl)éthynyl]benzyl}[4-(trifluorométhyl)benzyl]-amino} (oxo) acétique ;
- l'acide {[4-(4-éthyl-3-hydroxyoct-1-ynyl)benzyl][4-(trifluorométhyl)-benzyl] amino} (oxo) acétique ;
- l'acide {(2-déc-1-ynylbenzyl)[4-(trifluorométhyl) benzyl] amino} (oxo) acétique ;
- l'acide {(4-déc-1-ynylbenzyl)[4-(trifluorométhyl) benzyl] amino} (oxo) acétique, sel de L-lysine ;
- l'acide {(4-déc-1-ynylbenzyl)[4-(trifluorométhyl) benzyl] amino} (oxo) acétique, sel de trométhamine (c'est-à-dire (2-amino-2-hydroxyméthyl)-1,3-propanediol) ;
- l'acide {(4-déc-1-ynylbenzyl)[4-(trifluorométhyl) benzyl] amino} (oxo) acétique, sel de L-arginine ;
- le {(4-déc-1-ynylbenzyl)[4-(trifluorométhyl)benzyl] amino} (oxo) acétate de sodium.

17. Utilisation selon l'une quelconque des revendications précédentes, dans laquelle le trouble cardiovasculaire est une insuffisance cardiaque.

18. Utilisation selon l'une quelconque des revendications précédentes, dans laquelle le trouble cardiovasculaire est une insuffisance cardiaque chronique.

19. Utilisation selon l'une quelconque des revendications précédentes, dans laquelle le trouble cardiovasculaire est un dysfonctionnement endothélial.
